# FASCICULE DE BREVET EUROPEEN

(11) **EP 1 701 938 B1**
(45) Date de publication et mention de la délivrance du brevet: **25.07.2012**
(21) Numéro de dépôt: 05717386.6
(22) Date de dépôt: 07.01.2005
(51) Int. Cl.: C07C 323/22, C07C 59/90, C07D 339/04, C07C 323/12

(54) **COMPOSES DERIVES DE 1,3-DIPHENYLPROP-2-EN-1-ONE, PREPARATION ET UTILISATIONS**
1,3-DIPHENYLPROP-2-EN-1-ONDERIVATVERBINDUNGEN, VERFAHREN ZU DEREN HERSTELLUNG UND DEREN VERWENDUNG
1,3-DIPHENYLPROP-2-EN-1-ONE DERIVATIVE COMPOUNDS, PREPARATION METHOD THEREOF AND USES OF SAME

(30) Priorité: 08.01.2004 FR 0400123; 01.09.2004 FR 0409257
(43) Date de publication de la demande: 20.09.2006
(73) Titulaire: Genfit, 59120 Loos (FR)
(72) Inventeur: CAUMONT-BERTRAND, Karine, F-59236 Frelinghien (FR); DELHOMEL, Jean-François, F-62144 Acq (FR)
(74) Mandataire: Tezier Herman, Béatrice
(86) Numéro de dépôt international: PCT/FR2005/000040
(87) Numéro de publication internationale: WO 2005/073184

(56) Documents cités:
- EP-A- 0 659 743
- DE-A- 4 327 365
- US-A- 5 523 302

## Description

La présente invention concerne des dérivés de 1,3-diphénylprop-2-èn-1-one substitués, les compositions pharmaceutiques et/ou cosmétiques les comprenant, leurs applications en thérapeutique et/ou en cosmétique, notamment dans les domaines de la santé humaine et animale. La présente invention a également trait à un procédé de préparation de ces dérivés.

Les composés selon l'invention représentent un outil thérapeutique avantageux pour l'amélioration des pathologies liées aux dérèglements du métabolisme lipidique et/ou glucidique (en particulier hyperlipidémie, diabète, ou obésité) et sont utilisables notamment pour prévenir ou traiter les maladies cardiovasculaires (notamment les maladies coronariennes, l'ischémie cérébrale et les maladies artérielles périphériques), les dyslipidémies, les pathologies associées au syndrome X, le diabète, l'obésité, l'hypertension, les maladies inflammatoires, les maladies dermatologiques (en particulier psoriasis, dermatites atopiques, ou acné), l'asthme, les désordres liés au stress oxydatif, les effets du vieillissement en général, par exemple le vieillissement cutané, notamment dans le domaine cosmétique (en particulier l'apparition de rides). Les composés selon l'invention sont capables d'exercer une activité prophylactique en terme de neuroprotection, et également d'assurer une neuroprotection active dans la phase aiguë des accidents ischémiques cérébraux, qui constituent une des complications majeures des affections cardiovasculaires.

Par leur action simultanée sur plusieurs facteurs de risque des maladies cardiovasculaires, les composés selon l'invention permettent une diminution du risque cardiovasculaire global.

Les maladies coronariennes, l'ischémie cérébrale et les maladies artérielles périphériques constituent les principales maladies cardiovasculaires selon l'International Atherosclerosis Society (Harmonized Clinical Guidelines on Prevention of Atherosclerotic Vascular Disease, 2003).

Les maladies cardiovasculaires représentent aujourd'hui une des causes majeures de mortalité chez l'adulte dans la plupart des pays développés et dans certains pays en voie de développement. Parmi les maladies cardiovasculaires, la pathologie vasculaire cérébrale représente la 3^{ème} cause de mortalité et la 1^{ère} cause de handicap chez l'adulte. Le besoin de stratégies de traitement et/ou de prévention efficaces contre ces pathologies est devenu une urgence mondiale.

Les dyslipidémies (l'hypercholestérolémie, l'hypertriglycéridémie), le diabète et l'hypertension font partie des facteurs de risque cardiovasculaire clairement identifiés (IAS, 2003). Il semble également qu'une protection insuffisante des lipoprotéines contre l'oxydation soit un facteur de risque identifié.

Les études épidémiologiques ont montré qu'il existe un effet synergique entre ces différents facteurs. La présence concomitante de plusieurs d'entre eux conduit à une aggravation dramatique du risque cardiovasculaire. Il convient alors de parler de risque global (*global risk*) pour les maladies cardiovasculaires.
Il existe donc un réel besoin de produits capables d'agir de façon concomitante sur ces différents facteurs de risque et donc de diminuer le risque des maladies cardiovasculaires mais également de traiter chaque dérèglement et ses conséquences pris indépendamment (en particulier dyslipidémies, diabète, hypertension, ischémie cérébrale, pathologies associées au syndrome X, ou obésité).

Les inventeurs ont montré, de manière surprenante, que les composés selon l'invention sont des activateurs PPAR (Peroxisome Proliferator-Activated Receptor) et qu'ils représentent donc un outil thérapeutique avantageux.

Il est bien connu en effet que les PPARs sont associés au métabolisme des lipides et du glucose. Les activateurs de PPARs, les fibrates par exemple, permettent de réguler le cholestérol plasmatique ainsi que la concentration de triglycérides via l'activation de PPARα (Hourton, Delerive *et al.* 2001). Le traitement avec des fibrates entraîne une augmentation de l'oxydation des acides gras au niveau hépatique. Ils réduisent également la synthèse et l'expression des triglycérides (Staels and Auwerx 1998). Les activateurs de PPARα sont également capables de corriger une hyperglycémie ainsi que la concentration d'insuline. Les fibrates diminuent par ailleurs la masse du tissu adipeux grâce à un mécanisme indépendant de la prise alimentaire et de l'expression du gène codant pour la leptine (Guerre-Millo, Gervois *et al.* 2000).

L'intérêt thérapeutique des agonistes PPARγ a été largement étudié dans le traitement du diabète de type II (Spiegelman 1998). Il a été montré que les agonistes PPARγ permettent la restauration de la sensibilité à l'insuline des tissus cibles ainsi que la réduction des taux plasmatiques de glucose, de lipides et d'insuline aussi bien dans des modèles animaux de diabète de type II que chez l'homme (Ram 2003).

L'activation des PPARs par les ligands intervient également dans la régulation de l'expression de gènes participant à des processus comme l'inflammation, l'angiogenèse, la prolifération et la différenciation cellulaires, l'apoptose et les activités de iNOS, de la MMPase et des TIMPs. L'activation de PPARα dans des kératinocytes entraîne un arrêt de leur prolifération et de l'expression de gènes impliqués dans la différenciation cellulaire (Komuves, Hanley *et al.* 2000).

Il a été aussi démontré que l'activation des PPARs interfère avec la différenciation, la maturation, la migration et l'immunogénécité des cellules dendritiques, qui sont les cellules présentatrices d'antigène les plus puissantes (Gosset et al. 2001 ; Nencioni et al. 2002 ; Angeli et al. 2003).

Les PPARs ont des propriétés anti-inflammatoires car ils interfèrent négativement dans des mécanismes de transcription impliquant d'autres facteurs de transcription tels que NF-kB ou les activateurs de la transcription (STAT) et AP-1 (Desvergne and Wahli 1999). Ces propriétés anti-inflammatoires et anti-prolifératives font des PPARs des cibles thérapeutiques d'intérêt pour le traitement de maladies comme les maladies occlusives vasculaires (en particulier athérosclérose), l'ischémie cérébrale, l'hypertension, les maladies liées à une néo-vascularisation (en particulier rétinopathies diabétiques), les maladies inflammatoires (en particulier maladie de Bowel ou psoriasis), l'asthme et les maladies néoplasiques (en particulier carcinogenèse).

De plus, les composés selon l'invention présentent l'avantage d'être des antioxydants.

Les radicaux libres interviennent en effet dans un spectre très large de pathologies comme les pathologies cardiovasculaires (en particulier athérosclérose), l'ischémie cérébrale, les désordres génétiques et métaboliques (en particulier diabètes) mais également les maladies infectieuses et dégénératives (en particulier Alzheimer, Parkinson, ou Prion), les problèmes ophtalmiques, le vieillissement, les allergies, l'initiation et la promotion cancéreuse (Mates, Perez-Gomez *et al.* 1999).

Les espèces réactives oxygénées (ROS) sont produites pendant le fonctionnement normal de la cellule. Les ROS sont constituées de radicaux hydroxyle (°OH), de l'anion superoxyde (O₂^{°-}), du peroxyde d'hydrogène (H₂O₂) et de l'oxyde nitrique (NO^{°}). Ces espèces sont très labiles et du fait de leur grande réactivité chimique, elles peuvent également constituer un danger pour les fonctions biologiques des cellules en provoquant des réactions de peroxydation lipidique, l'oxydation de certaines enzymes et des oxydations très importantes de protéines qui mènent à leur dégradation.

La prise en charge des ROS se fait via un système antioxydant comprenant une composante enzymatique (superoxyde dismutase, catalase et gluthation peroxydase) et une composante non enzymatique essentiellement les caroténoïdes, la vitamine C et la vitamine E (Gilgun-Sherki, Melamed *et al.* 2001).

De plus, les résultats de nombreuses études *in vitro* et *in vivo* ont montré la participation potentielle des LDL (Low Density Lipoproteins) oxydées à l'athérosclérose. La plaque d'athérosclérose à développement lent comporte un noyau riche en cholestérol entouré d'une coque de tissus fibreux. La fissuration de la plaque est de plus en plus considérée comme le résultat de modifications inflammatoires chroniques dans la région de la coque fibreuse. Les médiateurs de l'inflammation tels que les cytokines influencent plusieurs processus biologiques dans la coque fibreuse de la plaque, diminuant sa résistance à la rupture.

Les cytokines inflammatoires dans la plaque athéromateuse, dont l'interleukine I, le facteur de nécrose tumorale (TNF-α et l'homologue de surface de TNFα appelé CD-40 ligand), conduisent à la production par les macrophages et les cellules musculaires lisses d'enzymes qui peuvent affaiblir la matrice extracellulaire. De la fissuration de la coque peuvent résulter des thromboses occlusives.

Les composés selon l'invention sont aussi des outils thérapeutiques avantageux pour le traitement et/ou la prévention de l'ischémie cérébrale de par leurs propriétés pharmacologiques et notamment anti-inflammatoires.

Le premier événement de l'ischémie cérébrale survient dans les premières heures, et consiste en une libération massive de glutamate qui aboutit à une dépolarisation neuronale ainsi qu'à un oedème cellulaire. L'entrée de calcium dans la cellule induit des dégâts mitochondriaux favorisant la libération de radicaux libres ainsi que l'induction d'enzymes qui provoquent la dégradation membranaire des neurones. L'entrée de calcium et la production de radicaux libres activent à leur tour certains facteurs de transcription, comme NF-κB. Cette activation induit des processus inflammatoires comme l'induction de protéines d'adhésion au niveau endothélial, l'infiltration du foyer ischémique par les polynucléaires neutrophiles, l'activation microgliale, l'induction d'enzymes comme l'oxyde nitrique (NO) synthase de type II ou la cyclooxygenase de type II. Ces processus inflammatoires conduisent à la libération de NO ou de prostanoïdes qui sont toxiques pour la cellule. L'ensemble de ces processus aboutit à un phénomène d'apoptose provoquant des lésions irréversibles (Dirnagl, ladecola *et al.* 1999).

Le concept de neuroprotection prophylactique s'appuie sur des bases expérimentales mettant en évidence une résistance vis-à-vis de l'ischémie dans des modèles animaux. Différents mécanismes de résistance à l'ischémie cérébrale été mis en évidence : cytokines, voies de l'inflammation, radicaux libres, NO, canaux potassique ATP dépendant, adénosine. Les composés selon l'invention présentent ainsi l'avantage de jouer le rôle de neuroprotecteur.

Enfin, les composés selon l'invention présentent un intérêt particulier dans le traitement des pathologies inflammatoires, notamment dans le traitement de l'asthme. En effet, la prévalence des maladies allergiques, notamment de l'asthme, s'accroît régulièrement dans l'ensemble des pays industrialisés, représentant un problème majeur de santé publique. Quel que soit le mécanisme en cause, les affections d'origine allergiques ont en commun une réaction inflammatoire initiée par les cellules dendritiques présentant l'antigène. Les inventeurs ont montré que les composés de la présente invention interfèrent avec la différenciation et la maturation des ces cellules dendritiques et inhibent la migration de ces cellules vers les organes lymphoïdes secondaires. De plus, il a été montré que les composés selon l'invention présentent une capacité réduite à induire la prolifération des cellules T CD4+ naïves.

Les composés selon l'invention interfèrent donc avec l'initiation de la réponse immunitaire et représentent donc un outil thérapeutique avantageux pour le traitement de l'asthme.

Le brevet US 5,523,302 décrit des composés aromatiques et leur utilisation comme antagonistes du récepteur au fibrinogène. Le document DE 43 27 365 décrit des composés dérivés du phénol et leurs utilisations comme agent abaissant le fibrinogène.

Le document EP 1 440 688 divulgue des dérivés de 1,3-diphénylprop-2-èn-1-one décrits comme des ligands PPAR (Peroxisome Proliferator-Activated Receptor) mais différents de la présente invention.

La présente invention concerne de nouveaux dérivés de 1,3-diphénylprop-2-èn-1-one substitués, des compositions pharmaceutiques et/ou cosmétiques les comprenant, leurs applications en thérapeutique et/ou en cosmétique, notamment dans les domaines de la santé humaine et animale. La présente invention a également trait à un procédé de préparation de ces dérivés.

Les inventeurs ont mis en évidence, de manière surprenante, que les composés selon l'invention possèdent une activité PPAR agoniste et des propriétés antioxydantes. Les composés selon l'invention sont donc capables d'interférer avec au moins deux voies de signalisation qui sont activées en particulier pendant l'inflammation : la production de cytokines ainsi que la production de radicaux libres. En agissant de manière synergique les composés selon l'invention représentent un moyen thérapeutique et/ou cosmétique avantageux pour le traitement des maladies cardiovasculaires, des pathologies associées au syndrome X, des dyslipidémies, du diabète, de l'obésité, de l'hypertension, des maladies inflammatoires, des maladies dermatologiques (en particulier psoriasis, dermatites atopiques, ou acné), de l'asthme, des désordres liés au stress oxydatif, du vieillissement en général, par exemple du vieillissement cutané, notamment dans le domaine cosmétique (en particulier l'apparition de rides).

D'autre part, les composés selon l'invention sont capables d'exercer une activité prophylactique en terme de neuroprotection, et également d'assurer une neuroprotection active dans la phase aiguë des accidents ischémiques cérébraux.

Enfin, les composés selon l'invention représentent un outil thérapeutique avantageux pour prévenir et/ou traiter plusieurs facteurs de risque cardiovasculaires liés aux dérèglements du métabolisme lipidique et/ou glucidique (en particulier hyperlipidémie, diabète, ou obésité). Ils permettent la diminution du risque global.

La présente invention a donc pour but de proposer de nouveaux dérivés de 1,3-diphénylprop-2-èn-1-one substitués présentant une formule améliorée et une efficacité thérapeutique satisfaisante.

Ces buts et d'autres sont atteints par la présente invention qui a notamment pour objet des dérivés de 1,3-diphénylprop-2-èn-1-one substitués de formule générale (I) suivante : 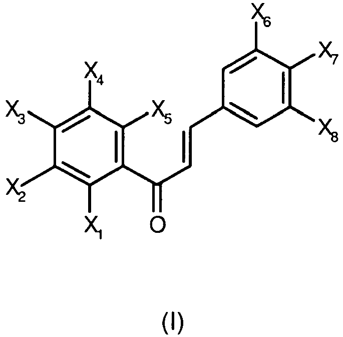 dans laquelle :
X₇ représente un groupement répondant à la formule suivante : G₇-R₇ dans laquelle G₇ est un atome d'oxygène et R₇ est une chaîne alkyle de 1 à 7 atomes de carbone substituée par un groupement carboxy de formule : -COORₐ,
avec Rₐ représentant un atome d'hydrogène ou un radical alkyle de 1 à 7 atomes de carbone,
les groupements Xᵢ avec i = 1, 2, 3, 4 ou 5, identiques ou différents, représentent un atome d'halogène ou un groupement thionitroso ou répondent respectivement à la formule (Gᵢ-Rᵢ)ₙ-G'ᵢ-R'ᵢ dans laquelle :
   ■ n peut prendre les valeurs 0 ou 1,
   ■ Gᵢ et G'ᵢ, identiques ou différents, représentent une simple liaison, un atome d'oxygène ou un atome de soufre,
   ■ Rᵢ et R'ᵢ, identiques ou différents, représentent un radical alkyle de 1 à 7 atomes de carbone, phényle, dithiolane, pyridine, furanne, thiophène ou morpholine,
   ■ R'ᵢ peut également représenter un atome d'hydrogène,
les groupements Xᵢ avec i = 6 ou i = 8, identiques ou différents, représentent un atome d'halogène ou répondent à la formule G'ᵢ-R'ᵢ , G'ᵢ et R'ᵢ étant tels que définis précédemment, X₆ et X₈ ne représentant pas simultanément un atome d'hydrogène,
Xi avec i = 1, 2, 3, 4, 5, 6 ou 8 ne pouvant représenter un radical dithiolane, pyridine, furanne, thiophène ou morpholine directement lié au cycle aromatique de la 1,3-diphényl prop-2-én-1-one,
à l'exclusion des composés de formule (I) pour lesquels simultanément :
   ■ un des groupements X₁, X₂, X₃, X₄ ou X₅ est un groupement hydroxyle,
   ■ G₇ est un atome d'oxygène,
   ■ et un des groupements X₆ ou X₈ est un atome d'hydrogène ou d'halogène ou un hydroxyle ou un groupement alkyloxy,
à l'exclusion des composés de formule (I) pour lesquels simultanément :
   ■ les groupements X₁, X₂ et X₄ représentent simultanément un atome d'hydrogène,
   ■ les groupements X₆ et X₈ représentent G'ᵢR'ᵢ,
   ■ le groupement X₅ représente un groupement thionitroso ou un groupement G'ᵢR'ᵢ,
   ■ le groupement X₃ représente un halogène ou un groupement G'ᵢR'ᵢ,
dans lesquels G'ᵢ représente un atome d'oxygène, un atome de soufre ou une simple liaison et R'ᵢ représente un groupement alkyle saturé, linéaire, ramifié ou cyclique, halogéné ou non, ou un atome d'hydrogène.

Selon un mode particulier, les composés de formule (I) sont tels que définis ci-dessus et excluent les composés de formule (I) pour lesquels simultanément :
■ les groupements X₁, X₂ et X₄ représentent simultanément un atome d'hydrogène,
■ et un des groupements X₃ ou X₅ représente un atome d'hydrogène ou un halogène ou un radical alkyle ou un radical alkyloxy ou un radical alkylthio ou un groupement hydroxyle ou un groupement thiol ou un groupement thionitroso.

De manière préférentielle, un objet particulier de l'invention concerne les composés de formule générale (la) qui correspondent aux composés de formule générale (I) dans laquelle X₁ et X₅ sont des atomes d'hydrogène.

De manière préférentielle, la présente invention concerne des composés de formule générale (Ib) qui correspondent aux composés de formule générale (I) dans laquelle X₂ et X₄ sont des radicaux alkyle de 1 à 7 atomes de carbone et plus avantageusement dans laquelle X₁ et X₅ sont des atomes d'hydrogène.

Un objet particulier de l'invention concerne les composés de formule générale (Ic) qui sont des composés de formule générale (I) dans laquelle X₁, X₃ et X₄ sont des radicaux alkyle de 1 à 7 atomes de carbone.

Un autre objet particulier de l'invention concerne les composés de formule générale (Id) qui sont des composés de formule générale (I) dans laquelle X₁, X₂, X₄ et X₅ sont des atomes d'hydrogène.

Un autre objet de l'invention concerne les composés de formule générale (II) qui sont des composés de formule générale (I) dans laquelle X₆ et X₈ sont des radicaux alkyle de 1 à 7 atomes de carbone.

De manière encore plus préférentielle, les composés de formule générale (II) sont ceux dans laquelle X₁ et X₅ sont des atomes d'hydrogène et avantageusement dans laquelle X₂ et X₄ sont des radicaux alkyle de 1 à 7 atomes de carbone.

Un autre objet particulier de l'invention concerne les composés de formule générale (II) dans laquelle X₁, X₃, X₄, X₆ et X₈ sont des radicaux alkyle de 1 à 7 atomes de carbone.

Un autre objet particulier de l'invention concerne les composés de formule générale (II) dans laquelle X₆ et X₈ sont des radicaux alkyles de 1 à 7 atomes de carbone et X₁, X₂, X₄ et X₅ sont des atomes d'hydrogène.

Selon un aspect particulier de l'invention, les composés de formule (I) sont tels que définis ci-avant avec X₃ qui représente un atome d'halogène ou un groupement thionitroso ou répond à la formule (Gᵢ-Rᵢ)ₙ-G'ᵢ-R'ᵢ telle que définie antérieurement, dans laquelle G'ᵢ représente un atome d'oxygène ou un atome de soufre.

La présente invention inclut également les isomères optiques et géométriques, les racémates, les tautomères, les sels, les hydrates et les mélanges des composés selon l'invention.

La divulgation concerne également les prodrogues des composés selon l'invention, qui, après administration chez un sujet, se transforment en composés selon l'invention et/ou les métabolites des composés selon l'invention qui présentent des activités thérapeutiques comparables aux composés selon l'invention.

De manière préférentielle, au moins un des groupements Gi ou G'i représente un atome de soufre avec i pouvant prendre une des valeurs 1, 2, 3, 4, 5, 6 ou 8.

Dans le cadre de la présente invention, les dérivés selon l'invention tels que décrits ci-dessus peuvent adopter la conformation cis ou *trans*.

Selon la présente invention, le terme « alkyle » désigne un radical hydrocarboné saturé, linéaire, ramifié ou cyclique, halogéné ou non, ayant plus particulièrement de 1 à 24, de préférence 1 à 10, atomes de carbone tels que méthyle, éthyle, propyle, isopropyle, n-butyle, isobutyle, tertiobutyle, pentyle, néopentyle, n-hexyle ou cyclohexyle. Les groupes comportant un ou deux atomes de carbone ou comportant de deux à sept atomes de carbone sont particulièrement préférés. Les groupes méthyle et éthyle sont tout particulièrement préférés.

Selon la présente invention, le terme « alkényle » désigne un radical hydrocarboné insaturé, linéaire, ramifié ou cyclique, halogéné ou non, ayant plus particulièrement de 1 à 24, de préférence 1 à 10, atomes de carbone.

Selon la présente invention, le terme « aryle » désigne un radical hydrocarboné aromatique substitué ou non, en particulier substitué par au moins un atome d'halogène, un radical alkyle, hydroxyle, thiol, alkyloxy, alkylthio, oxime ou thionitroso. Les groupes phényles sont tout particulièrement préférés.

Selon la présente invention, le terme « hétérocycle » désigne un radical cyclique saturé ou insaturé ou aromatique comprenant un ou plusieurs hétéroatomes, tels que azote, soufre et oxygène. Ils peuvent être substitués, avantageusement par au moins un groupement alkyle tel que défini précédemment. Les hétérocycles tels que dithiolanes, pyridine, furanne, thiophène ou morpholine sont particulièrement préférés. Dans le cadre de la présente invention, les hétérocycles pipéridine et pipérazine sont avantageusement substitués par au moins un groupement alkyle tel que défini précédemment.

Le terme thionitroso fait référence à un groupement nitroso lié au cycle aromatique par l'intermédiaire d'un atome de soufre.

Le terme alkyloxy fait référence à une chaîne alkyle liée au cycle par l'intermédiaire d'un atome d'oxygène. La chaîne alkyle répond à la définition précédemment énoncée.

Le terme alkylthio fait référence à une chaîne alkyle liée au cycle aromatique par l'intermédiaire d'un atome de soufre (liaison thioéther). La chaîne alkyle répond à la définition précédemment énoncée.

L'atome d'halogène représente un atome de chlore, brome, iode ou fluor.

Selon un mode particulier de l'invention, les composés préférés sont indiqués ci-dessous avec les formules qui leur sont associées :
La 1-(4-((R, S)-5-[1,2]dithiolan-3-ylpentyloxy)-3,5-diméthylphényl)-3-(4-carboxydiméthylméthyloxy-3,5-diméthylphényl)-prop-2-èn-1-one : 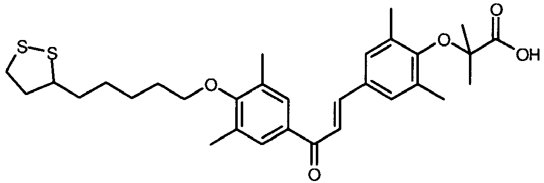
La 1-(4-Mercapto-3,5-diméthylphényl)-3-(4-carboxydiméthylméthyloxy-3,5-diméthyl phényl)-prop-2-èn-1-one : 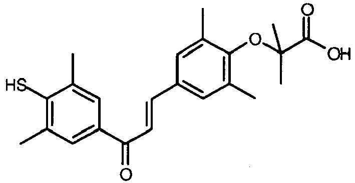
La 1-(4-Cyclohexyléthylthio-3,5-diméthylphényl)-3-(4-carboxydiméthylméthyloxy-3,5-diméthylphényl)-prop-2-èn-1-one : 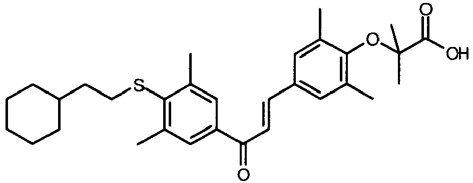
La 1-(2,5-Dihydroxy-3,4,6-triméthylphényl)-3-(4-carboxydiméthylméthyloxy-3,5-diméthylphényl)-prop-2-èn-1-one : 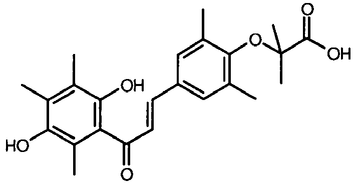
La 1-(2,5-Diméthoxy-3,4,6-triméthylphényl)-3-(4-carboxydiméthylméthoxy-3,5-diméthylphényl)-prop-2-èn-1-one : 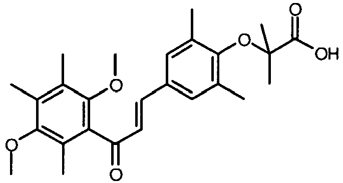
La 1-(2,5-Dihydroxyphényl)-3-(4-carboxydiméthylméthyloxy-3,5-diméthylphényl)-prop-2-èn-1-one : 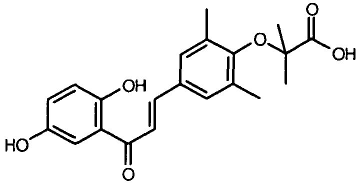
La 1-(2,5-Diméthoxyphényl)-3-(4-carboxydiméthylméthyloxy-3,5-diméthylphényl)-prop-2-èn-1-one : 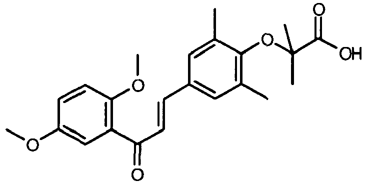
La 1-(4-Phényléthyloxyphényl)-3-(4-carboxydiméthylméthyloxy-3,5-diméthylphényl)-prop-2-èn-1-one : 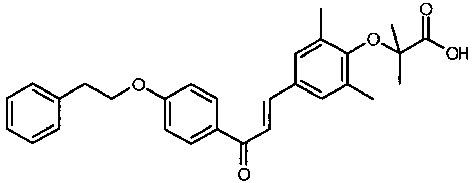
La 1-(4-(Morpholin-4-yléthyloxy)phényl)-3-(4-carboxydiméthylméthyloxy-3,5-diméthylphényl)-prop-2-èn-1-one : 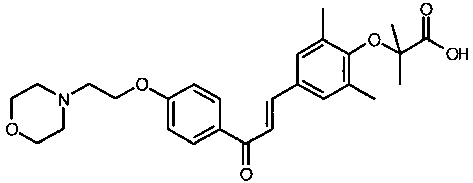
La 1-(4-(Pentylthioéthyloxy)phényl)-3-(4-tertiobutyloxycarbonyldiméthylméthyloxy-3,5-diméthylphényl)prop-2-èn-1-one : 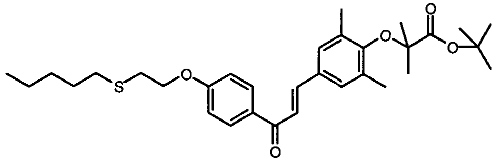
La 1-(4-(Pentylthioéthyloxy)phényl)-3-(4-carboxydiméthylméthyloxy-3,5-diméthylphényl)prop-2-èn-1-one : 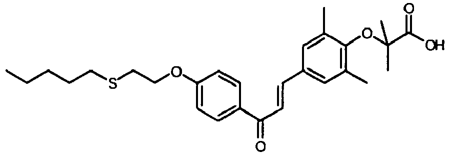
La 1-(4-Hydroxy-3,5-diméthylphényl)-3-(4-tertiobutyloxycarbonyldiméthylméthyloxy-3,5-diméthylphényl)-prop-2-ène-1-one : 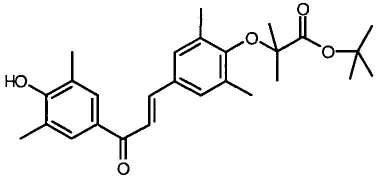
La 1-(4-Hydroxy-3,5-diméthylphényl)-3-(4-carboxydiméthylméthyloxy-3,5-diméthylphényl)-prop-2-ène-1-one : 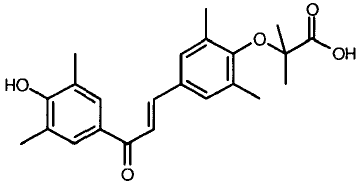
La 1-(4-((R,S)-5-[1,2]dithiolan-3-ylpentyloxy)phényl)-3-(4-tertiobutyloxycarbonyl diméthyl méthyloxy-3,5-diméthylphényl)-prop-2-èn-1-one : 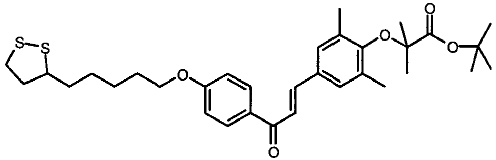
La 1-(4-((R,S)-5-[1,2]dithiolan-3-ylpentyloxy)phényl)-3-(4-carboxydiméthylméthyloxy-3,5-diméthylphényl)-prop-2-èn-1-one : 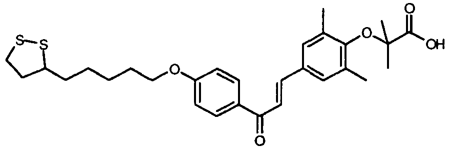
La 1-(4-Méthylthiophényl)-3-(4-tertiobutyloxycarbonyldiméthylméthyloxy-3,5-dibromophényl)-prop-2-ène-1-one : 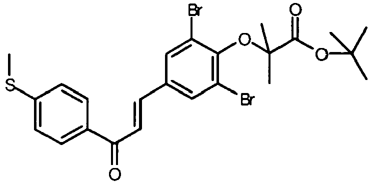
La 1-(4-Méthylthiophényl)-3-(4-carboxydiméthylméthyloxy-3,5-dibromophényl)-prop-2-ène-1-one : 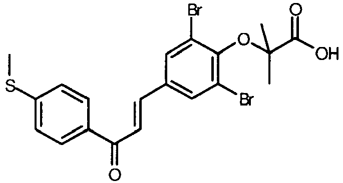
La 1-(4-Cyclohexyléthyloxyphényl)-3-(4-tertiobutyloxycarbonyldiméthylméthyloxy-3,5-diméthylphényl)prop-2-èn-1-one : 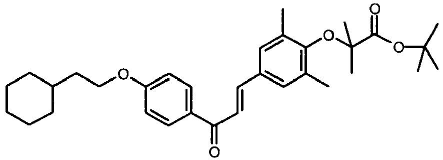
La 1-(4-Cyclohexyléthyloxyphényl)-3-(4-carboxydiméthylméthyloxy-3,5-diméthylphényl) prop-2-èn-1-one : 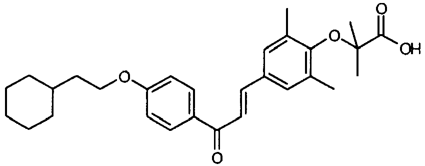
La 1-(4-Méthylthio-3,5-diméthylphényl)-3-(4-tertiobutyloxycarbonyldiméthylméthyloxy-3,5-diméthylphényl) prop-2-èn-1-one : 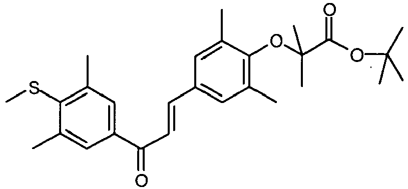
La 1-(4-Méthylthio-3,5-diméthylphényl)-3-(4-carboxydiméthylméthyloxy-3,5-diméthylphényl) prop-2-èn-1-one : 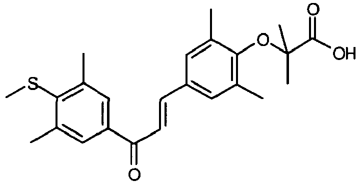
La 1-(4-Propyloxy-3,5-diméthytphényl)-3-(4-tertiobutyloxycarbonyldiméthylméthyloxy-3,5-diméthylphényl)prop-2-èn-1-one : 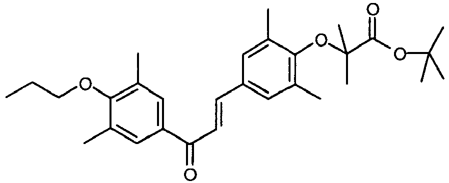
La 1-(4-Propyloxy-3,5-diméthylphényl)-3-(4-carboxydiméthylméthyloxy-3,5-diméthylphényl)prop-2-èn-1-one : 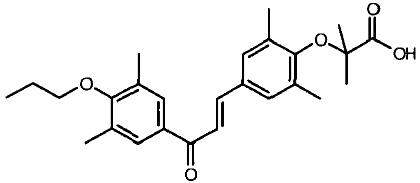
La 1-(4-Méthoxy-3,5-diméthylphényl)-3-(4-tertiobutyloxycarbonyldiméthylméthyloxy-3,5-diméthylphényl)prop-2-èn-1-one : 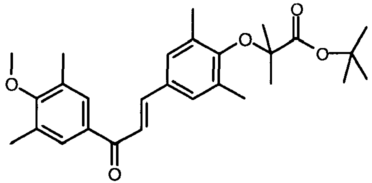
La 1-(4-Méthoxy-3, 5-diméthylphényl)-3-(4-carboxydiméthylméthyloxy-3,5-diméthylphényl)prop-2-èn-1-one : 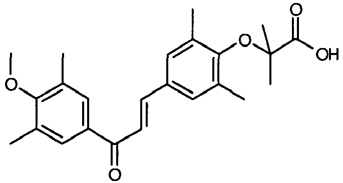
La 1-(4-Hexyloxy-3,5-diméthylphényl)-3-(4-tertiobutyloxycarbonyldiméthyl méthyloxy -3,5-diméthylphényl)prop-2-èn-1-one : 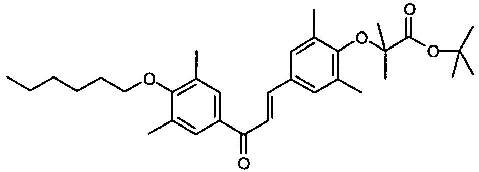
La 1-(4-Hexyloxy-3,5-diméthylphényl)-3-(4-carboxydiméthylméthyloxy-3,5-diméthyl phényl)prop-2-èn-1-one : 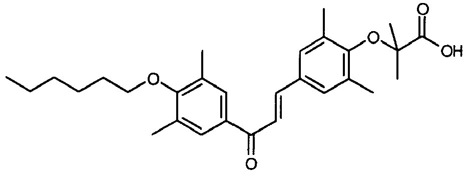
La 1-(4-Cyclohexyléthyloxy-3,5-diméthylphényl)-3-(4-tertiobutyloxycarbonyldiméthyl méthyloxy-3,5-diméthylphényl) prop-2-èn-1-one : 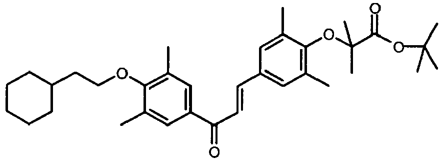
La 1-(4-Cyclohexyléthyloxy-3,5-diméthylphényl)-3-(4-carboxydiméthylméthyloxy-3,5-diméthylphényl) prop-2-èn-1-one : 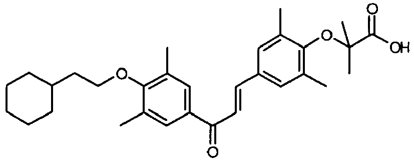
La 1-(4-Cyclohexylthioéthyloxyphényl)-3-(4-tertiobutyloxycarbonyldiméthylméthyloxy-3,5-diméthylphényl) prop-2-èn-1-one : 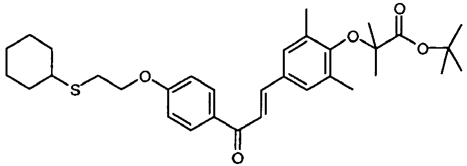
La 1-(4-Cyclohexylthioéthyloxyphényl)-3-(4-carboxydiméthylméthyloxy-3,5-diméthylphényl) prop-2-èn-1-one : 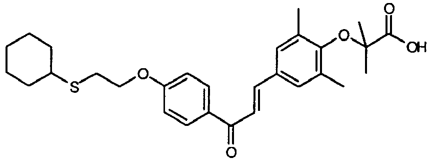
La 1-(2,4,5-Triméthylphényl)-3-(4-tertiobutyloxycarbonyldiméthylméthyloxy-3,5-diméthylphényl) prop-2-èn-1-one : 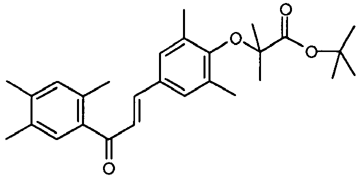
La 1-(2,4,5-Triméthylphényl)-3-(4-carboxydiméthylméthyloxy-3,5-diméthylphényl) prop-2-èn-1-one : 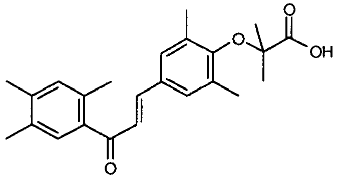
La 1-(4-Cyclohexylthioéthyloxy-3,5-diméthylphényl)-3-(4-tertiobutyloxycarbonyldiméthylméthyloxy-3,5-diméthylphényl) prop-2-èn-1-one : 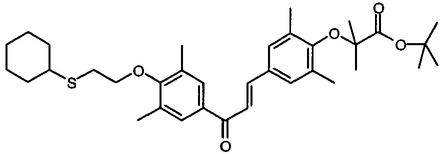
La 1-(4-Cyclohexylthioéthyloxy-3,5-diméthylphényl)-3-(4-carboxydiméthylméthyloxy-3,5-diméthylphényl)prop-2-èn-1-one : 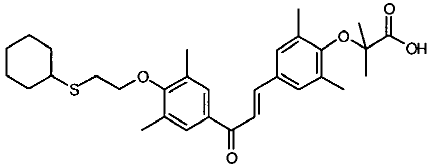
La 1-(4-Méthylthiophényl)-3-(4-tertiobutyloxycarbonyldiméthylméthyloxy-3-fluorophényl)prop-2-èn-1-one : 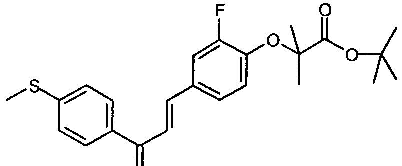
La 1-(4-Méthylthiophényl)-3-(4-carboxydiméthylméthyloxy-3-fluorophényl)prop-2-èn-1-one : 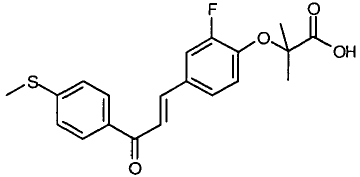
La 1-(2,3,4,5,6-Pentaméthylphényl)-3-(4-tertiobutyloxycarbonyldiméthylméthyloxy-3,5-diméthylphényl)prop-2-èn-1-one : 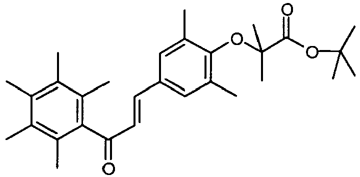
La 1-(2, 3,4,5,6-Pentaméthylphényl)-3-(4-carboxydiméthylméthyloxy-3,5-diméthylphényl)prop-2-èn-1-one : 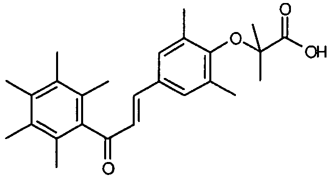
La 1-(4-Phényloxyphényl)-3-(4-tertiobutyloxycarbonyldiméthylméthyloxy-3,5-diméthylphényl)prop-2-èn-1-one : 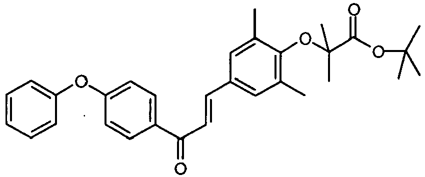
La 1-(4-Phényloxyphényl)-3-(4-carboxydiméthylméthyloxy-3,5-diméthylphényl)prop-2-èn-1-one : 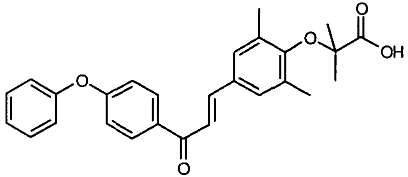
La 1-(4-Méthoxy-3-fluorophényl)-3-(4-tertiobutyloxycarbonyldiméthylméthyloxy-3,5-diméthylphényl)prop-2-èn-1-one : 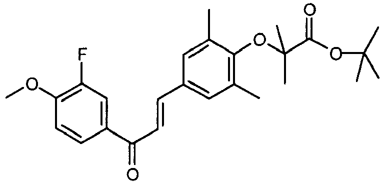
La 1-(4-Méthoxy-3-fluorophényl)-3-(4-carboxydiméthylméthyloxy-3,5-diméthylphényl) prop-2-èn-1-one : 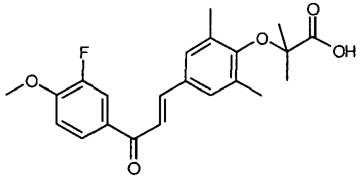
La 1-(4-Méthoxy-3-méthylphényl)-3-(4-tertiobutyloxycarbonyldiméthylméthyloxy-3,5-diméthylphényl)prop-2-èn-1-one : 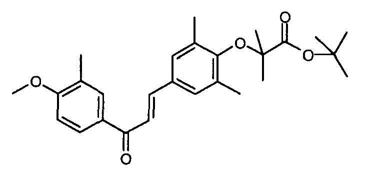
La 1-(4-Méthoxy-3-méthylphényl)-3-(4-carboxydiméthylméthyloxy-3,5-diméthylphényl) prop-2-èn-1-one : 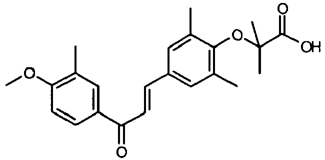
La 1-(4-Hexylthio-3,5-diméthylphényl)-3-(4-tertiobutyloxycarbonyldiméthylméthyloxy-3,5-diméthylphényl) prop-2-èn-1-one : 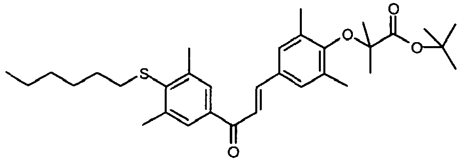
La 1-(4-Hexylthio-3,5-diméthylphényl)-3-(4-carboxydiméthylméthyloxy-3,5-diméthylphényl) prop-2-èn-1-one : 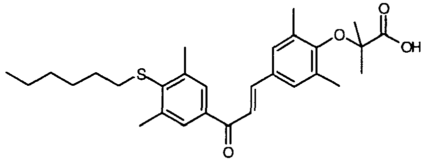
La 1-(2,5-Diméthoxyphényl)-3-(4-tertiobutyloxycarbonyldiméthylméthyloxy-3,5-diméthylphényl)prop-2-èn-1-one : 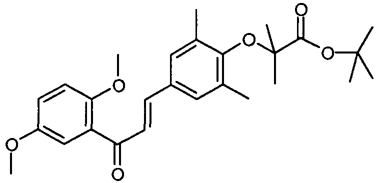
La 1-(2,5-Diméthoxyphényl)-3-(4-carboxydiméthylméthyloxy-3,5-diméthylphényl)prop-2-èn-1-one : 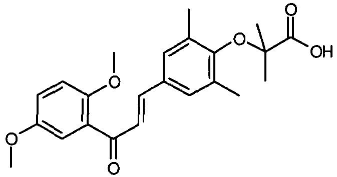
Le chlorhydrate de 1-(3,5-Diméthyl-4-(morpholin-4-yléthyloxy)phényl)-3-(4-ethyloxycarbonyl diméthylméthyloxy-3,5-diméthylphényl)-prop-2-èn-1-one : 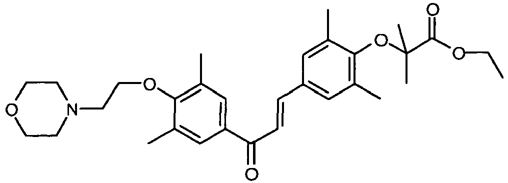
La 1-(3,5-Diméthyl-4-(morpholin-4-yléthyloxy)phényl)-3-(4-carboxydiméthylméthyloxy-3,5-diméthylphényl)-prop-2-èn-1-one : 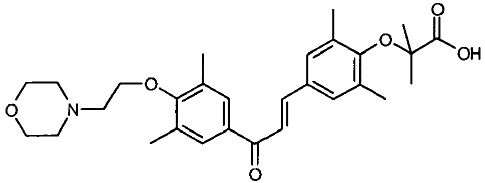
La 1-(4-Bromophényl)-3-(4-tertiobutyloxycarbonyldiméthylméthyloxy-3,5-difluorophényl)-prop-2-èn-1-one : 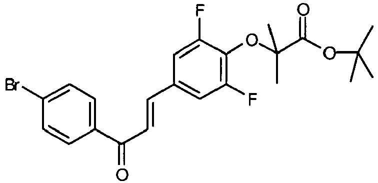
La 1-(4-Bromophényl)-3-(4-carboxydiméthylméthyloxy-3,5-difluorophényl)-prop-2-èn-1-one : 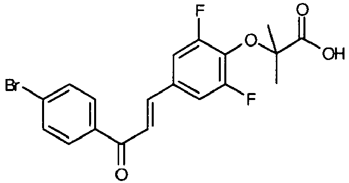
La 1-(4-Méthoxy-3-trifluorométhylphényl)-3-(4-tertiobutyloxycarbonyldiméthylméthyloxy-3,5-diméthylphényl)-prop-2-èn-1-one : 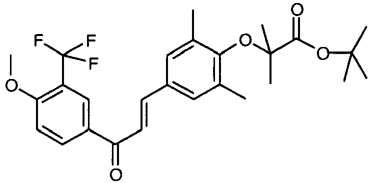
La 1-(4-Méthoxy-3-trifluorométhylphényl)-3-(4-carboxydiméthylméthyloxy-3,5-diméthylphényl)-prop-2-èn-1-one :

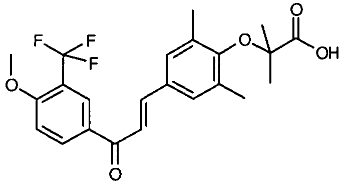

La présente invention a également pour objet un procédé de préparation des composés de formule (I).
Ce procédé de préparation présente de nombreux avantages. Il est simple à mettre en oeuvre industriellement et permet d'obtenir un rendement élevé en composés de formule (I).

Le procédé de la présente invention comprend une mise en contact en milieu basique ou en milieu acide d'au moins un composé de formule (A) avec au moins un composé de formule (B), les formules (A) et (B) étant : 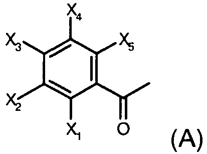 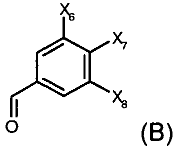 formules dans lesquelles X₁, X₂, X₃, X₄, X₅, X₆, X₇ et X₈ ont les définitions données précédemment, X₇ pouvant également représenter un groupement hydroxyle ou thiol. Les conditions de mise en oeuvre de cette réaction en milieu acide ou basique sont à la portée de l'homme du métier et peuvent varier dans une large mesure.

La mise en contact de ces deux composés est avantageusement réalisée de manière stoechiométrique. Elle est réalisée de préférence à une température ambiante (entre environ 18°C et 25°C) et à pression atmosphérique.

En milieu basique, la réaction est de préférence réalisée en présence d'une base, tel qu'un hydroxyde de métal alcalin, comme l'hydroxyde de sodium ou un alcoolate de métal alcalin comme l'éthylate de sodium.

En milieu acide, la réaction est de préférence réalisée en présence d'un acide fort, tel que l'acide chlorhydrique.

Le schéma réactionnel peut être représenté comme suit :

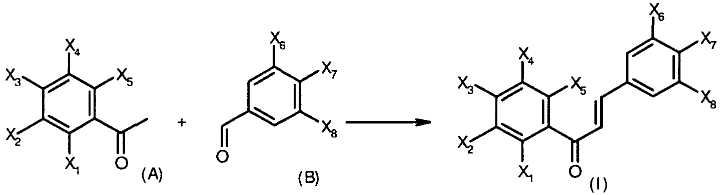

La synthèse en milieu basique peut être réalisée de la façon suivante :
La cétone (composé (A)) à 1 équivalent-molaire et l'aldéhyde (composé (B)) à 1 équivalent-molaire sont solubilisés dans une solution hydroalcoolique d'hydroxyde de sodium à 20 équivalents-molaire. L'ensemble est agité pendant environ 18 heures à température ambiante (entre 18 et 25°C). Le milieu est ensuite acidifié (pour atteindre en particulier un pH d'environ 2), notamment avec de l'acide chlorhydrique.
La 1,3-diphénylprop-2-èn-1-one substituée attendue peut être obtenue par précipitation ou extraction solide/liquide après évaporation du milieu réactionnel. Elle peut être ensuite purifiée par chromatographie sur gel de silice ou par recristallisation.

La synthèse en milieu acide peut être réalisée de la façon suivante :
La cétone (composé (A)) à 1 équivalent-molaire et l'aldéhyde (composé (B)) à 1 équivalent-molaire sont solubilisés dans une solution d'éthanol saturée d'acide chlorhydrique gazeux. L'ensemble est agité à température ambiante pendant environ 6 heures, le solvant est éliminé, notamment par évaporation sous pression réduite.
La 1,3-diphénylprop-2-èn-1-one substituée est purifiée, notamment par chromatographie sur gel de silice.

Le procédé de préparation des composés de formule (I) permet de préparer des composés appelés ci-dessous composés intermédiaires. La présente invention a également pour objet certaines matières premières et composés intermédiaires obtenus dans le cadre de la présente invention.

Ces composés intermédiaires sont plus particulièrement choisis parmi :
■ La 1-(4-(Pentylthioéthyloxy)phényl)-3-(4-hydroxy-3,5-diméthylphényl)prop-2-èn-1-one ;
■ La 1-(4-((R,S)-5-[1,2]dithiolan-3-ylpentyloxy)phényl)-3-(4-hydroxy-3,5-diméthylphényl) -prop-2-èn-1-one ;
■ La 1-(4-Méthylthiophényl)-3-(4-hydroxy-3,5-dibromophényl)prop-2-èn-1-one ;
■ La 1-(4-(Cyclohexyléthyloxy)phényl)-3-(4-hydroxy-3,5-diméthylphényl)prop-2-èn-1-one ;
■ La 1-(4-Méthylthio-3,5-diméthylphényl)-3-(4-hydroxy-3,5-diméthylphényl)prop-2-èn-1-one ;
■ La 1-(4-Méthoxy-3,5-diméthylphényl)-3-(4-hydroxy-3,5-diméthylphényl)prop-2-èn-1-one ;
■ La 1-(4-(Cyclohexyléthyloxy)-3,5-diméthylphényl)-3-(4-hydroxy-3,5-diméthylphényl)prop-2-èn-1-one ;
■ La 1-(4-(Cyclohexylthioéthyloxy)phényl)-3-(4-hydroxy-3,5-diméthylphényl)prop-2-èn-1-one ;
■ La 1-(2,4,5-Triméthylphényl)-3-(4-hydroxy-3,5-diméthylphényl)prop-2-èn-1-one ;
■ La 1-(4-(Cyclohexylthioéthyloxy)-3,5-diméthylphényl)-3-(4-hydroxy-3,5-diméthylphényl)prop-2-èn-1-one ;
■ La 1-(4-Méthylthiophényl)-3-(4-hydroxy-3-fluorophényl)prop-2-èn-1-one ;
■ La 1-(2,3,4,5,6-Pentaméthylphényl)-3-(4-hydroxy-3,5-diméthylphényl)prop-2-èn-1-one ;
■ La 1-(4-Phénoxyphényl)-3-(4-hydroxy-3,5-diméthylphényl)prop-2-èn-1-one ;
■ La 1-(4-Méthoxy-3-fluorophényl)-3-(4-hydroxy-3,5-diméthylphényl)prop-2-èn-1-one ;
■ La 1-(4-Méthoxy-3-méthylphényl)-3-(4-hydroxy-3,5-diméthylphényl)prop-2-èn-1-one ;
■ La 1-(4-Hexylthio-3,5-diméthylphényl)-3-(4-hydroxy-3,5-diméthylphényl)prop-2-èn-1-one ;
■ La 1-(2,5-Diméthoxyphényl)-3-(4-hydroxy-3,5-diméthylphényl)prop-2-èn-1-one ;
■ La 1-(4-Bromophényl)-3-(4-hydroxy-3,5-difluorophényl)-prop-2-èn-1-one ;
■ La 1-(4-Méthoxy-3-trifluorométhylphényl)-3-(4-hydroxy-3,5-diméthylphényl)-prop-2-èn-1-one.

La présente invention a aussi pour objet les composés de formule générale (I) tels que décrits ci-dessus, à titre de médicaments.

La présente invention a également pour objet une composition pharmaceutique et/ou cosmétique comprenant, dans un support acceptable sur le plan pharmaceutique et/ou cosmétique, au moins un composé de formule générale (I) tel que décrit ci-dessus, éventuellement en association avec un autre actif thérapeutique et/ou cosmétique.

Il s'agit avantageusement d'une composition pharmaceutique et/ou cosmétique pour le traitement des maladies cardiovasculaires, des dyslipidémies, des pathologies associées au syndrome X, du diabète, de l'obésité, de l'hypertension, des maladies inflammatoires, des maladies dermatologiques (en particulier psoriasis, dermatites atopiques, acné), de l'asthme, des désordres liés au stress oxydatif, du vieillissement en général et par exemple du vieillissement cutané notamment dans le domaine cosmétique (en particulier l'apparition de rides).

D'autre part, les compositions pharmaceutiques et/ou cosmétiques selon l'invention sont capables d'exercer une activité prophylactique en terme de neuroprotection, et également permettent d'assurer une neuroprotection active dans la phase aiguë des accidents ischémiques cérébraux. Enfin, il s'agit avantageusement d'une composition pharmaceutique et/ou cosmétique pour prévenir et/ou traiter l'apparition de plusieurs facteurs de risque cardiovasculaire liés aux dérèglements du métabolisme lipidique et/ou glucidique (en particulier hyperlipidémie, diabète, obésité) en permettant la diminution du risque global.

L'invention concerne aussi l'utilisation d'au moins un composé de formule (I) pour la préparation d'une composition pharmaceutique et/ou cosmétique destinée à la mise en oeuvre d'une méthode de traitement ou de prophylaxie du corps humain ou animal.

L'invention concerne également une méthode de traitement des pathologies liées au métabolisme des lipides et/ou des glucides comprenant l'administration à un sujet, notamment humain, d'une dose efficace d'un composé ou d'une composition pharmaceutique tels que définis ci-avant.

Les compositions pharmaceutiques selon l'invention comprennent avantageusement un ou plusieurs excipients ou véhicules, acceptables sur le plan pharmaceutique. On peut citer par exemple des solutions, en particulier salines, physiologiques, isotoniques, tamponnées., compatibles avec un usage pharmaceutique et connues de l'homme du métier. Les compositions peuvent contenir un ou plusieurs agents ou véhicules choisis en particulier parmi les dispersants, solubilisants, stabilisants, conservateurs. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, ou l'acacia. Les compositions peuvent être formulées par exemple sous forme de suspensions injectables, gels, huiles, comprimés, suppositoires, poudres, gélules, capsules, éventuellement au moyen de formes galéniques ou de dispositifs assurant une libération prolongée et/ou retardée. Pour ce type de formulation, on utilise avantageusement un agent tel que la cellulose, des carbonates ou des amidons.

Les composés ou compositions selon l'invention peuvent être administrés de différentes manières et sous différentes formes. Ainsi, ils peuvent être injectés par voie orale ou systémique, comme par exemple par voie intraveineuse, intramusculaire, sous-cutanée, trans-dermique, ou intra-artérielle. Pour les injections, les composés sont généralement conditionnés sous forme de suspensions liquides, qui peuvent être injectées au moyen de seringues ou de perfusions, par exemple. Il est entendu que le débit et/ou la dose injectée peuvent être adaptés par l'homme du métier en fonction en particulier du patient, de la pathologie, du mode d'administration. Typiquement, les composés sont administrés à des doses pouvant varier entre 1 µg et 2g /administration, préférentiellement de 0,1 mg à 1 g /administration. Les administrations peuvent être quotidiennes ou répétées plusieurs fois par jour, le cas échéant. D'autre part, les compositions selon l'invention peuvent comprendre, en outre, d'autres agents ou principes actifs.

### LEGENDE DES FIGURES :

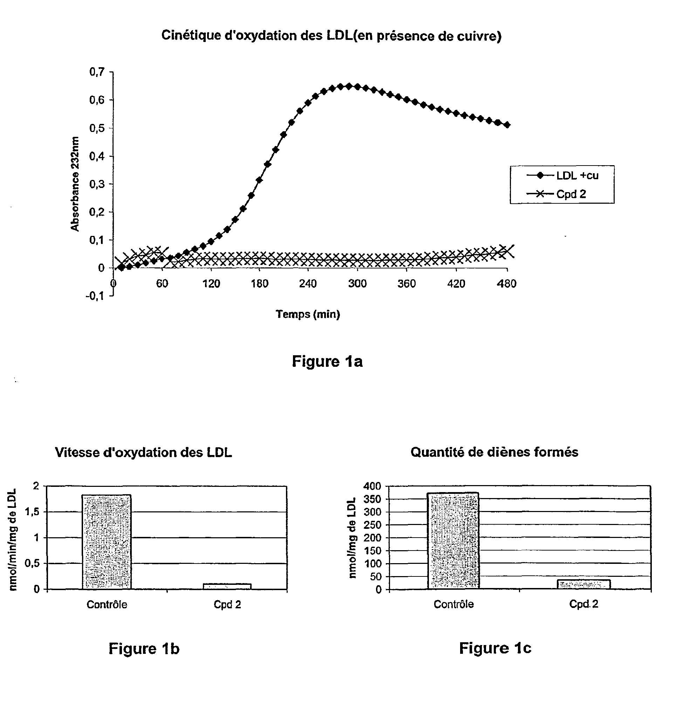

**Les** **Figures 1a, 1b, 1c** illustrent le caractère antioxydant du composé 2 (Cpd 2) selon l'invention testé à la concentration de 10⁻⁴M.

La figure 1a représente la cinétique de formation de diènes conjugués au cours du temps. La Lag-Phase est de 120 minutes lorsque les LDL sont incubées avec le cuivre seul. Ce délai est de 314 minutes lorsque le milieu contient également le composé 2.

La figure 1b illustre la vitesse de formation des diènes. Celle-ci est de 1,8 nmol/min/mg de LDL en présence de cuivre seul, elle n'est plus que de 0,1 nmol/min/mg de LDL lorsque le composé 2 est présent dans le milieu.

La figure 1c représente la quantité maximale de diènes formés au cours de l'expérience. Le cuivre seul induit la formation de 372 nmol/mg de diènes conjugués, cette quantité est de 35 nmol/mg quand le milieu contient également le composé 2, ce qui représente une diminution de 90% de la quantité de diènes formés.

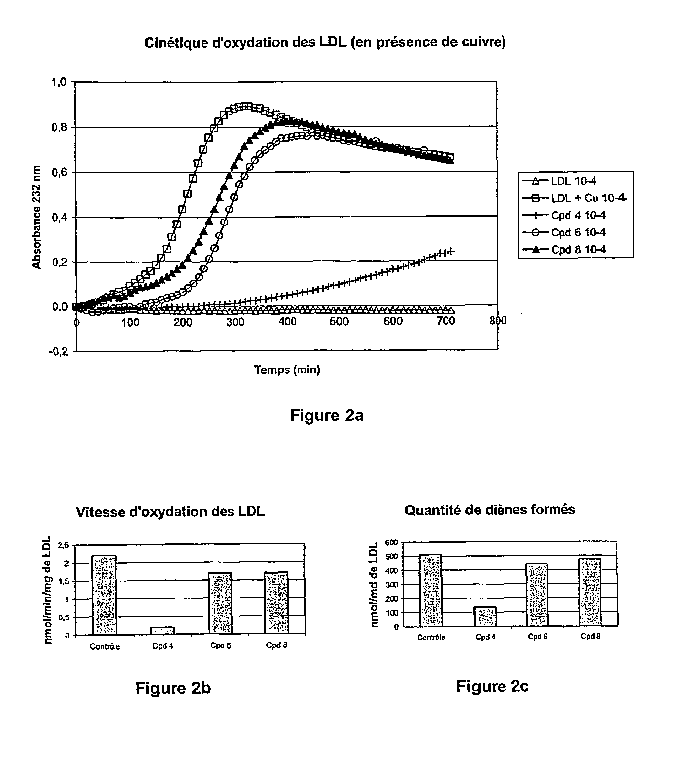

**Les** **Figures 2a, 2b, 2c** illustrent le caractère antioxydant des composé 4 (Cpd 4), composé 6 (Cpd 6) et composé 8 (Cpd 8) selon l'invention testés à la concentration de 10⁻⁴M.

La figure 2a représente la cinétique de formation de diènes conjugués.

La Lag-Phase est de 132 minutes lorsque les LDL sont incubées avec le cuivre seul. La valeur de la lag phase est respectivement de 401, 205 et 169 minutes en présence des composés 4, 6 et 8.

La figure 2b représente le calcul de la vitesse de formation des diènes. La vitesse de formation des diènes conjugués par le cuivre est de 2,2 nmol/min/mg de LDL. La présence des composés 4, 6 et 8 provoque une diminution de la vitesse de la réaction d'oxydation des diènes. Elle est de 0,2 nmol/min/mg en présence du composé 4 et de 1,7 nmol/min/mg en présence du composé 6 ou du composé 8.

La quantité totale de diènes formés (figure 2c) est de 511 nmol/mg de LDL en présence de cuivre seul. En présence des composés 4, 6 et 8, cette quantité passe à 138, 443 et 474 nmol/mg respectivement.

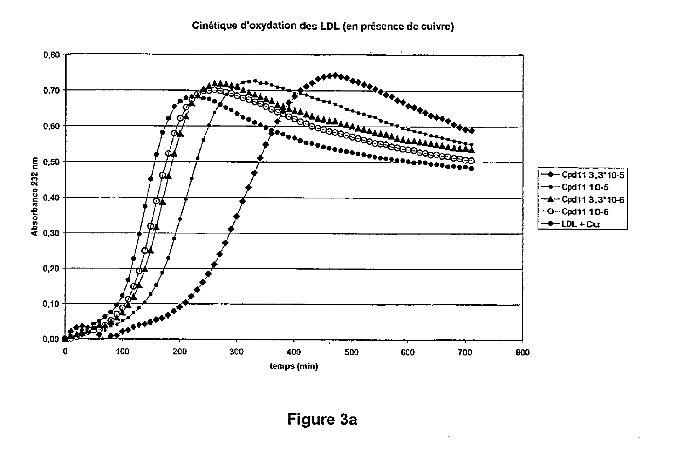

**La** **Figure 3a** illustre le caractère antioxydant du composé 11 (Cpd 11) selon l'invention.

Le caractère antioxydant du composé 11 a été mis en évidence pour différentes concentrations comprises entre 10⁻⁶ M et 3,5.10⁻⁵ M.

En l'absence du composé 11, la lag-phase est de 87,2 minutes. Dés la concentration de 10⁻⁶ M, on note une augmentation de la lag phase par rapport au contrôle : La lag phase est de 101,5 minutes en présence du composé 11 à la concentration de 10⁻⁶ M. Elle augmente avec la concentration en composé dans le milieu pour atteindre la valeur maximale observée : 210 minutes à la concentration de 3,3.10⁻⁵M.

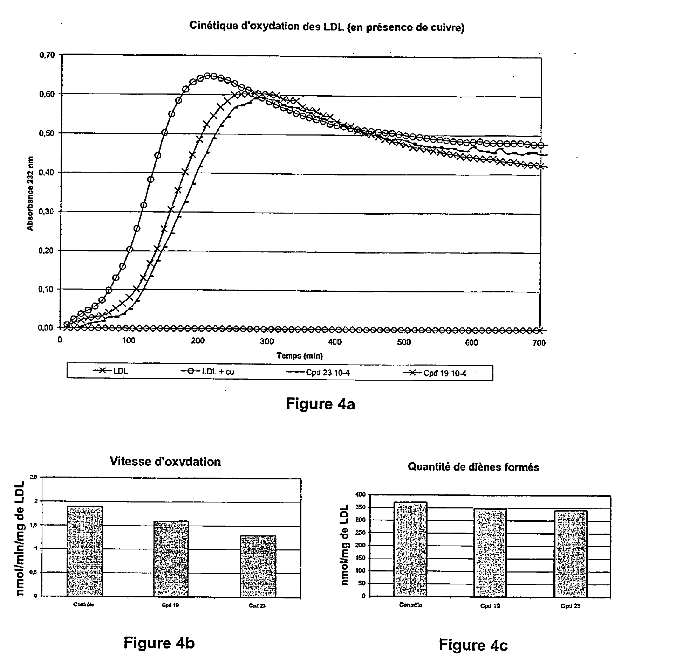

**Les** **Figures 4a, 4b, 4c** illustrent le caractère antioxydant des composé 19 (Cpd 19) et composé 23 (Cpd 23) selon l'invention testés à la concentration de 10⁻⁴M.

La figure 4a représente la cinétique de formation de diènes conjugués. La Lag-Phase est de 61 minutes en présence de cuivre seul. En présence du composé 19, la lag phase est de 92,5 minutes. Elle est de 96,4 minutes en présence du composé 23.

Le caractère antioxydant des composés 19 et 23 se traduit également par une diminution de la vitesse de formation des diènes et par une diminution de la quantité totale des diènes formés.

En l'absence de composé, la vitesse de formation des diènes est de 1,9 nmol/min/mg de LDL (figure 4b). Elle est respectivement de 1,6 et 1,3 nmol/min/mg de LDL en présence des composés 19 et 23. En l'absence de composé, la quantité totale de diènes formés est de 370,9 nmol/mg de LDL (figure 4c). Elle est respectivement de 346,6 et 340,3 nmol/ mg de LDL en présence des composés 19 et 23.

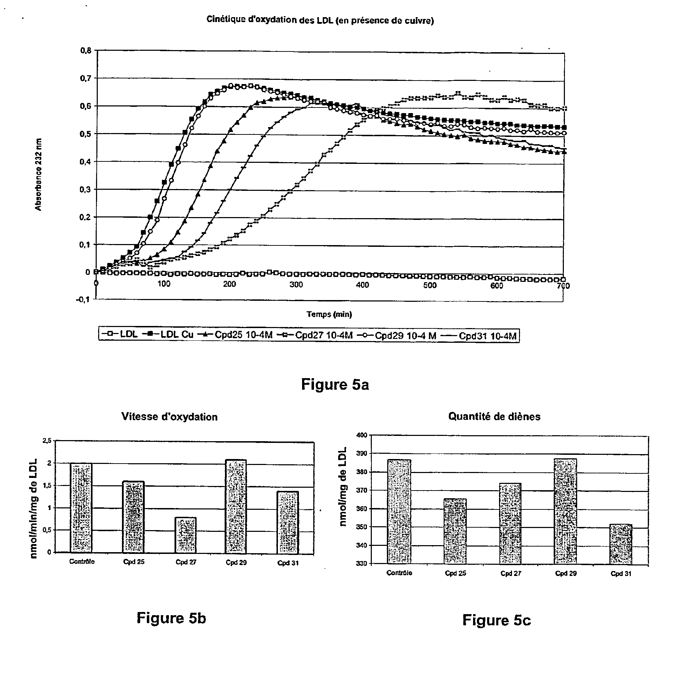

**Les** **Figures 5a, 5b, 5c** illustrent le caractère antioxydant des composé 25 (Cpd 25), composé 27 (Cpd 27), composé 29 (Cpd 29) et composé 31 (Cpd 31) selon l'invention testés à la concentration de 10⁻⁴M.

La Figure 5a montre la cinétique d'oxydation des LDL en présence des différents composés : elle augmente en présence des différents composés antioxydants.

Elle est de 54,9 minutes en présence du composé 29. Elle passe à 87,6 minutes en présence du composé 25, 124,5 minutes en présence du composé 31 et atteint 170,8 minutes en présence du composé 27.

Le caractère antioxydant de ces composés est également illustré par la vitesse d'oxydation des LDL (figure 5b et par la mesure de la quantité totale de diènes formés (figure 5c).

La vitesse d'oxydation des LDL est de 2 nmol/min/mg de LDL en l'absence de composés (figure 5b). La présence des composés induit une diminution de la vitesse d'oxydation. La vitesse est de 1,6 nmol/min/mg en présence du composé 25 et de 1,4 nmol/min/mg en présence du composé 31. Cette vitesse est minimale en présence du composé 27 et atteint 0,8 nmol/min/mg.

La quantité totale de diènes formés est de 386 nmol/mg de LDL en l'absence de composés (figure 5c). Elle est de 374 nmol/mg en présence du composé 27, elle est de 365 nmol/mg en présence du composé 25 et de 352 nmol/mg en présence du composé 31.

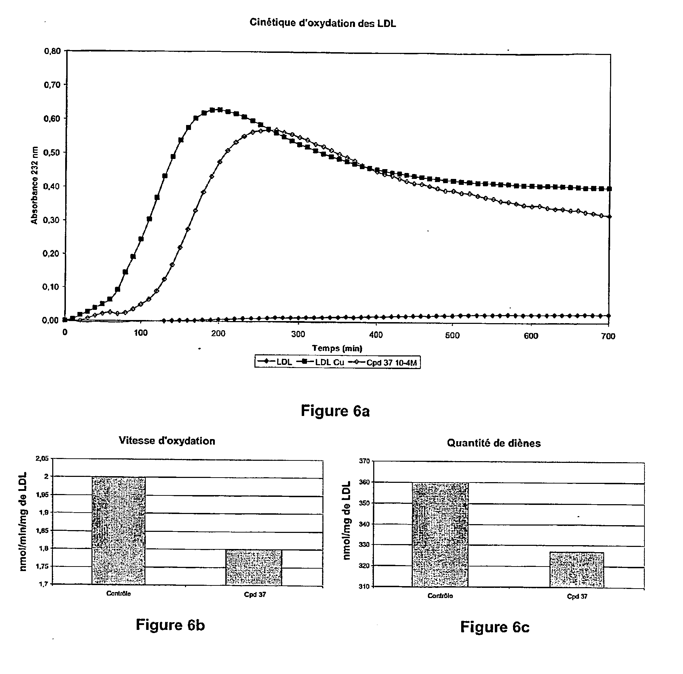

**Les** **Figures 6a, 6b, 6c** illustrent le caractère antioxydant du composé 37 (Cpd 37) selon l'invention testé à la concentration de 10⁻⁴M.

La Figure 6a montre la cinétique d'oxydation des LDL. La présence du composé dans le milieu se traduit par une augmentation de la lag-phase. Elle atteint 106 minutes en présence du composé 37 alors qu'elle n'est que de 56 minutes en l'absence de composé.

La diminution de la vitesse d'oxydation des LDL et la diminution de la quantité de diènes formés témoignent également du caractère antioxydant du composé testé. En l'absence de composé, la vitesse d'oxydation est de 2 nmol/min/mg de LDL, en présence du composé, elle est de 1,8 nmol/min/mg de LDL (figure 6b). En l'absence de composé, la quantité totale de diènes formés est de 360,0 nmol/mg de LDL. En présence du composé 37, elle n'est plus que de 326,9 nmol/mg de LDL (figure 6c).

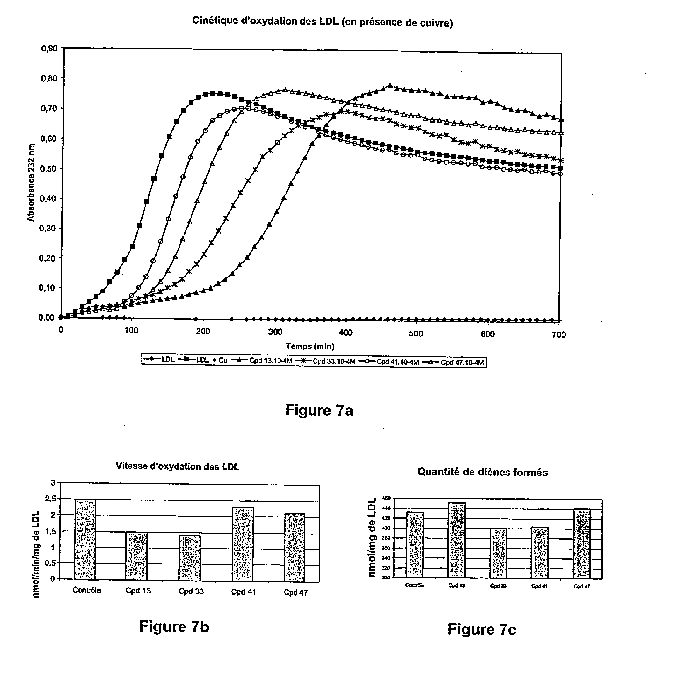

**Les** **Figures 7a, 7b, 7c** illustrent le caractère antioxydant des composé 13 (Cpd 13), composé 33 (Cpd 33), composé 41 (Cpd 41), composé 47 (Cpd 47), selon l'invention testés à la concentration de 10⁻⁴M.

La figure 7a représente la cinétique d'oxydation des LDL. En l'absence de composés antioxydants, la lag-phase est de 67,3 minutes. Elle augmente en présence des différents composés. Cette valeur est de 100 minutes en présence du composé 41, 126,5 minutes en présence du composé 47, 148 minutes en présence du composé 33 et 219 minutes en présence du composé 13.

La présence des composés dans le milieu a également une influence sur la vitesse d'oxydation des LDL et la quantité totale des diènes formés.

Les composés 13 et 33 provoquent une diminution importante de la vitesse d'oxydation des diènes (figure 7b). La vitesse d'oxydation des diènes est de 2,5 nmol/min/mg de LDL en l'absence de composés. Elle est respectivement de 1,5 et 1,4 nmol/min/mg de LDL en présence des composés 13 et 33.

Seuls les composés 33 et 41 provoquent une diminution de la quantité totale de diènes formées (figure 7c). La quantité totale de diènes formés est de 432,5 nmol/mg de LDL en l'absence de composé. Elle est de 399 nmol/mg de LDL en présence du composé 33 et de 403 nmol/mg de LDL en présence du composé 41.

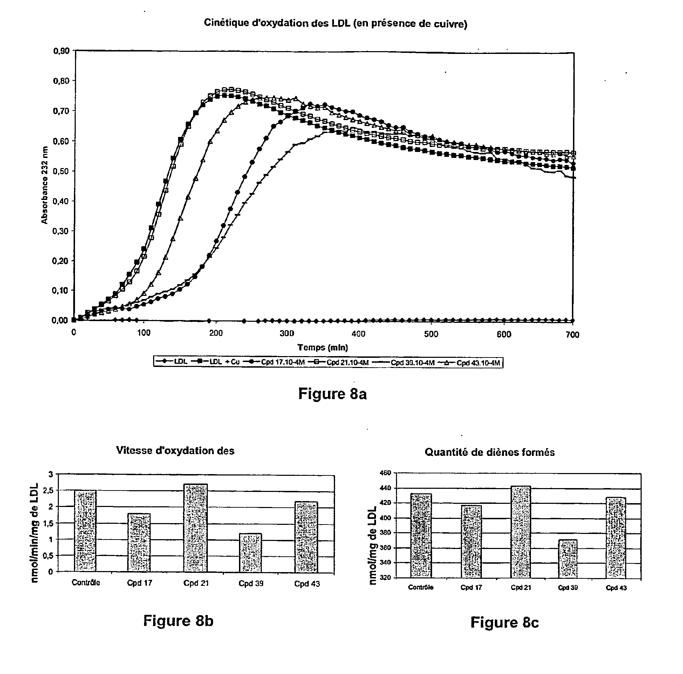

**Les** **Figures 8a, 8b, 8c** illustrent le caractère antioxydant des composé 17 (Cpd 17), composé 21 (Cpd 21), composé 39 (Cpd 39) et composé 43 (Cpd 43) selon l'invention testés à la concentration de 10⁻⁴M.

La cinétique d'oxydation des LDL est représentée par la figure 8a.

En l'absence de composé, la lag phase est de 67,3 minutes. Elle augmente en présence des composés 17, 39 et 43. En présence du composé 43, la lag phase est de 97 minutes. En présence du composé 17, elle est de 148 minutes et en présence du composé 39, elle est de 133 minutes.

La figure 8b représente la vitesse d'oxydation des LDL. En l'absence de composé elle est de 2,5 nmol/min/mg. En présence du composé 17, elle est de 1,8 nmol/min/mg En présence du composé 39, elle est de 1,2 nmol/min/mg et en présence du composé 43, elle est de 2,2 nmol/min/mg.

La figure 8c représente la quantité totale de diènes formés au cours de l'oxydation. Seul le composé 39 induit une diminution significative de la quantité totale des diènes formés. Cette quantité est de 432,3 nmol/mg en l'absence de composé et elle est de 371,2 nmol/mg en présence du composé 39.

Le retard de formation de diènes conjugués, la diminution de la vitesse de formation des diènes et la diminution de la quantité totale de diènes formés sont trois paramètres qui attestent du caractère antioxydant des composés selon l'invention.

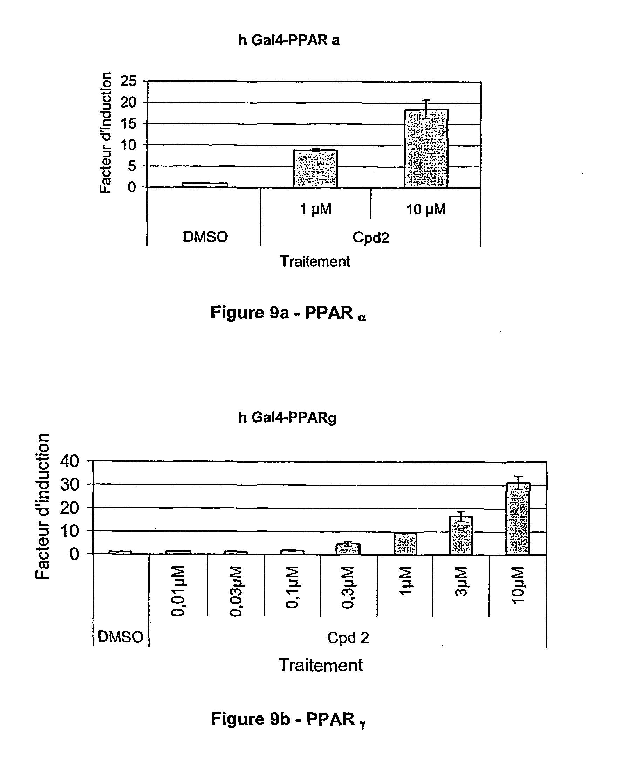

**Les** **Figures 9a et 9b** illustrent l'évaluation des propriétés d'agoniste PPARα et PPARγ des composés selon l'invention avec le système de transactivation PPARα/Gal4 et PPARγ/Gal4 dans les cellules RK13.

Les cellules RK13 sont incubées avec du composé 2 à des concentrations comprises entre 0,01 et 10 µM pendant 24h. Les résultats sont représentés par le facteur d'induction (Rapport entre le signal luminescent obtenu avec le composé et le signal luminescent obtenu sans composé) en fonction des différents traitements. Plus le facteur d'induction est élevé, meilleure est la propriété d'agoniste pour PPARα ou PPARγ.

Les résultats présentés par la figure 9a montrent les facteurs d'induction du composé 2 avec le système de transactivation PPARα/Gal4. Les valeurs de ces facteurs d'induction sont notées dans le tableau suivant.

| Composé | Traitement | Facteur d'induction |
|---|---|---|
| Cpd 2 | 1µM | 8,83 |
| | 10µM | 18,49 |

Le facteur d'induction mesuré avec le composé 2 est maximal pour la dose de 10µM et atteint la valeur de 18,49.

Les résultats présentés par la figure 9b montrent les facteurs d'induction du composé 2 avec le système de transactivation PPARγ/Gal4. Les valeurs de ces facteurs d'induction sont notées dans le tableau suivant :

| Composé | Traitement | Facteur d'induction |
|---|---|---|
| Cpd 2 | 0,01µM | 1,31 |
| | 0,03µM | 1,18 |
| | 0,1µM | 1,73 |
| | 0.3µM | 4,58 |
| | 1µM | 9,50 |
| | 3µM | 16,64 |
| | 10µM | 31,00 |

Pour ce qui concerne le système PPARγ/Gal4, le facteur d'induction varie de 1,31 à 31,00, il augmente avec la concentration en composé 2 dans le milieu.

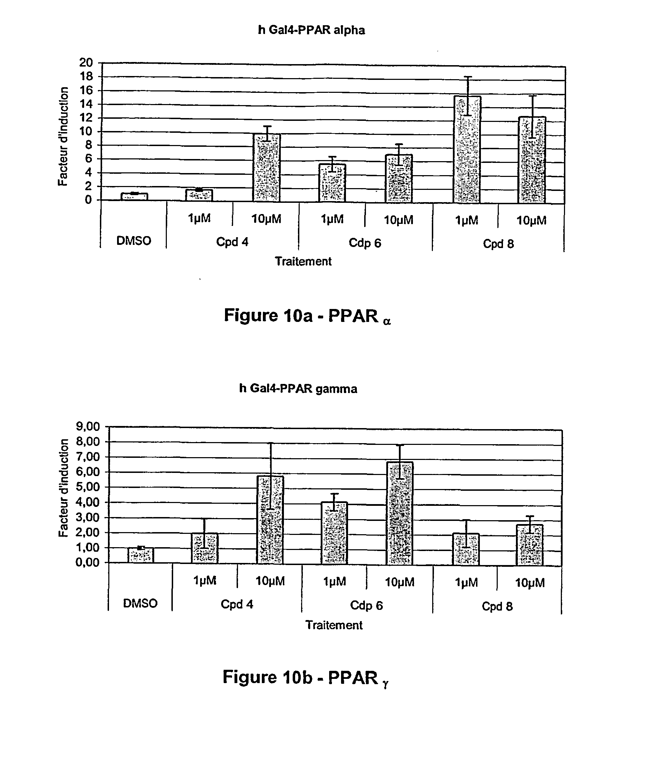

**Les** **Figures 10a 10b****,** **11a, 11b****,** **11c****,** **12a****,** **12b****,** **13a****,** **13b****,** **14a, 14b, 14c****,** **15a, 15b, 15c****,** **16a, 17a****,** **18a, 18b** illustrent l'évaluation des propriétés d'agoniste PPARα, PPARγ et PPARδ des composés selon l'invention avec le système de transactivation PPARα/Gal4, PPARγ/Gal4 et PPARδ/Gal4 dans les cellules COS-7.

Les cellules COS-7 sont incubées avec les composés selon l'invention à différentes concentrations pendant 24h. Les résultats sont représentés par le facteur d'induction (Rapport entre le signal luminescent obtenu avec le composé et le signal luminescent obtenu sans composé) en fonction des différents traitements.

Les figures 10a et 10b illustrent les facteurs d'induction des composé 4 (Cpd4), composé 6 (Cpd6) et composé 8 (Cpd8) selon l'invention.

Les résultats présentés par la figure 10a montrent les facteurs d'induction des composé 4 (Cpd4), composé 6 (Cpd6) et composé 8 (Cpd8) avec le système de transactivation PPARα/Gal4. Les valeurs de ces facteurs d'induction sont notées dans le tableau suivant :

| Composé | Traitement | Facteur d'induction |
|---|---|---|
| Cpd 4 | 1µM | 1,67 |
| | 10µM | 9,92 |
| Cpd 6 | 1µM | 5,48 |
| | 10µM | 7,01 |
| Cpd 8 | 1µM | 15,67 |
| | 10µM | 12,66 |

Le facteur d'induction maximal mesuré pour le composé 4 est de 9,92 à 10µM. Cette valeur est de 7,01 pour le composé 6 (10µM) et de 15,67 avec le composé 8 (1µM).

Les résultats présentés par la figure 10b montrent les facteurs d'induction des composé 4, composé 6 et composé 8 avec le système de transactivation PPARγ/Gal4. Les valeurs de ces facteurs d'induction sont notées dans le tableau suivant :

| Composé | Traitement | Facteur d'induction |
|---|---|---|
| Cpd 4 | 1µM | 2,00 |
| | 10µM | 5,82 |
| Cpd 6 | 1µM | 4,12 |
| | 10µM | 6,83 |
| Cpd 8 | 1µM | 2,13 |
| | 10µM | 2,74 |

Le composé 4 a un facteur d'induction maximal de 5,82, il est observé pour une concentration de 10µM.

La valeur maximale du facteur d'induction observée avec le composé 6 est de 6,83 (10µM) avec le composé 6 et 2,74 avec le composé 8 (10µM).

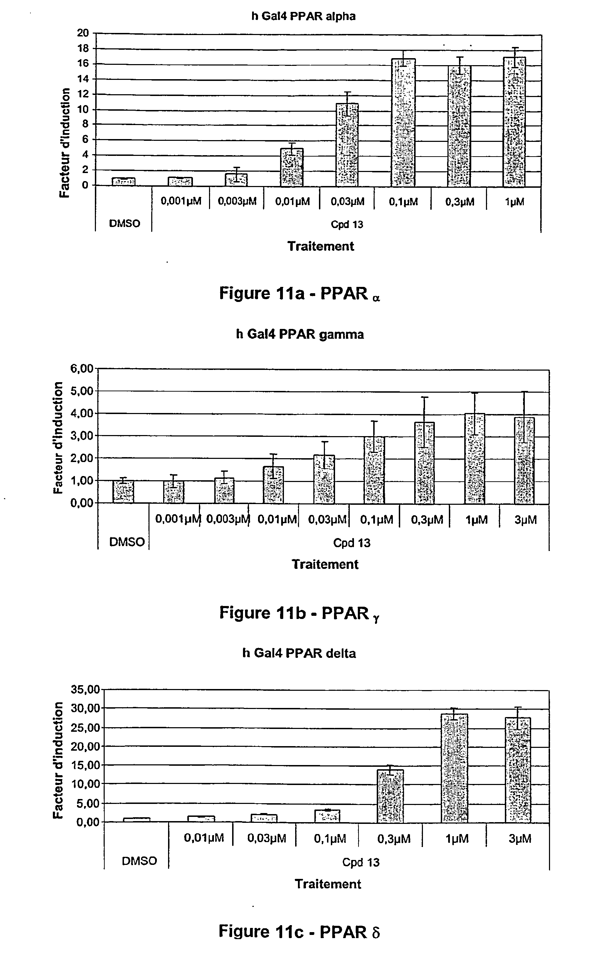

Les figures 11a, 11b et 11c illustrent les facteurs d'induction du composé 13 selon l'invention (Cpd13).

La figure 11a illustre les facteurs d'induction du composé 13 avec le système de transactivation PPARα/Gal4. Les valeurs de ces facteurs d'induction sont notées dans le tableau suivant.

| Composé | Traitement | Facteur d'induction |
|---|---|---|
| Cpd 13 | 0,001µM | 1,10 |
| | 0,003µM | 1,58 |
| | 0,01µM | 4,99 |
| | 0,03µM | 10,89 |
| | 0,1µM | 16,87 |
| | 0,3µM | 15,95 |
| | 1µM | 17,05 |

La valeur maximale est 17,05, elle est observée à la concentration de 1µM.

La figure 11 b illustre les facteurs d'induction du composé 13 avec le système de transactivation PPARγ/Gal4. Les valeurs de ces facteurs d'induction sont notées dans le tableau suivant.

| Composé | Traitement | Facteur d'induction |
|---|---|---|
| Cpd 13 | 0,001µM | 0,99 |
| | 0,003µM | 1,15 |
| | 0,01µM | 1,67 |
| | 0,03µM | 2,18 |
| | 0,1µM | 3,01 |
| | 0,3µM | 3,66 |
| | 1µM | 4,03 |
| | 3µM | 3,89 |

La valeur maximale est de 4,03, elle est observée pour la concentration de 1µM.

La figure 11 c illustre les facteurs d'induction du composé 13 avec le système de transactivation PPARδ/Gal4. Les valeurs de ces facteurs d'induction sont notées dans le tableau suivant.

| Composé | Traitement | Facteur d'induction |
|---|---|---|
| Cpd 13 | 0,01µM | 1,50 |
| | 0,03µM | 2,17 |
| | 0,1µM | 3,37 |
| | 0,3µM | 14,00 |
| | 1µM | 28,75 |
| | 3µM | 27,72 |

La valeur maximale est observée pour la concentration de 1µM, elle est de 28,75.

Les Figures 12a et 12b illustrent les facteurs d'induction du composé 23 selon l'invention (Cpd23).

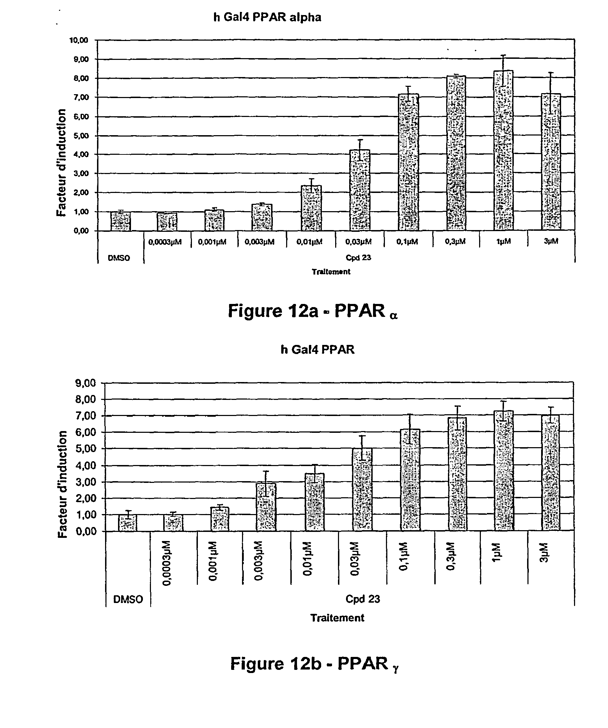

La figure 12a illustre les facteurs d'induction du composé 23 avec le système de transactivation PPARα/Gal4. Les valeurs de ces facteurs d'induction sont notées dans le tableau suivant.

| Composé | Traitement | Facteur d'induction |
|---|---|---|
| Cpd 23 | 0,0003µM | 0,92 |
| | 0,001µM | 1,11 |
| | 0,003µM | 1,33 |
| | 0,01µM | 2,35 |
| | 0,03µM | 4,22 |
| | 0,1µM | 7,16 |
| | 0,3µM | 8,08 |
| | 1µM | 8,35 |
| | 3µM | 7,15 |

La valeur maximale est 8,35, elle est observée dès la concentration de 1µM.

La figure 12b illustre les facteurs d'induction du composé 23 avec le système de transactivation PPARγ/Gal4. Les valeurs de ces facteurs d'induction sont notées dans le tableau suivant.

| Composé | Traitement | Facteur d'induction |
|---|---|---|
| Cpd 23 | 0,0003µM | 1,02 |
| | 0,001µM | 1,43 |
| | 0,003µM | 2,86 |
| | 0,01µM | 3,48 |
| | 0,03µM | 5,04 |
| | 0,1µM | 6,17 |
| | 0,3µM | 6,84 |
| | 1µM | 7,24 |
| | 3µM | 6,98 |

La valeur maximale est 7,24, elle est observée dès la concentration de 1µM.

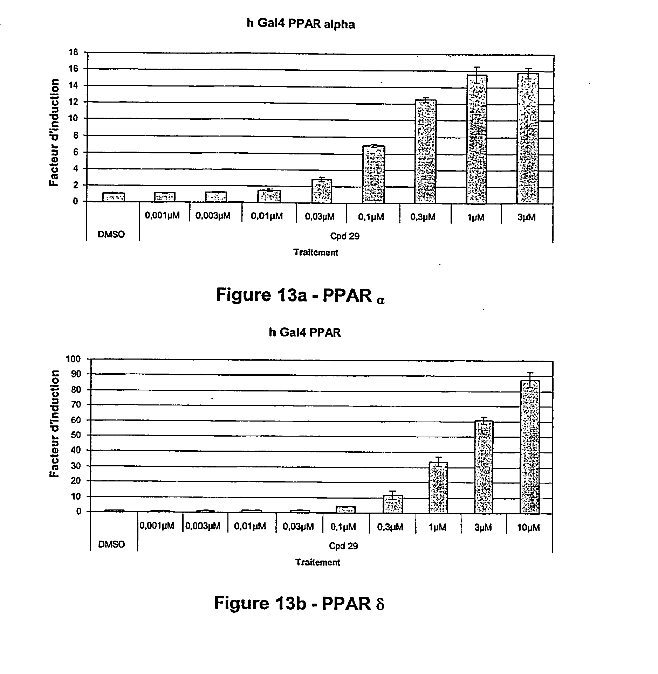

Les Figures 13a et 13b illustrent les facteurs d'induction du composé 29 selon l'invention (Cpd29).

La figure 13a illustre les facteurs d'induction du composé 29 avec le système de transactivation PPARα/Gal4. Les valeurs de ces facteurs d'induction sont notées dans le tableau suivant.

| Composé | Traitement | Facteur d'induction |
|---|---|---|
| Cpd 29 | 0,001µM | 1,09 |
| | 0,003µM | 1,20 |
| | 0,01µM | 1,49 |
| | 0,03µM | 2,85 |
| | 0,1µM | 6,93 |
| | 0,3µM | 12,51 |
| | 1µM | 15,56 |
| | 3µM | 15,75 |

La valeur maximale est 15,75, elle est observée à la concentration de 3µM.

La figure 13b illustre les facteurs d'induction du composé 29 avec le système de transactivation PPARδ/Gal4. Les valeurs de ces facteurs d'induction sont notées dans le tableau suivant.

| Composé | Traitement | Facteur d'induction |
|---|---|---|
| Cpd 29 | 0,001µM | 1,03 |
| | 0,003µM | 1,07 |
| | 0,01µM | 1,26 |
| | 0,03µM | 1,63 |
| | 0,1µM | 4,07 |
| | 0,3µM | 11,61 |
| | 1µM | 33,78 |
| | 3µM | 60,81 |
| | 10µM | 87,56 |

La valeur maximale est 87,56, elle est observée à la concentration de 10µM.

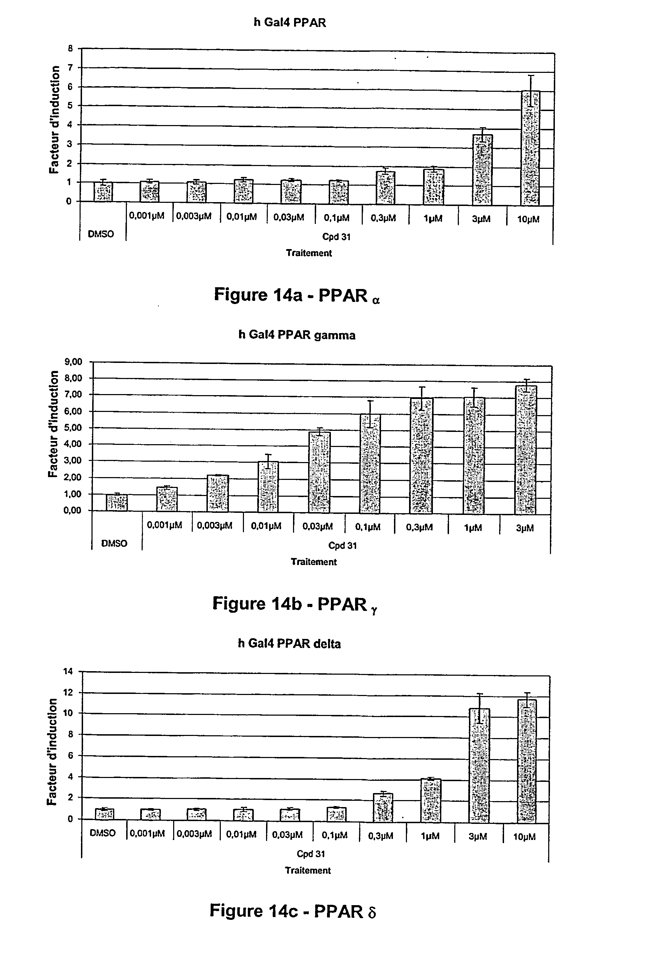

Les Figures 14a, 14b et 14c illustrent les facteurs d'induction du composé 31 selon l'invention (Cpd31).

La figure 14a illustre les facteurs d'induction du composé 31 avec le système de transactivation PPARα/Gal4. Les valeurs de ces facteurs d'induction sont notées dans le tableau suivant.

| Composé | Traitement | Facteur d'induction |
|---|---|---|
| Cpd 31 | 0,001µM | 1,10 |
| | 0,003µM | 1,09 |
| | 0,01µM | 1,23 |
| | 0,03µM | 1,23 |
| | 0,1µM | 1,23 |
| | 0,3µM | 1,73 |
| | 1µM | 1,88 |
| | 3µM | 3,69 |
| | 10µM | 6,03 |

La valeur maximale est 6,03, elle est observée pour la concentration de 10µM.

La figure 14b illustre les facteurs d'induction du composé 31 avec le système de transactivation PPARγ/Gal4. Les valeurs de ces facteurs d'induction sont notées dans le tableau suivant.

| Composé | Traitement | Facteur d'induction |
|---|---|---|
| Cpd 31 | 0,001µM | 1,44 |
| | 0,003µM | 2,19 |
| | 0,01µM | 3,06 |
| | 0,03µM | 4,87 |
| | 0,1µM | 5,99 |
| | 0,3µM | 6,96 |
| | 1µM | 7,05 |
| | 3µM | 7,79 |

La valeur maximale est 7,79, elle est observée pour la concentration de 3µM.

La figure 14c illustre les facteurs d'induction du composé 31 avec le système de transactivation PPARδ/Gal4. Les valeurs de ces facteurs d'induction sont notées dans le tableau suivant.

| Composé | Traitement | Facteur d'induction |
|---|---|---|
| Cpd 31 | 0,001µM | 1,00 |
| | 0,003µM | 1,05 |
| | 0,01µM | 1,09 |
| | 0,03µM | 1,16 |
| | 0,1µM | 1,35 |
| | 0,3µM | 2,67 |
| | 1µM | 4,12 |
| | 3µM | 10,82 |
| | 10µM | 11,70 |

La valeur maximale est 11,70, elle est observée pour la concentration de 10µM.

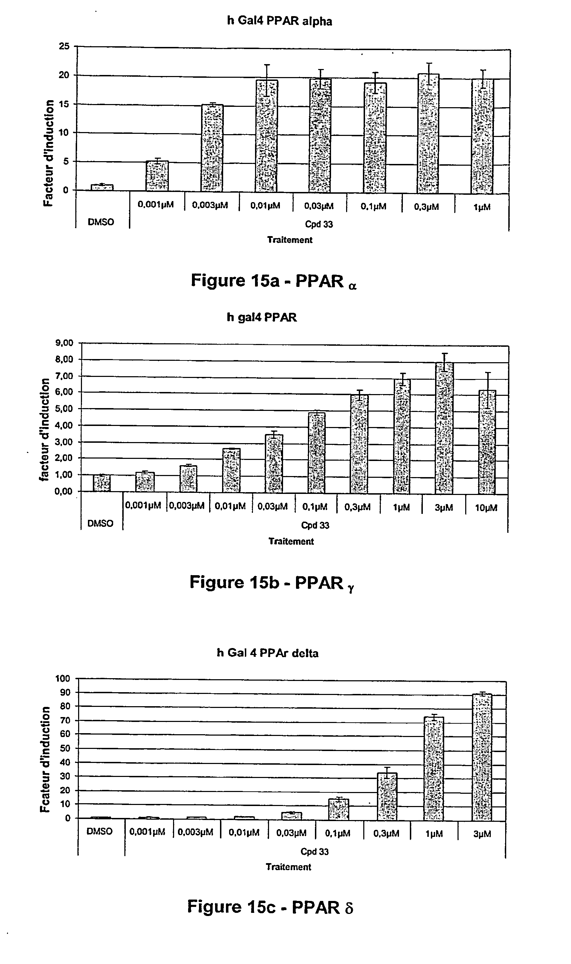

Les Figures 15a, 15b et 15c illustrent les facteurs d'induction du composé 33 selon l'invention (Cpd33).

La figure 15a illustre les facteurs d'induction du composé 33 avec le système de transactivation PPARα/Gal4. Les valeurs de ces facteurs d'induction sont notées dans le tableau suivant.

| Composé | Traitement | Facteur d'induction |
|---|---|---|
| Cpd 33 | 0,001µM | 5,23 |
| | 0,003µM | 15,18 |
| | 0,01µM | 19,53 |
| | 0,03µM | 19,71 |
| | 0,1µM | 19,17 |
| | 0,3µM | 20,82 |
| | 1µM | 19,97 |

La valeur maximale est 20,82, elle est observée dès la concentration de 0,3µM.

La figure 15b illustre les facteurs d'induction du composé 33 avec le système de transactivation PPARγ/Gal4. Les valeurs de ces facteurs d'induction sont notées dans le tableau suivant.

| Composé | Traitement | Facteur d'induction |
|---|---|---|
| Cpd 33 | 0,001µM | 1,18 |
| | 0,003µM | 1,61 |
| | 0,01µM | 2,65 |
| | 0,03µM | 3,54 |
| | 0,1µM | 4,88 |
| | 0,3µM | 5,95 |
| | 1µM | 6,93 |
| | 3µM | 7,99 |
| | 10µM | 6,30 |

La valeur maximale est 7,99, elle est observée dès la concentration de 3µM.

La figure 15c illustre les facteurs d'induction du composé 33 avec le système de transactivation PPARδ/Gal4. Les valeurs de ces facteurs d'induction sont notées dans le tableau suivant.

| Composé | Traitement | Facteur d'induction |
|---|---|---|
| Cpd 33 | 0,001µM | 1,17 |
| | 0,003µM | 1,23 |
| | 0,01µM | 1,87 |
| | 0,03µM | 5,29 |
| | 0,1µM | 15,01 |
| | 0,3µM | 33,89 |
| | 1µM | 74,09 |
| | 3µM | 90,84 |

La valeur maximale est 90,84, elle est observée dès la concentration de 3µM.

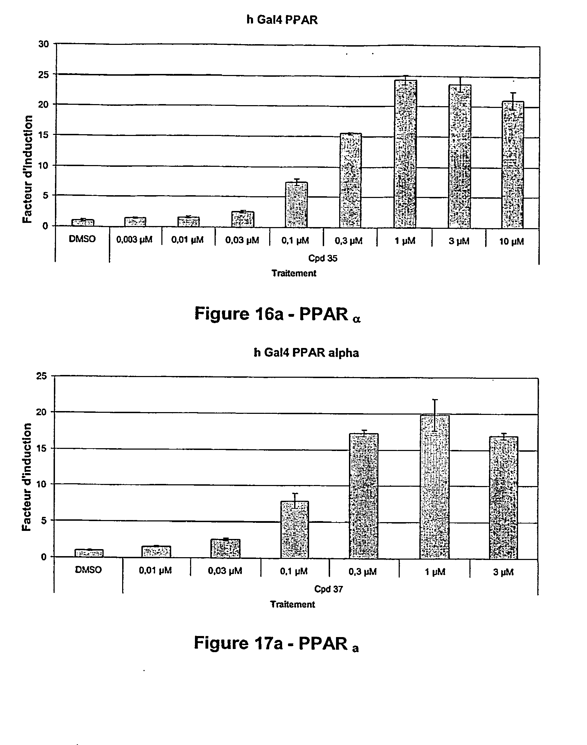

La Figure 16a illustre les facteurs d'induction du composé 35 selon l'invention (Cpd35).

La figure 16a illustre les facteurs d'induction du composé 35 avec le système de transactivation PPARα/Gal4. Les valeurs de ces facteurs d'induction sont notées dans le tableau suivant.

| Composé | Traitement | Facteur d'induction |
|---|---|---|
| Cpd 35 | 0,003 µM | 1,41 |
| | 0,01 µM | 1,54 |
| | 0,03 µM | 2,53 |
| | 0,1 µM | 7,47 |
| | 0,3 µM | 15,51 |
| | 1µM | 24,33 |
| | 3µM | 23,70 |
| | 10 µM | 21,03 |

La valeur maximale est 24,33, elle est observée dès la concentration de 1µM.

La Figure 17a illustre les facteurs d'induction du composé 37 selon l'invention (Cpd37) avec le système de transactivation PPARα/Gal4.

Les valeurs de ces facteurs d'induction sont notées dans le tableau suivant.

| Composé | Traitement | Facteur d'induction |
|---|---|---|
| Cpd 37 | 0,01µM | 1,54 |
| | 0,03µM | 2,54 |
| | 0,1µM | 7,89 |
| | 0,3µM | 17,25 |
| | 1µM | 19,77 |
| | 3µM | 16,89 |

La valeur maximale est 19,77, elle est observée dès la concentration de 1µM.

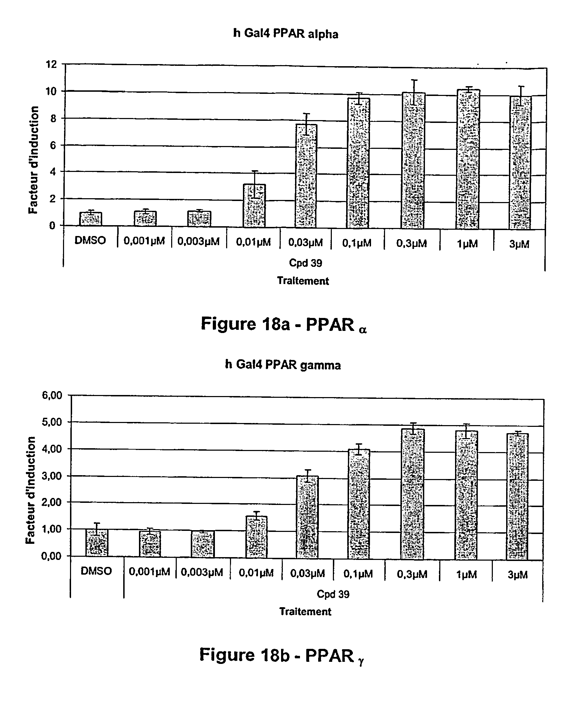

Les Figures 18a et 18b illustrent les facteurs d'induction du composé 39 selon l'invention (Cpd39).

La figure 18a illustre les facteurs d'induction du composé 39 avec le système de transactivation PPARα/Gal4. Les valeurs de ces facteurs d'induction sont notées dans le tableau suivant.

| Composé | Traitement | Facteur d'induction |
|---|---|---|
| Cpd 39 | 0,001µM | 1,08 |
| | 0,003µM | 1,17 |
| | 0,01µM | 3,19 |
| | 0,03µM | 7,69 |
| | 0,1µM | 9,68 |
| | 0,3µM | 10,16 |
| | 1µM | 10,42 |
| | 3µM | 9,96 |

La valeur maximale est 10,42, elle est observée dès la concentration de 1µM.

La figure 18b illustre les facteurs d'induction du composé 39 avec le système de transactivation PPARγ/Gal4. Les valeurs de ces facteurs d'induction sont notées dans le tableau suivant.

| Composé | Traitement | Facteur d'induction |
|---|---|---|
| Cpd 39 | 0,001µM | 0,95 |
| | 0,003µM | 0,96 |
| | 0,01µM | 1,56 |
| | 0,03µM | 3,06 |
| | 0,1µM | 4,08 |
| | 0,3µM | 4,86 |
| | 1µM | 4,78 |
| | 3µM | 4,72 |

La valeur maximale est 4,86, elle est observée dès la concentration de 0,3µM.

Les résultats illustrés par les figures montrent que les composés selon l'invention testés possèdent la propriété de ligand vis-à-vis de PPARα, PPARγ et/ou PPARδ. Ils permettent aussi l'activation au niveau transcriptionnel de ces récepteurs nucléaires.

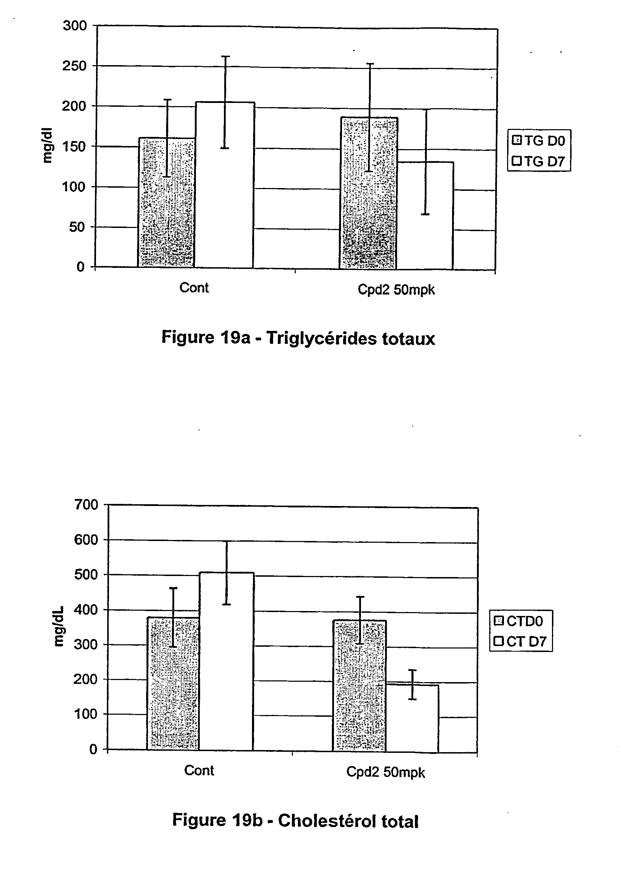

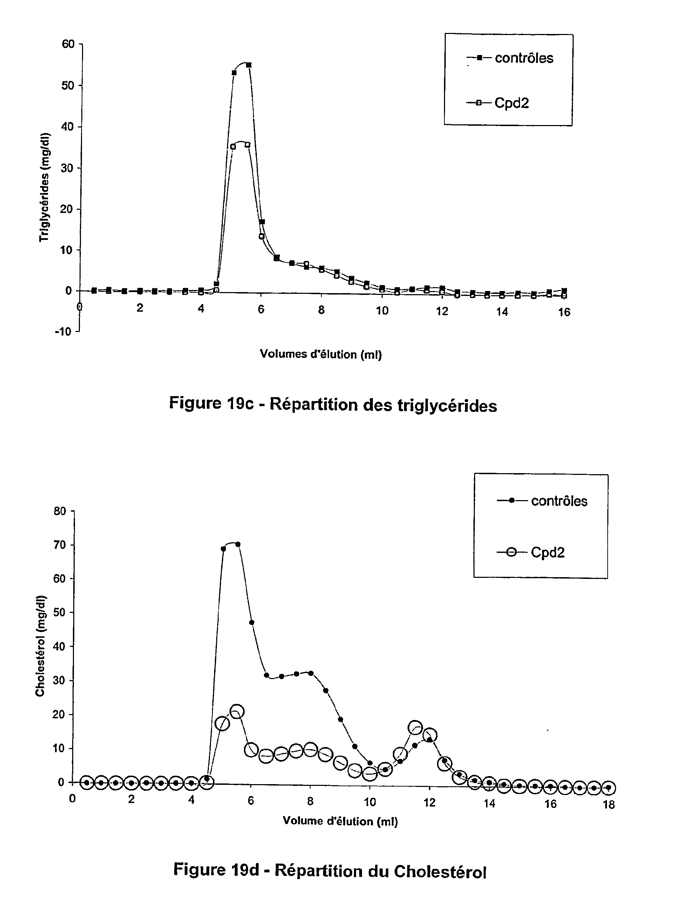

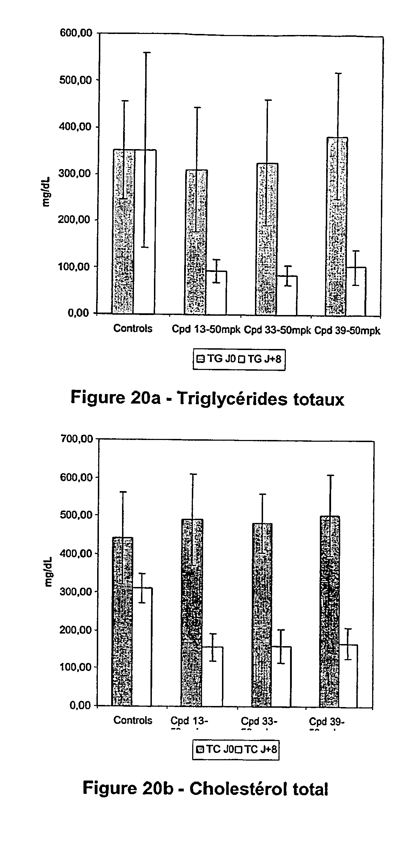

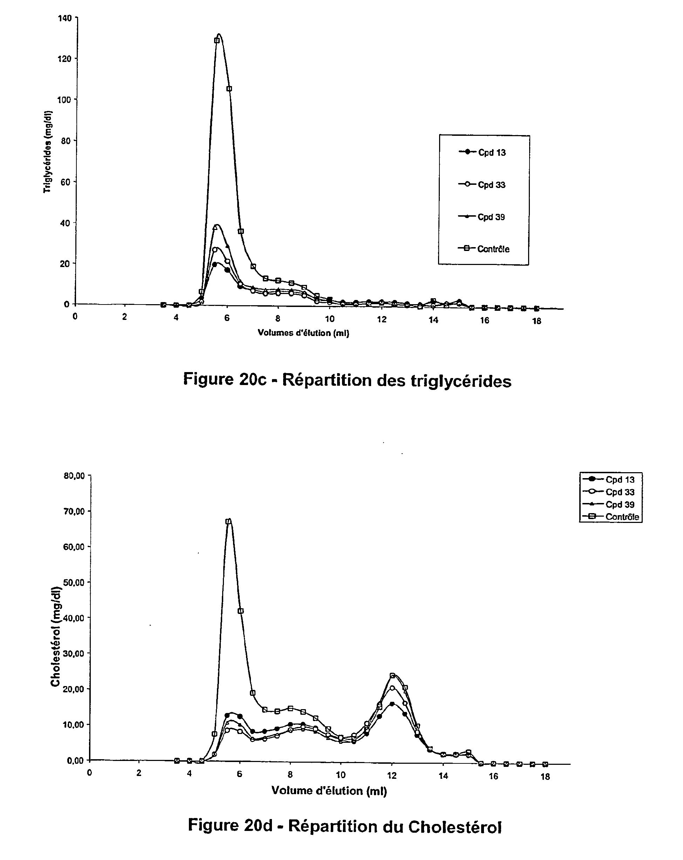

**Sur les** **figures 19a, 19b****,** **19c, 19d****,** **20a, 20b****,** **20c et 20d** sont représentés les effets du traitement avec les composé 2 (Cpd2), composé 13 (Cpd13), composé 33 (Cpd33) et composé 39 (Cpd39) sur le métabolisme des triglycérides et du cholestérol chez les souris transgéniques Apo E2/E2. Les animaux ont été traités par gavage avec 50mg de composé par kg et par jour, pendant 7 jours.

Les figures 19a et 19b montrent la diminution des taux plasmatiques de triglycérides et de cholestérol induite par le composé 2.

Les figures 20a et 20b montrent la diminution des taux plasmatiques de triglycérides et de cholestérol induite par les composés 13, 33 et 39.

Les figures 19c et 19d montrent la distribution des triglycérides et du cholestérol dans les lipoparticules évaluée par chromatographie d'exclusion induite par le traitement avec le composé 2.

Les figures 20c et 20d montrent les distributions des triglycérides et du cholestérol dans les lipoparticules évaluées par chromatographie d'exclusion induite par le traitement avec les composés 13, 33 et 39.

On observe une distribution typique des triglycérides et du cholestérol principalement localisée dans les lipoparticules de grande taille. On observe également une diminution des triglycérides et du cholestérol dans cette classe de lipoparticules induite par le traitement avec les différents composés testés.

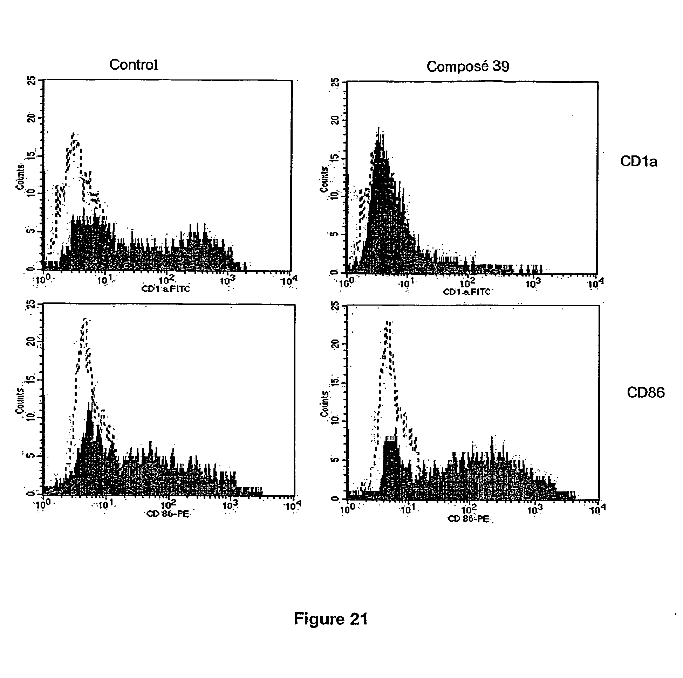

**La** **Figure 21** illustre l'interférence des composés selon l'invention avec la différenciation des cellules dendritiques.

Le composé 39 (10⁻⁶ M) est ajouté à j = 0 de la différenciation des monocytes en cellules dendritiques. Après 6 jours de différenciation (en présence des cytokines GM-CSF et IL-4), les cellules dendritiques sont analysées par cytométrie de flux.
(---): Ac de l'isotype contrôle couplé au fluorochrome
(en noir): Ac anti-CD1a couplé au FITC (Fluorescein IsoThioCyanate) ou Ac anti-CD86 couplé au PE (PhycoErythrine).

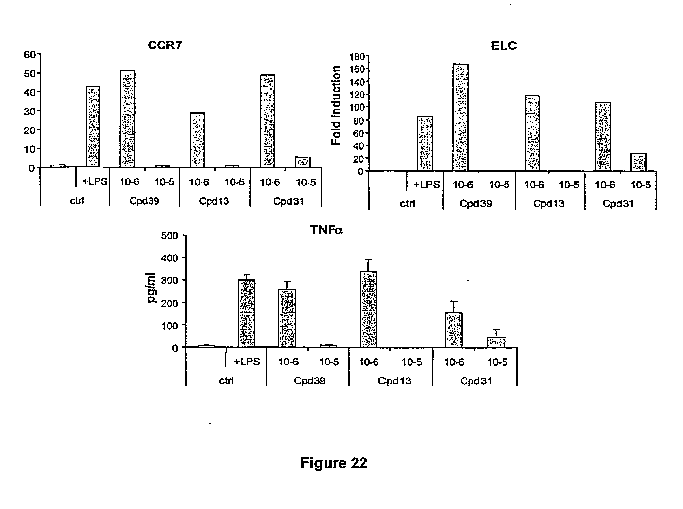

**La** **Figure 22** illustre l'interférence des composés selon l'invention avec la maturation des cellules dendritiques induites par le LPS (LipoPolySaccharide).

Les cellules dendritiques sont incubées pendant 4 heures avec les composés 31, 13 ou 39, puis sont stimulées avec le LPS pendant 16 heures. Les transcrits CCR7 et ELC sont analysés par RT-PCR quantitative tandis que la cytokine TNFalpha est analysée par la technique ELISA.

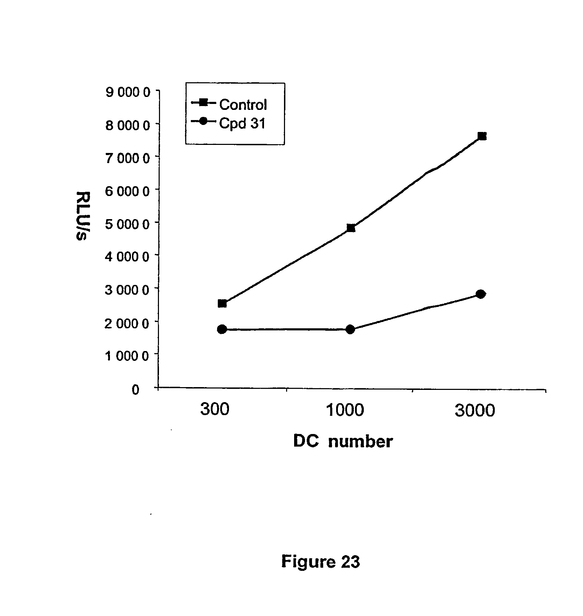

**La** **Figure 23** illustre une Réaction Mixte Lymphocytaire (MLR) effectuée en présence de quantités croissantes de cellules dendritiques traitées ou non avec le composé 31 et incubées avec des cellules T CD4+ naïves pendant 7 jours. La prolifération des cellules T est mesurée par mesure de l'incorporation de BrdU (BromodeoxyUridine).

D'autres aspects et avantages de la présente invention apparaîtront à la lecture des exemples qui suivent, qui doivent être considérés comme illustratifs et non limitatifs.

### EXEMPLES

### Exemple 1 : Synthèse des composés selon l'invention

Les composés de l'invention sont préparés selon les méthodes générales décrites ci-dessous.

**Description des méthodes générales de synthèse selon l'invention :**

### Synthèse de 1,3-diphénylprop-2-èn-1-ones :

### Méthode générale 1 :

### Synthèse de 1,3-diphénylprop-2-èn-1-ones en milieu acide :

La cétone (1 éq) et l'aldéhyde (1 éq) sont solubilisés dans une solution d'éthanol saturée d'acide chlorhydrique gazeux. L'ensemble est agité à température ambiante pendant 6 heures puis le solvant est éliminé par évaporation sous pression réduite. La 1,3-diphénylprop-2-èn-1-one est purifiée par chromatographie sur gel de silice ou par recristallisation.

### Méthode générale 2 :

### Synthèse de 1,3-diphénylprop-2-èn-1-ones en présence d'hydroxyde de sodium :

La cétone (1 éq) et l'aldéhyde (1 éq) sont solubilisés dans une solution hydroalcoolique d'hydroxyde de sodium (20 éq). L'ensemble est agité 18 heures à température ambiante. Le milieu est acidifié (pH = 2) avec de l'acide chlorhydrique.

La 1,3-diphénylprop-2-èn-1-one est obtenue par précipitation ou extraction solide liquide après évaporation du milieu réactionnel. Elle est purifiée par chromatographie sur gel de silice ou par recristallisation.

### Méthode générale 3 :

### Synthèse de 1,3-diphénylprop-2-en-1-ones substituées en présence d'éthylate de sodium :

Le sodium (1 éq) est dissout dans l'éthanol absolu. La cétone (1 éq) et l'aldéhyde (1 éq) sont ajoutés. L'ensemble est maintenu sous agitation à température ambiante pendant 12 heures puis une solution de soude 2N (5 éq) est ajoutée. L'ensemble est maintenu à 100°C pendant 12 heures. Le milieu réactionnel est acidifié par addition d'une solution aqueuse d'acide chlorhydrique 6N. Le solvant est éliminé par évaporation sous pression réduite. Le résidu est purifié par chromatographie sur gel de silice ou par recristallisation.

### O-Alkylation de phénols ou du thiophénols :

### Méthode générale 4 :

Le phénol (1 éq) ou le thiophénol (1éq) est solubilisé dans l'acétonitrile, le dérivé halogéné (1 à 10 éq) et le carbonate de potassium (5 éq) sont ajoutés. Le milieu réactionnel est maintenu environ 10 heures sous vive agitation à reflux. Les sels sont éliminés par filtration, le solvant et l'excès de réactif sont éliminés par évaporation sous pression réduite, le produit attendu est purifié par chromatographie sur gel de silice.

### Méthode générale 5 :

L'alcool (1éq), le phénol (1éq) et la triphénylphosphine sont solubilisés dans du dichlorométhane. Le diisopropylazodicarboxylate (1éq) est ajouté, l'ensemble est laissé 12 heures, sous agitation à température ambiante.

Le milieu réactionnel est lavé avec de l'eau, séché sur sulfate de magnésium et évaporé sous pression réduite. Le résidu d'évaporation est purifié par chromatographie sur gel de silice

### Acidolyse d'esters tertiobutyliques

### Méthode générale 6 :

L'ester tertiobutylique (1 éq) est solubilisé dans du dichlorométhane puis l'acide trifluoroacétique (10 éq) est additionné. L'ensemble est maintenu 12 heures sous agitation à température ambiante. Le produit formé est purifié par chromatographie sur gel de silice ou par recristallisation.

### Synthèse des matières premières servant à la synthèse des composés selon l'invention :

### Matière première 1 :

### 4'-(Bromoéthyloxy)acétophénone

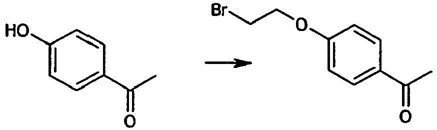

Ce composé est synthétisé à partir de 4'-hydroxyacétophénone et de dibromoéthane selon la méthode générale 4 précédemment décrite.

Purification par chromatographie sur gel de silice (élution : cyclohexane-acétate d'éthyle : 9-1).
RMN 1H CDCl₃ δppm : 2,55(s, 3H), 3,66(t, 2H, J = 6,50Hz), 4,35(t, 2H, J = 6,50Hz), 6,94(d, 2H, J = 7,23Hz), 7,94(d, 2H, J = 7,23Hz)

### Matière première 2:

### 4'-(Pentylthioéthyloxy)acétophénone

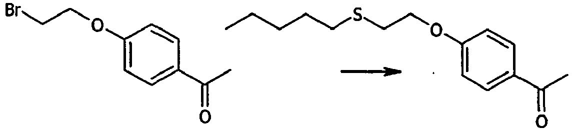

La matière première 1 (1éq) et le penthanethiol (1éq) sont solubilisés dans du méthanol en présence de triéthylamine (2 éq). Le milieu réactionnel est maintenu 18 heures à reflux, le solvant est éliminé par évaporation sous pression réduite. L'huile est reprise par de l'acétate d'éthyle, lavée par une solution aqueuse d'acide chlorhydrique 2N. La 4'-(pentylthioéthyloxy)acétophénone est obtenue après purification sur gel de silice (élution : cyclohexane-acétate d'éthyle : 9-1).
RMN 1H CDCl₃ δppm : 0,85(m, 3H), 1,24-1,39(m, 4H), 1,52-1,62(m, 2H), 2,50(s, 3H), 2,64(t, 2H, J = 7,2Hz) , 2,94(t, 2H, J = 6,8Hz), 4,14(t, 2H, J = 6,8Hz), 6,88(d, 2H, J = 8,7 Hz), 7,89(d, 2H, J = 8,7Hz)

### Matière première 3 :

### 3,5-Diméthyl-4-tertiobutyloxycarbonyldiméthylméthyloxybenzaldéhyde

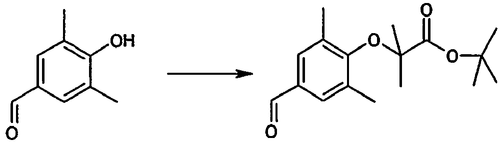

Ce composé est synthétisé à partir de 4-hydroxy-3,5-diméthylbenzaldéhyde et de bromoisobutyrate de tertiobutyle selon la méthode générale 4.

Purification par chromatographie sur gel de silice (élution : cyclohexane-acétate d'éthyle : 8-2).
RMN 1H CDCl₃ δppm : 1,43(s, 6H), 1,49(s, 9H), 2,28(s, 6H), 7,53(s, 2H), 9,88(s, 1H)

### Matière première 4:

### 4'-Hydroxy-3',5'-acetophénone

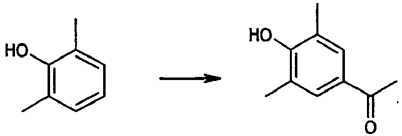

Le 2,6-diméthylphénol (1éq) est solubilisé dans du chlorure de méthylène, la solution est refroidie à 0°C puis sont ajoutés le chlorure d'aluminium (3éq) et le bromure d'acétyle (2éq). L'ensemble est agité 3 heures à température ambiante, puis versé sur de la glace. La phase aqueuse est extraite par du dichlorométhane, la phase organique est lavée à l'eau jusqu'à neutralité, séchée sur sulfate de magnésium puis le solvant est éliminé par évaporation sous pression réduite. L'ester intermédiaire obtenu est purifié par chromatographie sur gel de silice (éluant : cyclohexane-acétate d'éthyle : 9-1) puis repris par une solution aqueuse de soude 2N (2.5éq). L'ensemble est agité 48 heures à température ambiante puis acidifié par une solution d'acide chlorhydrique diluée. Le précipité est lavé avec de l'eau jusqu'à neutralité des eaux de lavage.
RMN 1H CDCl₃ δppm : 2,30(s, 6H), 2,54(s, 3H), 7,65(s, 2H)

### Matière première 5:

### 4'-((R,S)-5-[1,2]dithiolan-3-ylpentyloxy)acétophénone

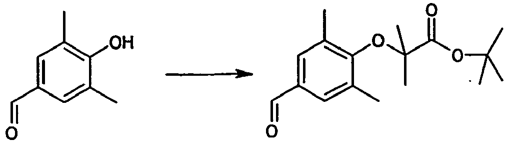

Ce composé est synthétisé à partir de 4-hydroxy-3,5-diméthylbenzaldéhyde et de bromoisobutyrate de tertiobutyle selon la méthode générale 4.

Purification par chromatographie sur gel de silice (élution : cyclohexane-acétate d'éthyle : 8-2).
RMN 1H CDCl₃ δppm : 1,43(s, 6H), 1,49(s, 9H), 2,28(s, 6H), 7,53(s, 2H), 9,88(s, 1H)

### Matière première 4:

### 4'-Hydroxy-3',5'-acetophénone

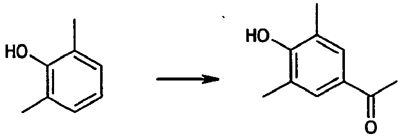

Le 2,6-diméthylphénol (1éq) est solubilisé dans du chlorure de méthylène, la solution est refroidie à 0°C puis sont ajoutés le chlorure d'aluminium (3éq) et le bromure d'acétyle (2éq). L'ensemble est agité 3 heures à température ambiante, puis versé sur de la glace. La phase aqueuse est extraite par du dichlorométhane, la phase organique est lavée à l'eau jusqu'à neutralité, séchée sur sulfate de magnésium puis le solvant est éliminé par évaporation sous pression réduite. L'ester intermédiaire obtenu est purifié par chromatographie sur gel de silice (éluant : cyclohexane-acétate d'éthyle : 9-1) puis repris par une solution aqueuse de soude 2N (2.5éq). L'ensemble est agité 48 heures à température ambiante puis acidifié par une solution d'acide chlorhydrique diluée. Le précipité est lavé avec de l'eau jusqu'à neutralité des eaux de lavage.
RMN 1H CDCl₃ δppm : 2,30(s, 6H), 2,54(s, 3H), 7,65(s, 2H)

### Matière première 5 :

### 4'-((R,S)-5-[1,2]dithiolan-3-ylpentyloxy)acétophénone

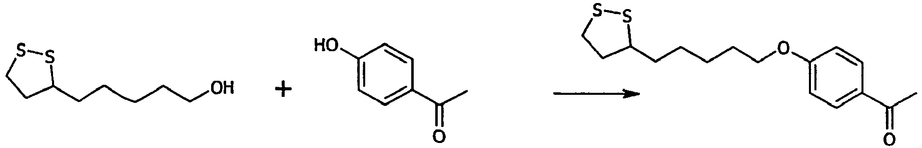

Ce composé est synthétisé à partir de 4'-hydroxyacétophénone et de (R,S)-5-[1,2] dithiolan-3-ylpentanol selon la méthode générale 5 précédemment décrite. Purification par chromatographie sur gel de silice (élution : cyclohexane-acétate d'éthyle : 95-5).
RMN 1H CDCl₃ δppm : 1,42-1,62(m, 4H), 1,62-1,75(m, 2H), 1,75-1,89(m, 2H), 1,89-1,98(m, 1H), 2,42-2,51 (m, 1H), 2,56(s, 3H), 3,08-3,21 (m, 2H), 3,55-3,61 (m, 1H), 4,06(t, 2H, J = 6,2Hz), 6,92(d, 2H, J = 8,7Hz), 7,93(d, 2H, J = 8,7Hz)

### Matière première 6 :

### (R,S)-2-phényl-2-(4-formyl-1,6-diméthylphényloxy) acétate d'éthyle

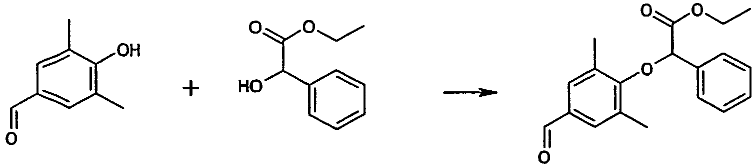

Ce composé est synthétisé à partir de 4-hydroxy-3,5-diméthylbenzaldéhyde et de 2-hydroxy-2-phényl acétate d'éthyle selon la méthode générale 5 précédemment décrite.

Purification par chromatographie sur gel de silice (élution : cyclohexane-acétate d'éthyle:9-1).
RMN 1H CDCl₃ δppm : 1,22(t, 3H, J = 7,35Hz), 2,20(s, 6H), 4,16-4,28(m, 2H), 5,3(s, 1H), 7,38-7,51 (m, 7H), 9,87(s, 1H)

### Matière première 7 :

### 4'-(Cyclohexyléthyl)acétophénone

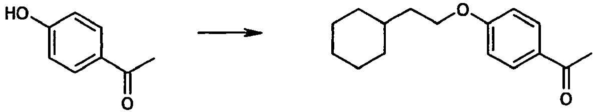

Ce composé est synthétisé à partir de 4'-hydroxyacétophénone et de 2-cyclohexyléthanol selon la méthode générale 5 précédemment décrite. Purification par chromatographie sur gel de silice (cyclohexane-acétate d'éthyle : 9-1).
RMN 1H CDCl₃ δppm : 0,90-1,80(m, 13H), 2,56 (s, 3H), 4,07(t, 2H, J = 6,45Hz), 6,92(d, 2H, J = 8,80Hz), 7,93(d, 2H, J = 8,80Hz)

### Matière première 8:

### 2,6-Diméthylthioanisole

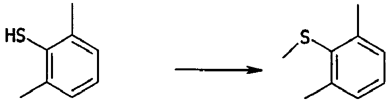

Ce composé est synthétisé à partir de 2,6-diméthylthiophénol et d'iodure de méthyle selon la méthode générale 4 précédemment décrite.

*Purification par chromatographie sur gel de silice (élution : cyclohexane-acétate d'éthyle : 9-1).
RMN 1H CDCl₃ δppm : 2,28(s, 3H), 2,62(s, 6H), 7,16(m, 3H)

### Matière première 9:

### 3',5'-Diméthyl-4'-méthylthioacétophénone

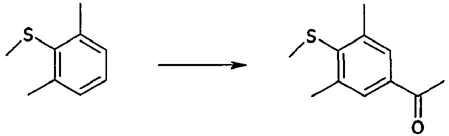

La matière première 8 (1éq) est solubilisée dans du chlorure de méthylène, la solution est refroidie à 0°C puis sont ajoutés le chlorure d'aluminium (2,5éq) et le bromure d'acétyle (2éq). L'ensemble est agité 72 heures à température ambiante, puis versé sur de la glace. La phase aqueuse est extraite par du dichlorométhane, la phase organique est lavée à l'eau jusqu'à neutralité, séchée sur sulfate de magnésium puis le solvant est éliminé par évaporation sous pression réduite. Purification par chromatographie sur gel de silice (élution : cyclohexane-acétate d'éthyle : 9-1).
RMN 1H CDCl₃ δppm : 2,23(s, 3H), 2,54(s, 3H), 2,56(s, 6H), 7,63(s, 2H)

### Matière première 10:

### 4'-Méthoxy-3',5'-diméthylacétophénone

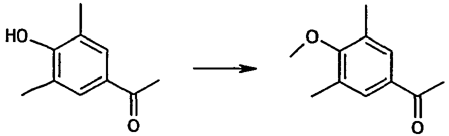

Ce composé est synthétisé à partir de la matière première 4 et d'iodure de méthyle selon la méthode générale 4 précédemment décrite.

Le produit brut obtenu après élimination du carbonate de potassium par filtration et élimination des solvants par évaporation sous pression réduite est utilisé pour la synthèse du composé intermédiaire correspondant.
RMN 1H CDCl₃ δppm : 2,31 (s, 6H), 2,54(s, 3H), 3,74(s, 3H), 7,63(s, 2H)

### Matière première 11:

### 4'-Cyclohexyléthyl-3',5'-d iméthylacétophénone

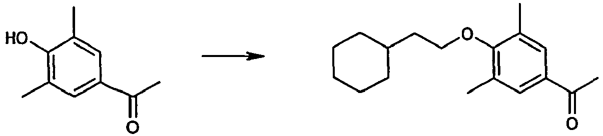

Ce composé est synthétisé à partir de la matière première 4 et de 2-cyclohexyléthanol selon la méthode générale 5 précédemment décrite. Purification par chromatographie sur gel de silice (élution : cyclohexane-acétate d'éthyle : 85-15).
RMN 1H CDCl₃ δppm : 0,92-1,80(m, 13H), 2,31(s, 6H), 2,55(s, 3H), 3,86(t, 2H, J = 7,05Hz), 7,63(s, 2H)

### Matière première 12 :

### 4'-(Bromoéthyloxy)-3',5'-diméthylacétophénone

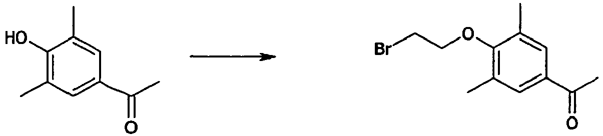

Ce composé est synthétisé à partir de la matière première 4 et de dibromoéthane selon la méthode générale 4 précédemment décrite.

Purification par chromatographie sur gel de silice (élution : cyclohexane-acétate d'éthyle : 85-15).
RMN 1H CDCl₃ δppm : 2,36(s, 6H), 2,56(s, 3H), 3,68(t, 2H, J = 6,21 Hz), 4,14(t, 2H, J = 6,21 Hz), 7,65 (s, 2H)

### Matière première 13 :

### 4'-(Cyclohexylthioéthyloxy)acétophénone

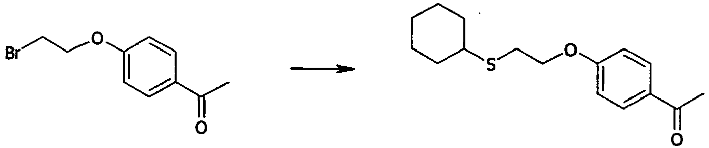

Ce composé est synthétisé à partir de la matière première 1 et de cyclohexane thiol selon la méthode générale 4 précédemment décrite.
RMN 1H CDCl₃ δppm : 1,08(m, 5H), 1,40(m, 1H), 1,56(m, 2H),1,80(m, 2H), 2,30(s, 3H), 2,53(m, 1H), 2,69(t, 2H, J = 6,96Hz), 3,95(t, 2H, J = 6,96Hz), 6,68(d, 2H, J = 8,88Hz), 7,69(d, 2H, J = 8,88Hz)

### Matière première 14 :

### 4'-(Cyclohexylthioéthyloxy)-3',5'-diméthylacétophénone

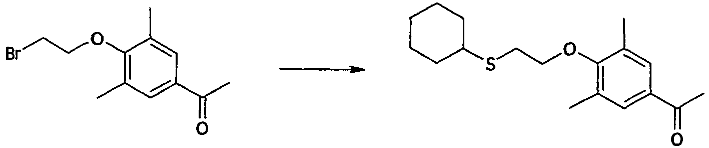

Ce composé est synthétisé à partir de la matière première 12 et de cyclohexane thiol selon la méthode générale 4 précédemment décrite.

Purification par chromatographie sur gel de silice (élution : cyclohexane-acétate d'éthyle : 9-1).
RMN 1H CDCl₃ δppm : 1,26-1,42(m, 5H), 1,59-1,65(m, 1H), 1,80(m, 2H), 2,00(m, 2H), 2,35(s, 6H), 2,56(s, 3H), 2,75(m, 1H), 2,95(t, 2H, J = 6,81 Hz), 3,96(t, 2H, J = 6,81 Hz Hz), 7,64 (s, 2H)

### Matière première 15 :

### 4'-Méthoxy-3'-méthylacétophénone

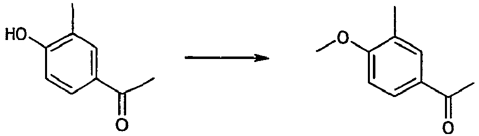

Ce composé est synthétisé à partir de 4'-hydroxy-3-méthylacétophénone et d'iodure de méthyle selon la méthode générale 4 précédemment décrite.

Le produit brut obtenu après élimination du carbonate de potassium par filtration et élimination des solvants par évaporation sous pression réduite est utilisé pour la synthèse du composé intermédiaire correspondant.
RMN 1H CDCl₃ δppm : 2,53(s, 3H), 2,56(s, 3H), 3,90(s, 3H), 6,85(d, 1H, J = 8,46Hz), 7,78(s, 1H), 7,82(d, 1H, J = 8,46Hz)

### Matière première 16 :

### 1,3-Diméthyl-2-hexylthiobenzène

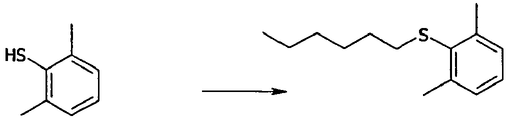

Ce composé est synthétisé à partir de 2,6-diméthylthiophénol et de bromure d'hexyle selon la méthode générale 4 précédemment décrite.

Purification par chromatographie sur gel de silice (élution : cyclohexane).
RMN 1H CDCl₃ δppm: 0,90(t, 3H, J = 6,57Hz), 1,27-1,58(m, 8H), 2,57(s, 6H), 2,66(t, 2H, J = 7,11 Hz), 7,12 (m, 3H)

### Matière première 17 :

### 3',5'-Diméthyl-4'-hexylthioacétophénone

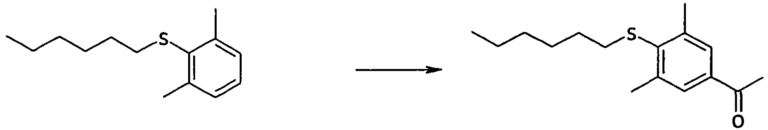

La matière première 16 (1éq) est solubilisée dans du chlorure de méthylène, la solution est refroidie à 0°C puis sont ajoutés le chlorure d'aluminium (1 éq) et le bromure d'acétyle (1éq). L'ensemble est agité 2 heures à température ambiante, puis versé sur de la glace. La phase aqueuse est extraite par du dichlorométhane, la phase organique est lavée à l'eau jusqu'à neutralité, séchée sur sulfate de magnésium puis le solvant est éliminé par évaporation sous pression réduite.

Purification par chromatographie sur gel de silice (élution : cyclohexane).
RMN 1H CDCl₃ δppm : 0,87(t, 3H, J = 6,72Hz), 1,22-1,53(m, 8H), 2,58(s, 3H), 2,59 (s, 6H), 2,68(t, 2H, J = 7,23Hz), 7,66(s, 2H)

### Matière première 18 :

### 3',5'-Diméthyl-4'-(Morpholin-4-yléthyloxy)acétophénone

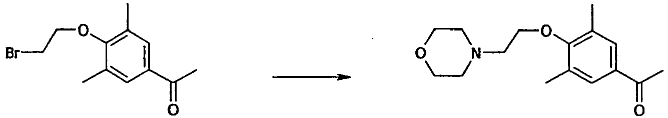

La matière première 12 (1éq) et la morpholine (0,7éq) sont solubilisées dans l'acétone. Du carbonate de potassium (1éq) est ajouté, l'ensemble est maintenu 72h00 à reflux. Le carbonate est éliminé par filtration, le solvant est éliminé par évaporation sous pression réduite. L'huile résiduelle est reprise par une solution aqueuse d'acide chlorhydrique 1 N et lavée avec de l'acétate d'éthyle. La phase aqueuse est rendue basique (pH = 9) par addition de carbonate de potassium puis extraite par de l'acétate d'éthyle. La phase organique est séchée sur sulfate de magnésium puis le solvant est éliminé par évaporation sous pression réduite.
RMN 1H CDCl₃ δppm : 2,33(s, 6H), 2,54(s, 3H), 2,60(t, 4H, J = 4,70Hz), 2,81 (t, 2H, J = 5,76Hz), 3,76(t, 4H, J = 4,70Hz) 3,93(t, 2H, J = 5,76Hz), 7,62(s, 2H)

### Matière première 19 :

### 3,5-Difluoro-4-hydroxybenzaldéhyde

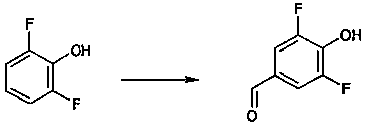

Le 2,6-difluorophénol (1éq) et l'hexaméthylènetétramine (2éq) sont solubilisés dans un mélange eau/acide acétique (10-90). L'ensemble est maintenu 18h00 à reflux puis refroidi à température ambiante.

Le milieu réactionnel est extrait par du dichlorométhane. Les phases organiques sont rassemblées, séchées sur sulfate de magnésium. Le solvant est éliminé par évaporation sous pression réduite.
RMN 1H CDCl₃ δppm : 7,35(dd, 2H, J = 6,57Hz, J = 2,82Hz), 9,67(s, 1H)

### Matière première 20 :

### 4'-Méthoxy-3'-trifluorométhylacétophénone

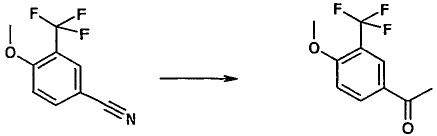

Le 4-méthoxy-3-trifluorométhylbenzonitrile (1 éq) est dissout dans du THF anhydre. Du chlorure de méthyl magnésium en solution dans le THF (1éq) est additionné. Le milieu réactionnel est laissé 16h00 sous agitation à température ambiante puis une heure à reflux après addition d'un complément de chlorure de méthyl magnésium (1éq).

Le milieu réactionnel est versé sur une solution aqueuse d'acide chlorhydrique 1 N, et extrait par du dichlorométhane. La phase organique est neutralisée avec une solution aqueuse de bicarbonate de potassium puis lavée avec de l'eau. La phase organique est séchée sur sulfate de magnésium puis le solvant est éliminé par évaporation sous pression réduite.

Purification par chromatographie sur gel de silice (élution : cyclohexane-acétate d'éthyle : 9-1).
RMN 1H CDCl₃ δppm : 2,60(s, 3H), 3,99(s, 3H), 7,07(d, 1H, J = 8,79 Hz), 8,14(d, 1H, J = 8,79Hz, J = 1,77Hz), 8,19(s, 1H)

### Synthèse de composés intermédiaires servant à la synthèse des composés selon l'invention :

### Composé intermédiaire 1:

### 1-(4-(Pentylthioéthyloxy)phényl)-3-(4-hydroxy-3,5-diméthylphényl)prop-2-èn-1-one

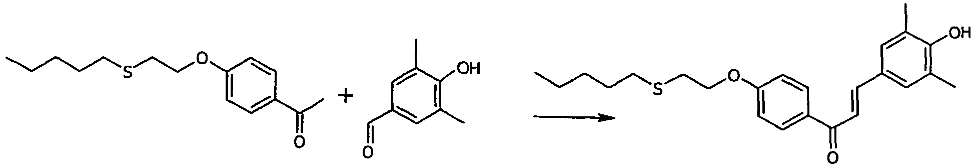

Ce composé est synthétisé à partir de la matière première 2 et de 4-hydroxy-3,5-diméthylbenzaldéhyde selon la méthode générale 1 précédemment décrite. Purification par chromatographie sur gel de silice (élution : cyclohexane-acétate d'éthyle : 85-15).
RMN 1H CDCl₃ δppm : 0,91 (m, 3H), 1,33-1,42(m, 4H), 1,59-1,67(m, 2H), 2,29(s, 6H), 2,64(t, 2H, J = 7,60Hz) , 2,96(t, 2H, J = 6,80Hz), 4,24(t, 2H, J = 6,80Hz), 6,97(d, 2H, J = 8,70Hz), 7,31(s, 2H), 7,37(d, 1H, J = 15,54Hz), 7,72(d, 1H, J = 15,54Hz), 8,03(d, 2H, J = 8,70Hz)

### Composé intermédiaire 2 :

### 1-(4-((R,S)-5-[1,2]dithiolan-3-ylpentyloxy)phényl)-3-(4-hydroxy-3,5-diméthylphényl) -prop-2-èn-1-one

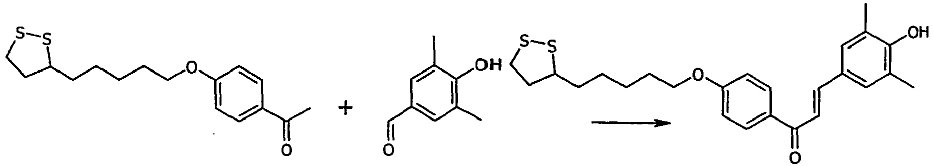

Ce composé est synthétisé à partir de la matière première 5 et de 4-hydroxy-3,5-diméthylbenzaldéhyde selon la méthode générale 1 précédemment décrite. Purification par chromatographie sur gel de silice (élution : cyclohexane-acétate d'éthyle : 8-2).
RMN 1H CDCl₃ δppm : 1,45-1,65(m, 4H), 1,65-1,77(m, 2H), 1,77-1,87(m, 2H), 1,87-2,0(m, 1H), 2,30(s, 6H), 2,43-2,51(m, 1H), 3,09-3,22(m, 2H), 3,56-3,62(m, 1H), 4,04(t, 2H, J = 6,40Hz), 6,96(d, 2H, J = 8,50Hz), 7,31 (s, 2H), 7,41 (d, 1H, J = 15,40Hz), 7,73(d, 1H, J = 15,40Hz), 8,04(d, 2H, J = 8,50Hz)

### Composé intermédiaire 3 :

### 1-(4-Méthylthiophényl)-3-(4-hydroxy-3,5-dibromophényl)prop-2-èn-1-one

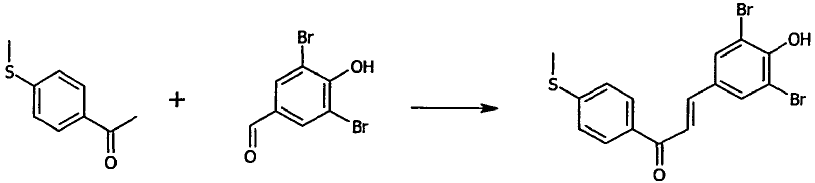

Ce composé est synthétisé à partir de 4'-méthylthioacétophénone et de 3,5-dibromo-4-hydroxybenzaldéhyde selon la méthode générale 1 précédemment décrite.

Purification par chromatographie sur gel de silice (élution : cyclohexane-acétate d'éthyle : 8-2).
RMN 1H CDCl₃ δppm : 2,55(s, 3H), 6,19(s, 1H), 7,32(d, 2H, J = 8,70Hz), 7,41 (1H, J = 15,40Hz), 7,63(d, 1H, J = 15,40), 7,75(s, 2H), 7,96(d, 2H, J = 8,70Hz)

### Composé intermédiaire 4:

### 1-(4-(Cyclohexyléthyloxy)phényl)-3-(4-hydroxy-3,5-diméthylphényl)prop-2-èn-1-one

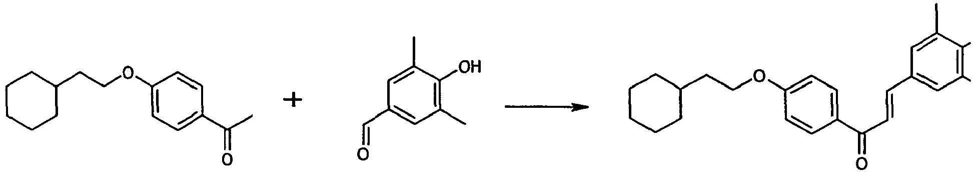

Ce composé est synthétisé à partir de la matière première 7 et de 3,5-diméthyl-4-hydroxybenzaldéhyde selon la méthode générale 1 précédemment décrite.

Le produit cristallise dans le milieu réactionnel, il est essoré, lavé avec de l'éthanol préalablement refroidi à 0°C et séché sous vide.
RMN 1H CDCl₃ δppm : 0,90-1,80(m, 13H), 2,30(s, 6H), 4,08(t, 2H, J = 6,54Hz), 6,97(d, 2H, J = 9,00Hz), 7,30(s, 2H), 7,42(d, 1H, J = 15,50Hz), 7,73(d, 1H, J = 15,50Hz), 8,03(d, 2H, J = 9,00Hz)

### Composé intermédiaire 5 :

### 1-(4-Méthylthio-3,5-diméthyl-phényl)-3-(4-hydroxy-3,5-diméthylphényl)prop-2-èn-1-one

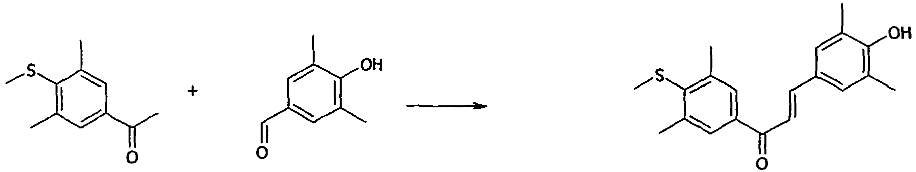

Ce composé est synthétisé à partir de la matière première 9 et de 3,5-diméthyl-4-hydroxybenzaldéhyde selon la méthode générale 1 précédemment décrite. Purification par chromatographie sur gel de silice (élution : cyclohexane-acétate d'éthyle : 8-2).
RMN 1H CDCl₃ δppm : 2,28 (s, 3H), 2,30(s, 6H), 2,64(s, 6H), 7,32(s, 2H), 7,36(d, 1H, J = 15,76Hz), 7,72(s, 2H), 7,73(d, 1H, J = 15,76Hz)

### Composé intermédiaire 6:1

### 1-(4-Méthoxy-3,5-diméthylphényl)-3-(4-hydroxy-3,5-diméthylphényl)prop-2-èn-1-one

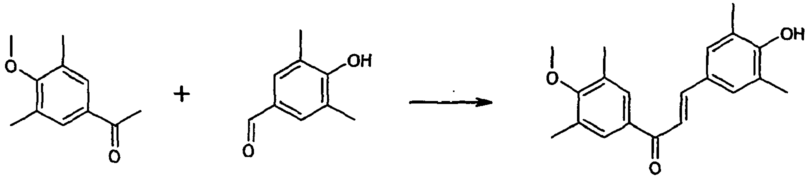

Ce composé est synthétisé à partir de la matière première 10 et de 3,5-diméthyl-4-hydroxybenzaldéhyde selon la méthode générale 1 précédemment décrite.

Le produit cristallise dans le milieu réactionnel, il est essoré, lavé avec de l'éthanol préalablement refroidi à 0°C et séché sous vide.
RMN 1H CDCl₃ δppm : 2,28(s, 6H), 2,35(s, 6H), 3,77(s, 3H), 7,30(s, 2H), 7,35(d, 1H, J = 15,63Hz), 7,70(d, 1H, J = 15,63Hz), 7,72(s, 2H)

### Composé intermédiaire 7 :

### 1-(4-(Cyclohexyléthyloxy)-3,5-diméthylphényl)-3-(4-hydroxy-3,5-diméthylphényl)prop-2-èn-1-one

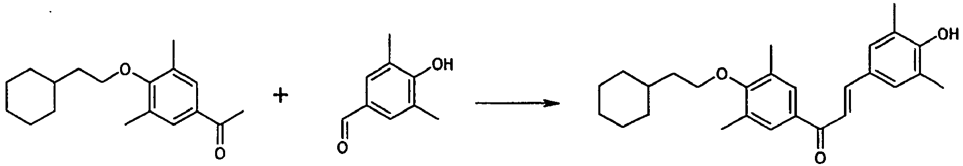

Ce composé est synthétisé à partir de la matière première 11 et de 3,5-diméthyl-4-hydroxybenzaldéhyde selon la méthode générale 1 précédemment décrite. Purification par chromatographie sur gel de silice (élution : cyclohexane-acétate d'éthyle : 8-2).
RMN 1H CDCl₃ δppm : 0,94-1,05(m, 2H), 1,16-1,31 (m, 4H), 1,57-1,82(m, 7H), 2,30(s, 6H), 2,35(s, 6H), 3,86(t, 2H, J = 7,08 Hz), 7,32(s, 2H), 7,38(d, 1H, J = 15,81 Hz), 7,71 (s, 2H), 7,72(d, 1H, J = 15,81Hz)

### Composé intermédiaire 8 :

### 1-(4-(Cyclohexylthioéthyloxy)phényl)-3-(4-hydroxy-3,5-diméthylphényl)prop-2-èn-1-one

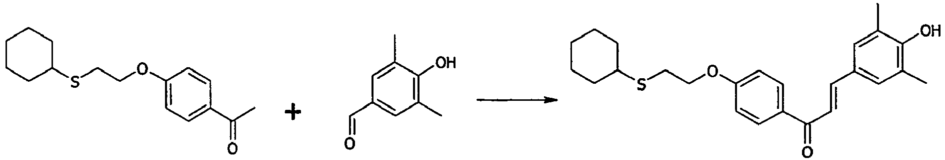

Ce composé est synthétisé à partir de la matière première 13 et de 3,5-diméthyl-4-hydroxybenzaldéhyde selon la méthode générale 1 précédemment décrite.

Le produit cristallise dans le milieu réactionnel, il est essoré, lavé avec de l'éthanol préalablement refroidi à 0°C.
RMN 1H CDCl₃ δppm : 1,23-1,42(m, 5H), 1,63-1,65(m, 1H), 1,79-1,81(m, 2H), 2,01-2,08(m, 2H), 2,29(s, 6H), 2,73-2,81(m, 1H), 2,96(t, 2H, J = 7,08Hz), 4,20(t, 2H, J = 7,08Hz), 6,97(d, 2H, J = 8,73Hz), 7,30(s, 2H), 7,41 (d, 1H, J = 15,53Hz), 7,73(d, 1H, 15,53Hz), 8,04(d, 2H, J = 8,73Hz)

### Composé intermédiaire 9 :

### 1-(2,4,5-Triméthylphényl)-3-(4-hydroxy-3,5-diméthylphényl)prop-2-èn-1-one

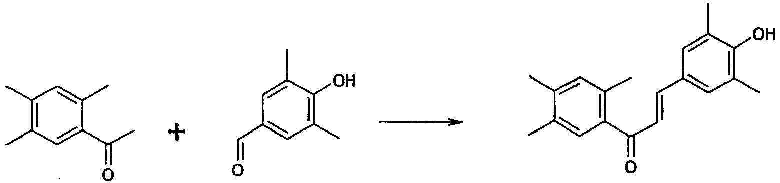

Ce composé est synthétisé à partir de 2',4',5'-triméthylacétophénone et de 3,5-diméthyl-4-hydroxybenzaldéhyde selon la méthode générale 1 précédemment décrite.

Purification par chromatographie sur gel de silice (élution : cyclohexane-acétate d'éthyle : 7-3).
RMN 1H CDCl₃ δppm : 2,27(s, 9H), 2,29(s, 3H), 2,38(s, 3H), 7,00(d, 1H, J=15,90Hz), 7,04 (s, 1H), 7,23(s, 2H), 7,27(s, 1H,), 7,39(d, 1H, J=15,90Hz)

### Composé intermédiaire 10 :

### 1-(4-(Cyclohexylthioéthyloxy)-3,5-diméthylphényl)-3-(4-hydroxy-3,5-diméthylphényl)prop-2-èn-1-one

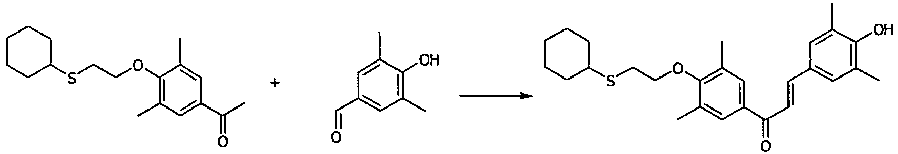

Ce composé est synthétisé à partir de la matière première 14 et de 3,5-diméthyl-4-hydroxybenzaldéhyde selon la méthode générale 1 précédemment décrite. Purification par chromatographie sur gel de silice (élution : cyclohexane-acétate d'éthyle : 7-3).
RMN 1H CDCl₃ δppm : 1,32(m, 5H), 1,63(m, 1H), 1,79(m, 2H), 2,03(m, 2H), 2,29(s, 6H), 2,37(s, 6H), 2,75(m, 1H), 2,97(t, 2H, J = 7,05Hz), 3,97(t, 2H, J = 7,05Hz), 7,30(s, 2H) 7,37(d, 1H, J = 15,70Hz), 7,70(d, 1H, J = 15,70Hz), 7,71 (s, 2H)

### Composé intermédiaire 11 :

### 1-(4-Méthylthiophényl)-3-(4-hydroxy-3-fluorophényl)prop-2-èn-1-one

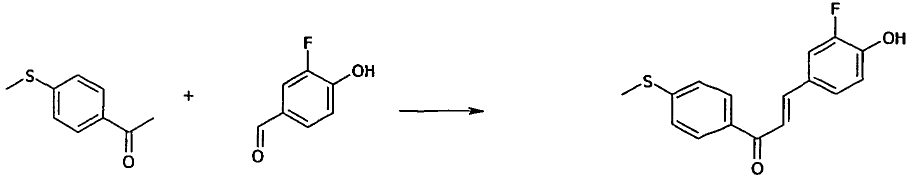

Ce composé est synthétisé à partir de 4'-méthylthioacétophénone et de 3-fluoro-4-hydroxybenzaldéhyde selon la méthode générale 1 précédemment décrite.
Le produit cristallise dans le milieu réactionnel, il est essoré et séché sous vide. RMN 1H CDCl₃ δppm: 2,55(s, 3H), 7,04(t, 1H, J = 8,37Hz), 7,30-7,42(m, 5H), 7,73(d, 1H, J = 15,54Hz), 7,95(d, 2H, J = 8,40Hz)

### Composé intermédiaire 12:

### 1-(2,3,4,5,6-Pentaméthyiphényl)-3-(4-hydroxy-3,5-diméthylphényl)prop-2-èn-1-one

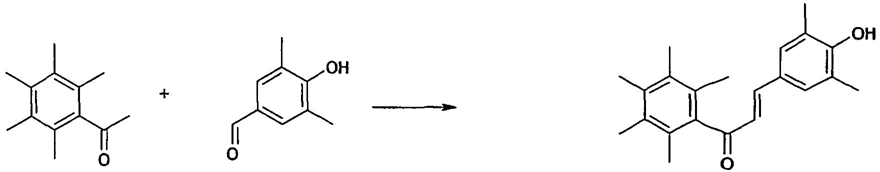

Ce composé est synthétisé à partir de pentaméthylacétophénone et de 3,5-diméthyl-4-hydroxybenzaldéhyde selon la méthode générale 1 précédemment décrite.

Le produit cristallise dans le milieu réactionnel, il est essoré et purifié par recristallisation dans l'éthanol.
RMN 1H CDCl₃ δppm : 2,09(s, 6H), 2,20(s, 6H), 2,22(s, 6H), 2,26(s, 3H), 6,83(d, 1H, J = 16,11 Hz), 7,05(d, 1H, J = 16,11 Hz), 7,16(s, 2H)

### Composé intermédiaire 13: 1-(4-Phénoxyphényl)-3-(4-hydroxy-3,5-diméthylphényl)prop-2-èn-1-one

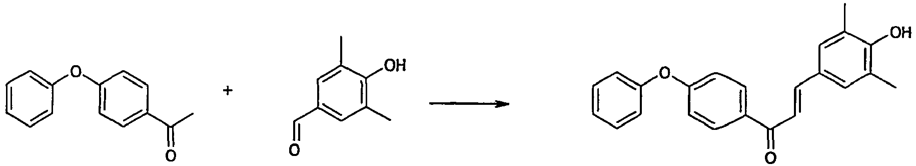

Ce composé est synthétisé à partir de 4'-phénoxyacétophénone et de 3,5-diméthyl-4-hydroxybenzaldéhyde selon la méthode générale 1 précédemment décrite.

Purification par chromatographie sur gel de silice (élution : cyclohexane-acétate d'éthyle : 7-3).
RMN 1H CDCl₃ δppm : 2,30(s, 6H), 7,05(d, 2H, J = 8,67Hz), 7,1 (d, 2H, J = 8,47Hz), 7,21 (t, 1H, J = 7,30Hz), 7,31 (s, 2H), 7,43-7,38(m, 3H), 7,75 (d, 1H, J = 15,36Hz), 8,05 (d, 2H, J = 8,47Hz)

### Composé intermédiaire 14 :

### 1-(4-Méthoxy-3-fluorophényl)-3-(4-hydroxy-3,5-diméthylphényl)prop-2-èn-1-one

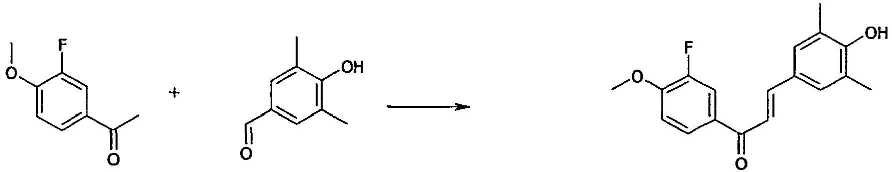

Ce composé est synthétisé à partir de 4'-méthoxy-3'-fluoroacétophénone et de 3,5-diméthyl-4-hydroxybenzaldéhyde selon la méthode générale 1 précédemment décrite.

Le produit cristallise dans le milieu réactionnel, il est essoré puis lavé avec de l'heptane.
RMN 1H CDCl₃ δppm : 2,30(s, 6H), 3,98(s, 3H), 7,04(t, 1H, J = 8,30Hz), 7,31 (s, 2H), 7,35(d, 1H, J = 15,69Hz), 7,74(d, 1H, J = 15,69Hz), 7,79-7,87(m, 2H)

### Composé intermédiaire 15: 1-(4-Méthoxy-3-méthylphényl)-3-(4-hydroxy-3,5-diméthylphényl)prop-2-èn-1-one

Ce composé est synthétisé à partir de la matière première 15 et de 3,5-diméthyl-4-hydroxybenzaldéhyde selon la méthode générale 1 précédemment décrite.

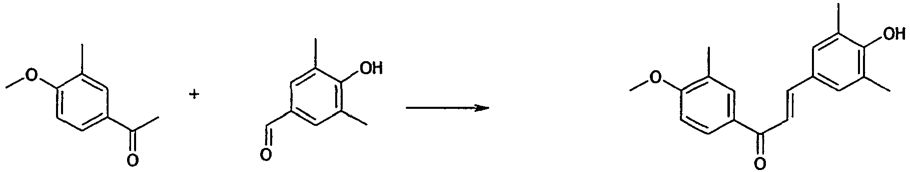

Le produit cristallise dans le milieu réactionnel, il est essoré et lavé avec de l'heptane.
RMN 1H CDCl₃ δppm : 2,30(s, 9H), 3,92(s, 3H), 6,90(d, 1H, J = 8,45Hz), 7,31 (s, 2H), 7,43(d, 1H, J = 15,52Hz), 7,73(d, 1H, J = 15,52Hz), 7,88(s, 1H), 7,93(d, 1H, J = 8,45Hz)

### Composé intermédiaire 16 :

### 1-(4-Hexylthio-3,5-diméthylphényl)-3-(4-hydroxy-3,5-diméthylphényl)prop-2-èn-1-one

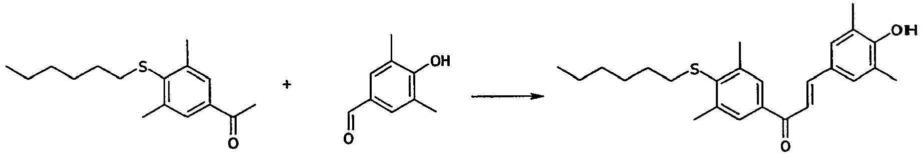

Ce composé est synthétisé à partir de la matière première 17 et de 3,5-diméthyl-4-hydroxybenzaldéhyde selon la méthode générale 1 précédemment décrite. Purification par chromatographie sur gel de silice (élution : cyclohexane-acétate d'éthyle : 8-2).
RMN 1H CDCl₃ δppm : 0,88(t, 3H, J = 6,90Hz), 1,20-1,50(m, 8H), 2,30(s, 6H), 2,63(s, 6H), 2,70(t, 2H, J = 6,9Hz), 7,32(s, 2H), 7,36(d, 1H, J = 15,51Hz), 7,72(s, 2H), 7,73 (d, 1H, J = 15,51 Hz)

### Composé intermédiaire 17 :

### 1-(2,5-Diméthoxyphényl)-3-(4-hydroxy-3,5-diméthylphényl)prop-2-èn-1-one

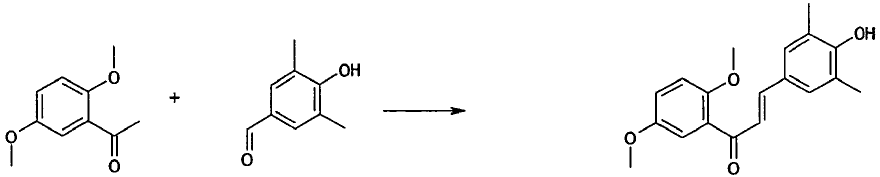

Ce composé est synthétisé à partir de 2',5'-diméthoxyacétophénone et de 3,5-diméthyl-4-hydroxybenzaldéhyde selon la méthode générale 1 précédemment décrite.

Purification par chromatographie sur gel de silice (élution : cyclohexane-acétate d'éthyle : 7-3).
RMN 1H CDCl₃ δppm : 2,27(s, 6H), 3,74(s, 3H), 3,82(s, 3H), 6,93(d, 1H, J = 8,73Hz), 7,02(dd, 1H, J = 8,73Hz, J = 3,27Hz), 7,14(d, 1H, J = 3,27Hz), 7,22(d, 1H, J = 15,81 Hz), 7,25(s, 2H), 7,53(d, 1H, J = 15,81 Hz)

### Composé intermédiaire 18 :

### 1-(4-Bromophényl)-3-(4-hydroxy-3,5-difluorophényl)-prop-2-èn-1-one

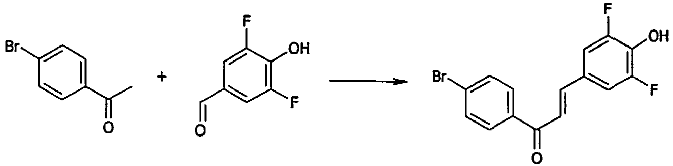

Ce composé est synthétisé à partir de 4'-bromoacétophénone et de la matière première 19 selon la méthode générale 1 précédemment décrite.

L'éthanol est éliminé par évaporation sous pression réduite, le solide est lavé avec de l'éthanol absolu.
RMN 1H CDCl₃ δppm: 5,97(s, 1H), 7,18(d, 2H, J = 8,30Hz), 7,35(d, 1H, J = 15,36Hz), 7,65(m, 3H), 7,89(d, 2H, J=8,30Hz)

### Composé intermédiaire 19 :

### 1-(4-Méthoxy-3-trifluorométhylphényl)-3-(4-hydroxy-3,5-diméthylphényl)-prop-2-èn-1-one

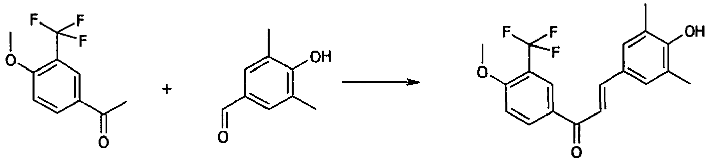

Ce composé est synthétisé à partir de la matière première 20 et de 3,5-diméthyl-4-hydroxybenzaldéhyde selon la méthode générale 1 précédemment décrite. L'éthanol est éliminé par évaporation sous pression réduite, le solide est lavé avec de l'éthanol absolu.
RMN 1H DMSOd₆ δppm : 2,22(s, 6H), 4,01 (s, 3H), 7,41 (d, 1H, J = 9,00 Hz), 7,52(s, 2H), 7,64(d, 1H, J = 15,40Hz), 8,96(s, 1H), 7,76(d, 1H, J = 15,40Hz), 8,29 (d, 1H, J = 1,60Hz), 8,49(dd, 1H, J = 9,00Hz, J = 1,60Hz)

### Synthèse des composés selon l'invention :

### Composé 1 selon l'invention :

### 1-(4-(Pentylthioéthyloxy)phényl)-3-(4-tertiobutyloxycarbonyldiméthylméthyloxy-3,5-diméthylphényl)prop-2-èn-1-one

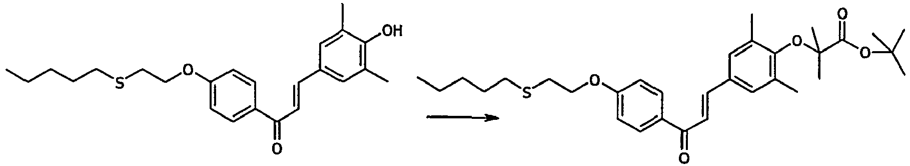

Ce composé est synthétisé à partir du composé intermédiaire 1 et de bromoisobutyrate de tertiobutyle selon la méthode générale 4 précédemment décrite.

Purification par chromatographie sur gel de silice (élution : cyclohexane-acétate d'éthyle : 9-1).
RMN 1H CDCl₃ δppm : 0,91 (t, 3H, J = 7,10Hz), 1,37-1,69(m, 21H) 2,27(s, 6H), 2,63(t, 2H, J = 7,10Hz), 2,93(t, 2H, J = 7,10Hz), 4,21(t, 2H, J = 7,10Hz), 6,97(d, 2H, J = 8,70Hz), 7,28(s, 2H), 7,44(d, 1H, J = 15,81 Hz), 7,70(d, 1H, J = 15,81 Hz), 8,03(d, 2H, J = 8,70Hz)

### Composé 2 selon l'invention :

### 1-(4-(Pentylthioéthyloxy)phényl)-3-(4-carboxydiméthylméthyloxy-3,5-diméthylphényl)prop-2-èn-1-one

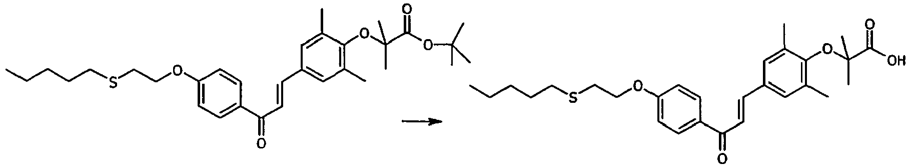

Ce composé est synthétisé à partir du composé 1 selon la méthode générale 6 précédemment décrite.

Purification par chromatographie sur gel de silice (élution : dichlorométhane-méthanol : 98-2).
RMN 1H CDCl₃ δppm: 0,84-0,89(m, 3H), 1,39-1,24(m, 4H), 1,39(s, 6H), 1,50-1,57 (m, 2H), 2,22(s, 6H), 2,61 (t, 2H, J = 7,40Hz), 2,90(t, 2H, J = 6,20Hz), 4,26(t, 2H, J = 6,20Hz), 7,09(d, 2H, J = 8,50Hz), 7,57(s, 2H), 7,59(d, 1H, J = 15,40Hz), 7,83(d, 1H, J = 15,40Hz), 8,15(d, 2H, J = 8,50Hz), 12,90(s, 1H)
SM(ES-MS): 483,2(m-1)
F°C = 85,2-89,8

### Composé 3 selon l'invention :

### 1-(4-Hydroxy-3,5-diméthylphényl)-3-(4-tertiobutyloxycarbonyldiméthylméthyloxy-3,5-diméthylphényl)-prop-2-ène-1-one

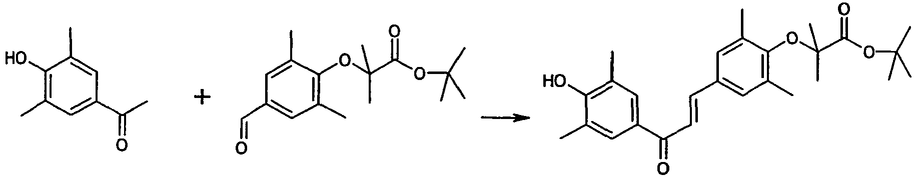

Ce composé est synthétisé à partir de la matière première 3 et de la matière première 4 selon la méthode générale 1 précédemment décrite.

Purification par chromatographie sur gel de silice (élution : dichlorométhane-méthanol : 95-5).
RMN 1H CDCl₃ δppm : 1,46(s, 6H), 1,53(s, 9H), 2,27(s, 6H), 2,33(s, 6H), 7,28(s, 2H), 7,43(d, 1H, J = 15,81 Hz), 7,69(d, 1H, J = 15,81 Hz), 7,74(s, 2H)

### Composé 4 selon l'invention :

### 1-(4-Hydroxy-3,5-diméthylphényl)-3-(4-carboxydiméthylméthyloxy-3,5-diméthylphényl)-prop-2-ène-1-one

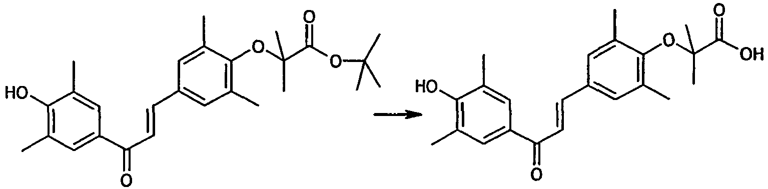

Ce composé est synthétisé à partir du composé 3 selon la méthode générale 6 précédemment décrite.

Purification par chromatographie sur gel de silice (élution : dichlorométhane-méthanol : 98-2).
RMN 1H CDCl₃ δppm : 1,39(s, 6H), 2,22(s, 6H), 2,25(s, 6H), 7,33(s, 2H), 7,45(d, 1H, J = 15,5Hz), 7,69(d, 1H, J = 15,5Hz), 7,75(s, 2H)
SM(ES-MS) : 381,3(m-1)
F°C = 199,3-199,8

### Composé 5 selon l'invention :

### 1-(4-((R,S)-5-[1,2]dithiolan-3-ylpentyloxy)phényl)-3-(4-tertiobutyloxycarbonyl diméthyl méthyloxy-3,5-diméthylphényl)-prop-2-èn-1-one

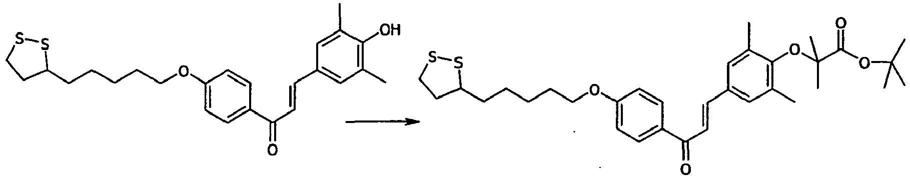

Ce composé est synthétisé à partir du composé intermédiaire 2 et de bromoisobutyrate de tertiobutyle selon la méthode générale 4 précédemment décrite.

Purification par chromatographie sur gel de silice (élution : cyclohexane-acétate d'éthyle:85-15).
RMN 1H CDCl₃ δppm : 1,43(s, 6H), 1,53(m, 13H), 1,65-1,75(m, 2H), 1,75-1,85(m, 2H), 1,85-1,97(m, 1H), 2,28(s, 6H), 1,46-1,52(m, 1H), 3,12-3,21(m, 2H), 3,58-3,63(m, 1H),4,05(t, 2H, J = 6,21 Hz), 6,97(d, 2H, J = 8,30Hz), 7,29(s, 2H), 7,45(d, 1H, J = 15,50Hz), 7,70(d, 1H, J = 15,50Hz), 8,03(d, 2H, J = 8,30Hz)

### Composé 6 selon l'invention :

### 1-(4-((R,S)-5-[1,2]dithiolan-3-ylpentyloxy)phényl)-3-(4-carboxydiméthylméthyloxy-3,5-diméthylphényl)-prop-2-èn-1-one

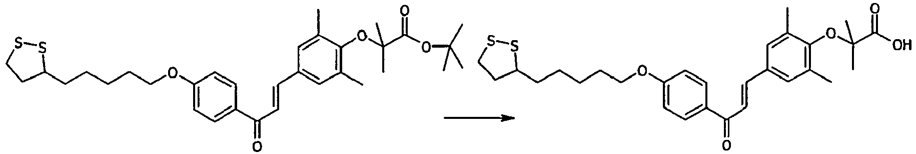

Ce composé est synthétisé à partir du composé 5 selon la méthode générale 6 précédemment décrite.

Purification par chromatographie sur gel de silice (élution : dichlorométhane-méthanol : 98-2 ).
RMN 1H CDCl₃ δppm : 1,56(m, 10H), 1,67-1,77(m, 2H), 1,77-1,90(m, 2H), 1,90-1,97(m, 1H), 2,30(s, 6H), 2,43-2,52(m, 1H), 3,11-3,22(m, 2H), 3,58-3,63(m, 1H), 4,05(t, 2H, J = 6,20Hz), 6,98(d, 2H, J = 8,80Hz), 7,31(s,2H), 7,46(d,1H, J = 15,80Hz), 7,71 (d, 1H, J = 15,80Hz), 8,03(d, 2H, J = 8,80Hz)
SM(ES-MS) : 529,1(M+1)
F°C= 182,7-186,6°C

### Composé 7 selon l'invention :

### 1-(4-Méthylthiophényl)-3-(4-tertiobutyloxycarbonyldiméthylméthyloxy-3,5-dibromophényl)-prop-2-ène-1-one

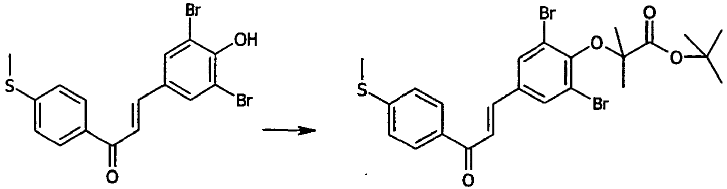

Ce composé est synthétisé à partir du composé intermédiaire 3 et de bromoisobutyrate de tertiobutyle selon la méthode générale 4 précédemment décrite.

Purification par chromatographie sur gel de silice (élution : cyclohexane-acétate d'éthyle : 9-1).
RMN 1H CDCl₃ δppm : 1,54(s, 9H), 1,63(s, 6H), 2,56(s, 3H), 7,33(d, 2H, J = 8,50Hz), 7,44(d, 1H, J = 15,70Hz), 7,62(d, 1H, J = 15,70Hz), 7,78(s, 2H), 7,96(d, 2H, J = 8,50Hz)

### Composé 8 selon l'invention :

### 1-(4-Méthylthiophényl)-3-(4-carboxydiméthylméthyloxy-3,5-dibromophényl)-prop-2-ène-1-one

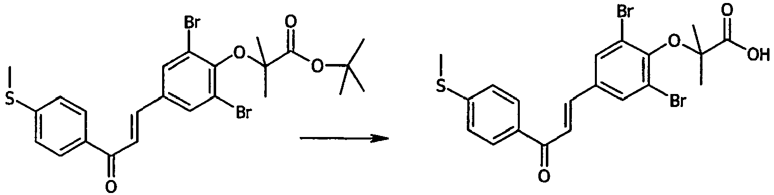

Ce composé est synthétisé à partir du composé 7 selon la méthode générale 6 précédemment décrite.

Purification par chromatographie sur gel de silice (élution : dichlorométhane-méthanol : 98-2).
RMN 1H CDCl₃ δppm : 1,54(s, 6H), 2,51(s, 3H), 7,41(d, 2H, J = 8,5Hz), 7,64(d, 1H, J = 15,4Hz), 8,04(d, 1H, J = 15,4Hz), 8,15(d, 2H, J = 8,5Hz), 8,29(s, 2H), 12,93(s, 1H)
SM(ES-MS): 513,2(m-1)

### Composé 10 selon l'invention :

### 1-(4-Cyclohexyléthyloxyphényl)-3-(4-tertiobutyloxycarbonyldiméthylméthyloxy-3,5-diméthylphényl)prop-2-èn-1-one

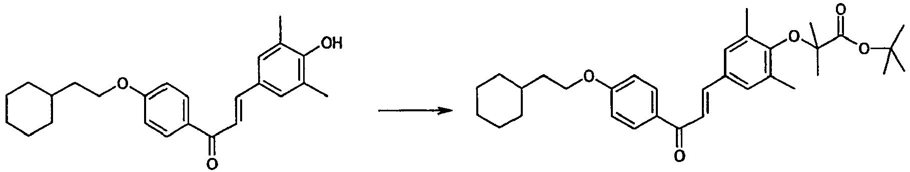

Ce composé est synthétisé à partir du composé intermédiaire 4 et de bromoisobutyrate de tertiobutyle selon la méthode générale 4 précédemment décrite.

Purification par chromatographie sur gel de silice (élution : dichlorométhane-cyclohexane : 7-3 ).
RMN 1H CDCl₃ δppm : 0 ,90-1,30(m, 5H), 1,50(m, 16H), 1,73(m, 7H), 2,28(s, 6H), 4,08(t, 2H, J = 6,54Hz), 6,97(d, 2H, J = 8,70 Hz), 7,29(s, 2H), 7,45(d, 1H, J = 15,75Hz), 7,70(d, 1H, J = 15,75Hz), 8,03(d, 2H, J = 8,70Hz)

### Composé 11 selon l'invention :

### 1-(4-Cyclohexyléthyloxyphényl)-3-(4-carboxydiméthylméthyloxy-3,5-diméthylphényl) prop-2-èn-1-one

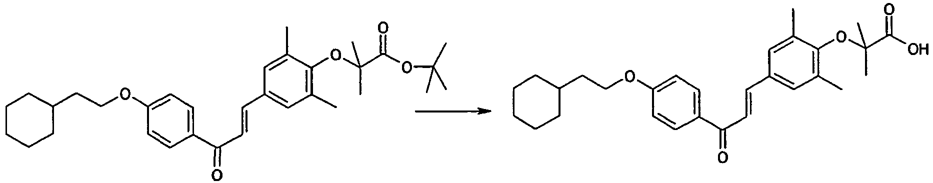

Ce composé est synthétisé à partir du composé 10 selon la méthode générale 6 précédemment décrite.

Purification par précipitation dans le mélange dichlorométhane-heptane.
RMN 1H CDCl₃ δppm : 0,90-1,30(m, 5H), 1,56(m, 7H), 1,70(m, 7H), 2,30(s, 6H), 4,09(t, 2H, J = 6,57Hz), 6,98(d, 2H, J = 9,09Hz), 7,32(s, 2H), 7,4(d, 1H, J = 15,60Hz), 7,71(d, 1H, J = 15,60Hz), 8,04 (d, 2H, J = 9,09Hz)
SM(ES-MS): 465,3(m+1)
F°C = 134,8-135,3

### Composé 12 selon l'invention :

### 1-(4-Méthylthio-3,5-diméthylphényl)-3-(4-tertiobutyloxycarbonyldiméthylméthyloxy-3,5-diméthylphényl) prop-2-èn-1-one

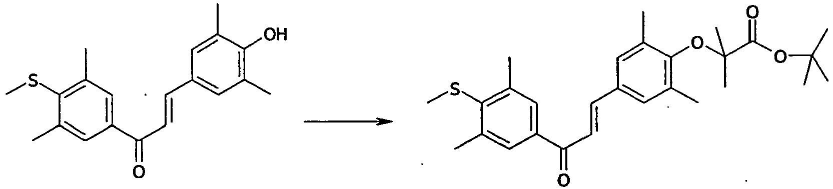

Ce composé est synthétisé à partir du composé intermédiaire 5 et de bromoisobutyrate de tertiobutyle selon la méthode générale 4 précédemment décrite.

Purification par chromatographie sur gel de silice (élution : cyclohexane-acétate d'éthyle : 8-2 ).
RMN 1H CDCl₃ δppm : 1,50(s, 6H), 1,51(s, 3H), 1,53 (s, 9H), 2,28(s, 6H), 2,63(s, 6H), 7,30(s, 2H), 7,39(d, 1H, J=15,69Hz), 7,69(d, 1H, J = 15.69Hz), 7,72(s, 2H)

### Composé 13 selon l'invention :

### 1-(4-Méthylthio-3,5-diméthylphényl)-3-(4-carboxydiméthylméthyloxy-3,5-diméthylphényl) prop-2-èn-1-one

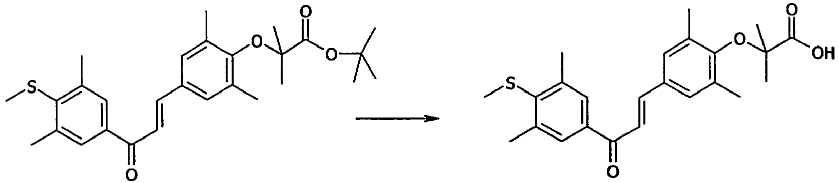

Ce composé est synthétisé à partir du composé 12 selon la méthode générale 6 précédemment décrite.

Purification par chromatographie sur gel de silice (élution : dichlorométhane-méthanol : 98-2).
RMN 1H DMSOd₆ δppm : 1,39(s, 6H), 2,22(s, 6H), 2,28(s, 3H), 2,59(s, 6H), 7,56(s, 2H), 7,62(d, 1H, J = 15,37Hz), 7,79(d, 1H, J = 15,37Hz), 7,89(s, 2H), 12,95 (s, 1H)
SM(ES-MS): 412,9(m+1)
F°C = 177,0-179,0

### Composé 14 selon l'invention :

### 1-(4-Propyloxy-3,5-diméthylphényl)-3-(4-tertiobutyloxycarbonyldiméthylméthyloxy-3,5-diméthylphényl)prop-2-èn-1-one

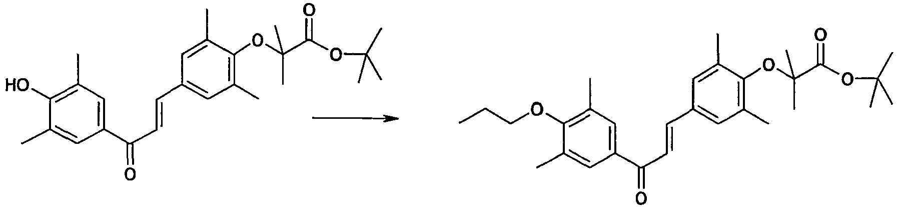

Ce composé est synthétisé à partir du composé 3 et de bromure de propyle selon la méthode générale 4 précédemment décrite, Le produit brut obtenu après élimination du carbonate de potassium et élimination des solvants par évaporation sous pression réduite a été utilisé pour la synthèse du composé 15.
RMN 1H CDCl₃ δppm : 1,09(t, 3H, J = 7,41 Hz), 1,46(s, 6H), 1,58(s, 9H), 1,83(m, 2H), 2,27(s, 6H), 2,35(s, 6H), 3,78 (t, 2H, J = 6,09Hz), 7,29(s, 2H), 7,41 (d, 1H, J = 15,32Hz), 7,68(d, 1H, J = 15,32Hz), 7,70(s, 2H)

### Composé 15 selon l'invention :

### 1-(4-Propyloxy-3,5-diméthylphényl)-3-(4-carboxydiméthylméthyloxy-3,5-diméthylphényl)prop-2-èn-1-one

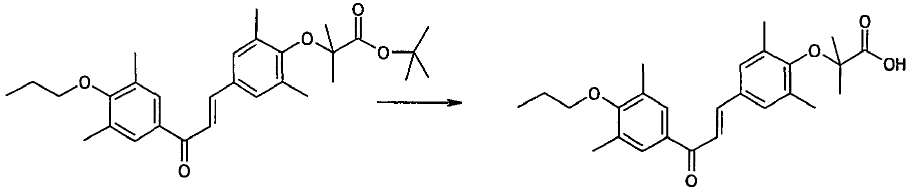

Ce composé est synthétisé à partir du composé 14 selon la méthode générale 6 précédemment décrite.

Purification par chromatographie sur gel de silice (élution : dichlorométhane-méthanol : 95-5).
RMN 1H CDCl₃ δppm : 1,05(t, 3H, J = 7,29Hz), 1,39(s, 6H), 1,78(m, 2H), 2,23(s, 6H), 2,32(s, 6H), 3,78(m, 2H), 7,56(s, 2H), 7,58(d, 1H, J = 16,26Hz), 7,80(d, 1H, J = 16,26Hz), 7,86(s, 2H)
SM(ES-MS): 424,9(m+1)
F°C = 188,5-189,7

### Composé 16 selon l'invention :

### 1-(4-Méthoxy-3,5-diméthylphényl)-3-(4-tertiobutyloxycarbonyldiméthylméthyloxy-3,5-diméthylphényl)prop-2-èn-1-one

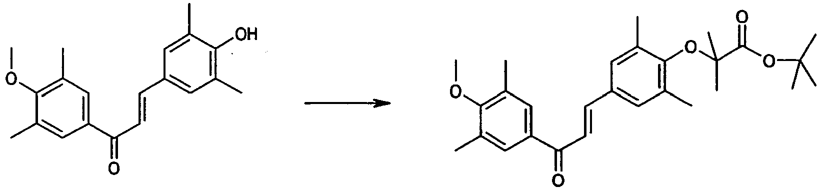

Ce composé est synthétisé à partir du composé intermédiaire 6 et de bromoisobutyrate de tertiobutyle selon la méthode générale 4 précédemment décrite.

Purification par chromatographie sur gel de silice (élution : cyclohexane-acétate d'éthyle : 95-5 ).
RMN 1H CDCl₃ δppm : 1,47(s, 9H), 1,53(s, 6H), 2,29(s, 6H), 2,31 (s, 6H), 3,79(s, 3H), 7,30(s, 2H), 7,40 (d, 1H, J = 15,50Hz), 7,70(d, 1H, J = 15,50Hz), 7,71 (s, 2H)

### Composé 17 selon l'invention :

### 1-(4-Méthoxy-3,5-diméthylphényl)-3-(4-carboxydiméthylméthyloxy-3,5-diméthylphényl)prop-2-èn-1-one

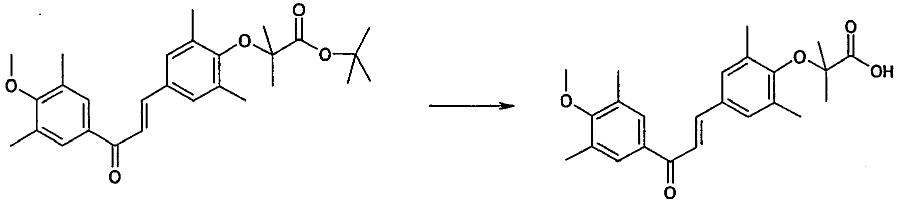

Ce composé est synthétisé à partir du composé 16 selon la méthode générale 6 précédemment décrite.

Purification par chromatographie sur gel de silice (élution : dichlorométhane-méthanol : 98-2).
RMN 1H CDCl₃ δppm : 1,57(s ,6H), 2,31 (s, 6H), 2,38( s, 6H), 3,79(s, 3H), 7,33(s, 2H), 7,43(d, 1H, J = 15,81 Hz), 7,71(d, 1H, J = 15,81 Hz), 7,72(s, 2H)
SM(ES-MS) : 396,9 (m+1)
F°C = 166,6-168,8

### Composé 18 selon l'invention :

### 1-(4-Hexyloxy-3,5-diméthylphényl)-3-(4-tertiobutyloxycarbonyldiméthyl méthyloxy - 3,5-diméthylphényl)prop-2-èn-1-one

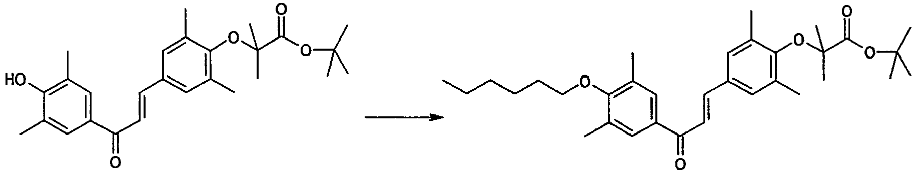

Ce composé est synthétisé à partir du composé 3 et de bromure d'hexyle selon la méthode générale 4 précédemment décrite. Le produit brut obtenu après élimination du carbonate de potassium et élimination des solvants par évaporation sous pression réduite a été utilisé pour la synthèse du composé 19.
RMN 1H CDCl₃ δppm : 0,93(t, 3H, J = 8,58Hz), 1,37(m, 4H), 1,47(s, 6H), 1,53(m, 11H), 1,83(m, 2H), 2,28(s, 6H), 2,36(s, 6H), 3,82(t, 2H, J = 6,54Hz), 7,29(s, 2H), 7,40(d, 1H, J = 15,57Hz), 7,70(d, 1H, J = 15,57Hz), 7,71 (s, 2H)

### Composé 19 selon l'invention :

### 1-(4-Hexyloxy-3,5-diméthylphényl)-3-(4-carboxydiméthylméthyloxy-3,5-diméthyl phényl)prop-2-èn-1-one

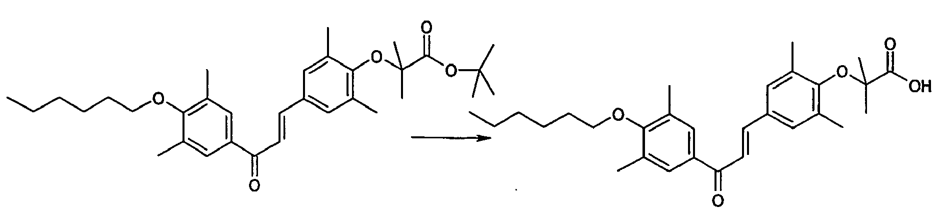

Ce composé est synthétisé à partir du composé 18 selon la méthode générale 6 précédemment décrite.

Purification par chromatographie sur gel de silice (élution : dichlorométhane-méthanol : 95-5).
RMN 1H CDCl₃ δppm : 0,93(t, 3H, J = 7,02Hz), 1,37(m, 4H), 1,50(m, 2H), 1,56(s, 6H), 1,83(m, 2H), 2,30(s, 6H), 2,34(s, 6H), 3,82(t, 2H, J = 6,57Hz), 7,32(s, 2H), 7,42(d, 1H, J = 15,48Hz), 7,69(d,1H, J = 15,48Hz), 7,71 (s, 2H)
SM(ES-MS) : 466,9(m+1)
F°C = 171,0-172,0

### Composé 20 selon l'invention :

### 1-(4-Cyclohexyléthyloxy-3, 5-diméthylphényl)-3-(4-tertiobutyloxycarbonyldiméthyl méthyloxy-3,5-diméthylphényl) prop-2-èn-1-one

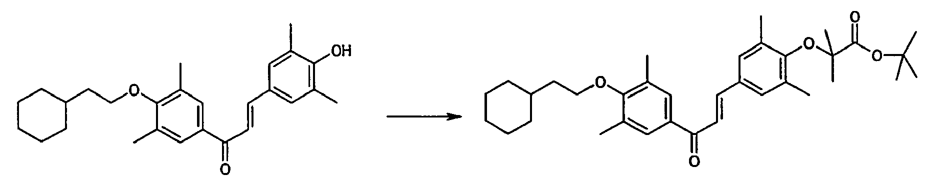

Ce composé est synthétisé à partir du composé intermédiaire 7 et de bromoisobutyrate de tertiobutyle selon la méthode générale 4 précédemment décrite.

Purification par chromatographie sur gel de silice (élution : cyclohexane-acétate d'éthyle: 85-15).
RMN 1H CDCl₃ δppm : 0,94-1,53(m, 28H), 2,28(s, 6H), 2,35(s, 6H), 3,86(t, 2H, J=6,75Hz), 7,29(s, 2H), 7,41(d, 1H, J=15,76Hz), 7,70 (d, 1H, J=15,76Hz), 7,71 (s, 2H)

### Composé 21 selon l'invention :

### 1-(4-Cyclohexyléthyloxy-3, 5-diméthylphényl)-3-(4-carboxydiméthylméthyloxy-3,5-diméthylphényl) prop-2-èn-1-one

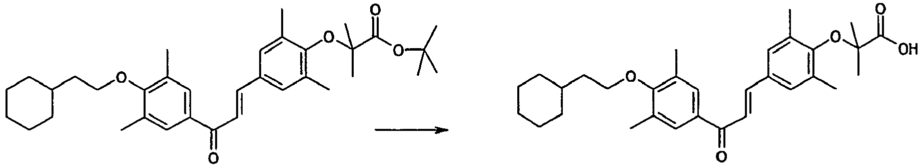

Ce composé est synthétisé à partir du composé 20 selon la méthode générale 6 précédemment décrite.

Purification par chromatographie sur gel de silice (élution : dichlorométhane-méthanol : 98-2).
RMN 1H CDCl₃ δppm : 0,97-1,04(m, 2H), 1,16-1,34(m, 4H), 1,56(s, 6H), 1,63-1,82(m, 7H), 2,30(s, 6H), 2,35(s, 6H), 3,86(t, 2H, J = 6,60Hz), 7,32(s, 2H), 7,43 (d, 1H, J = 15,81 Hz), 7,70(d, 1H, 15,81 Hz), 7,71 (s, 2H)
SM(ES-MS) : 492,9(m+1)
F°C = 166,4-167,7

### Composé 22 selon l'invention :

### 1-(4-Cyclohexylthioéthyloxyphényl)-3-(4-tertiobutyloxycarbonyldiméthylméthyloxy-3,5-diméthylphényl) prop-2-èn-1-one

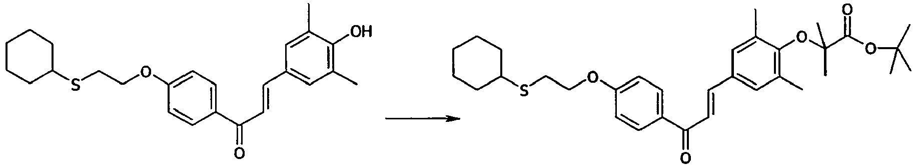

Ce composé est synthétisé à partir du composé intermédiaire 8 et de bromoisobutyrate de tertiobutyle selon la méthode générale 4 précédemment décrite.

Purification par chromatographie sur gel de silice (élution : cyclohexane-acétate d'éthyle : 7-3).
RMN 1H CDCl₃ δppm : 1,29(m, 5H), 1,46 (s, 6H), 1,53(s, 9H), 1,62(m, 1H), 1,80(m, 2H), 2,03(m, 2H), 2,27(s, 6H), 2,75(m, 1H), 2,95(t, 2H, 6,81Hz), 4,20(t, 2H, J = 6.81Hz), 6,97(d, 2H, J = 9,24Hz), 7,28(s, 2H), 7,43,(d, 1H, J = 15,78Hz), 7,70(d, 1H, J = 15,78Hz), 8,03(d, 2H, J = 9,24Hz)

### Composé 23 selon l'invention:

### 1-(4-Cyclohexylthioéthyloxyphényl)-3-(4-carboxydiméthylméthyloxy-3,5-diméthylphényl) prop-2-èn-1-one

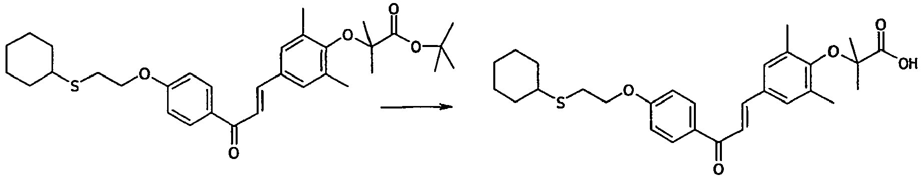

Ce composé est synthétisé à partir du composé 22 selon la méthode générale 6 précédemment décrite.

Purification par chromatographie sur gel de silice (élution : dichlorométhane-méthanol : 98-2).
RMN 1H CDCl₃ δppm : 1,27-1,38(m, 4H), 1,56(s, 6H), 1,63-1,66(m, 2H), 1,79-1,81 (m, 2H), 2,01-2,04(m, 2H), 2,30(s, 6H), 2,76-2,77(m, 1H), 2,96(t, 2H, J = 7,08 Hz), 4,21 (t, 2H, J = 7,08Hz), 6,97(d, 2H, J = 8,61 Hz), 7,31 (s, 2H), 7,41 (d, 1H, J = 15,60Hz), 7,73(d, 1H, J = 15,60Hz), 8,04(d, 2H, J = 8,61 Hz)
SM(Maldi-tof: 496,67(m+1)
F°C = 112,3-114

### Composé 24 selon l'invention :

### 1-(2,4,5-Triméthylphényl)-3-(4-tertiobutyloxycarbonyldiméthylméthyloxy-3,5-diméthylphényl) prop-2-èn-1-one

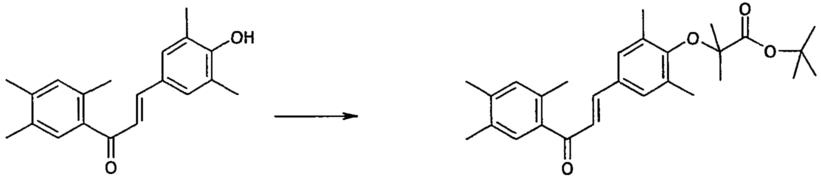

Ce composé est synthétisé à partir du composé intermédiaire 9 et de bromoisobutyrate de tertiobutyle selon la méthode générale 4 précédemment décrite.

Purification par chromatographie sur gel de silice (élution : cyclohexane-acétate d'éthyle : 8-2).
RMN 1H CDCl₃ δppm : 1,40-1,65(m, 15H), 2,22(s, 6H), 2,25(s, 3H), 2,28(s, 3H), 2,35(s, 3H), 7,00(s, 1H), 7,01 (d, 1H, J = 15,70Hz), 7,18 (s, 2H), 7,24(s, 1H), 7,35(d, 1H, 15,70Hz)

### Composé 25 selon l'invention:

### 1-(2,4,5-Triméthylphényl)-3-(4-carboxydiméthylméthyloxy-3,5-diméthylphényl) prop-2-èn-1-one

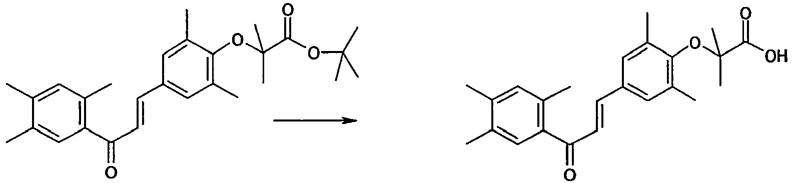

Ce composé est synthétisé à partir du composé 24 selon la méthode générale 6 précédemment décrite.

Purification par chromatographie sur gel de silice (élution : dichlrorométhane-méthanol: 98-2).
RMN 1H CDCl₃ δppm : 1,55(s, 6H), 2,27,(s, 6H), 2,27-2,30 (m, 6H), 2,39 (s, 3H), 7,05 (s, 1H), 7,07 (d, 1H, J = 15,24Hz), 7,24(s, 2H); 7,28 (s, 1H), 7,4 (d, 1H, J = 15,78Hz)
SM(ES-MS) : 381,2(m+1)
F°C = 168,7-173,3

### Composé 26 selon l'invention :

### 1-(4-Cyclohexylthioéthyloxy-3,5-diméthylphényl)-3-(4-tertiobutyloxycarbonyldiméthylméthyloxy-3,5-diméthylphényl) prop-2-èn-1-one

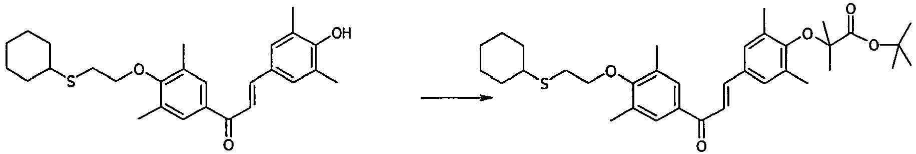

Ce composé est synthétisé à partir du composé intermédiaire 10 et de bromoisobutyrate de tertiobutyle selon la méthode générale 4 précédemment décrite.

Purification par chromatographie sur gel de silice (élution : cyclohexane-acétate d'éthyle : 95-5).
RMN 1H CDCl₃ δppm : 1,27-2,04 (m, 10H), 1,47(s, 6H), 1,53(s, 9H), 2,29(s, 6H), 2,38(s, 6H), 2,75(m, 1H), 2,98(t, 2H, J=6,84Hz), 3,98(t, 2H, J=6,84Hz), 7,29(s, 2H), 7,40(d, 1H, J=15,63Hz), 7,70(d, 1H, J=15,63Hz), 7,71(s, 2H)

### Composé 27 selon l'invention :

### 1-(4-Cyclohexylthioéthyloxy-3,5-diméthylphényl)-3-(4-carboxydiméthylméthyloxy-3,5-diméthylphényl)prop-2-èn-1-one

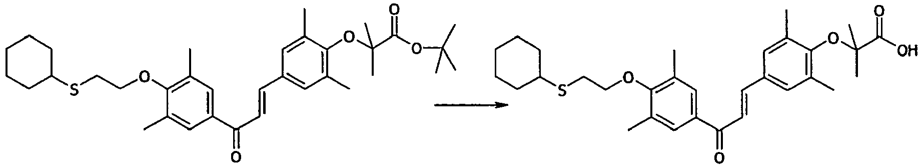

Ce composé est synthétisé à partir du composé 26 selon la méthode générale 6 précédemment décrite.

Purification par chromatographie sur gel de silice (élution : dichlorométhane-méthanol : 98-2).
RMN 1H CDCl₃ δppm : 1,26-1,42(m, 5H), 1,56(s, 6H), 1,62-1,64(m, 1H), 1,79-1,81 (m, 2H), 2,03-2,00(m, 2H), 2,3(s, 6H), 2,38(s, 6H), 2,71-2,78(m, 1H), 2,97(t, 2H, J = 7,00Hz), 3,98(t, 2H, J = 7,00Hz), 7,32(s, 2H), 7,43(d, 1H, J = 15,78Hz), 7,7(d, 1H, J = 15,24Hz), 7,71 (s, 2H)
SM(MALDI-TOF) : 524,78(m+1)
F°C = 156,0-158,0

### Composé 28 selon l'invention :

### 1-(4-Méthylthiophényl)-3-(4-tertiobutyloxycarbonyldiméthylméthyloxy-3-fluorophényl)prop-2-èn-1-one

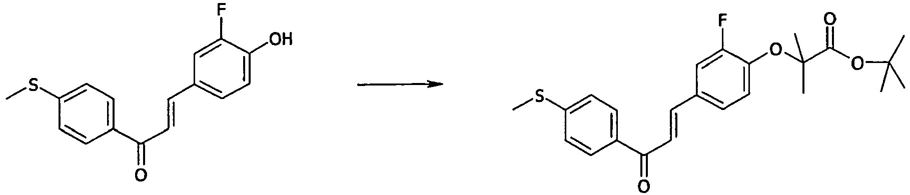

Ce composé est synthétisé à partir du composé intermédiaire 11 et de bromoisobutyrate de tertiobutyle selon la méthode générale 4 précédemment décrite.

Purification par chromatographie sur gel de silice (élution : cyclohexane-acétate d'éthyle : 8-2).
RMN 1H CDCl₃ δppm : 1,43(s, 9H), 1,62(s, 6H), 2,53(s, 3H), 6,95(t, 1H, J = 8,07 Hz), 7,32(d, 2H, J = 8,64Hz), 7,39(m, 3H), 7,72(d, 1H, J = 15,50Hz), 7,95(d, 2H, J = 8,64Hz)

### Composé 29 selon l'invention :

### 1-(4-Méthylthiophényl)-3-(4-carboxydiméthylméthyloxy-3-fluorophényl)prop-2-èn-1-one

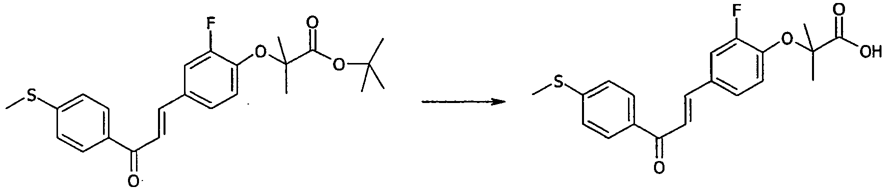

Ce composé est synthétisé à partir du composé 28 selon la méthode générale 6 précédemment décrite.

Il est purifié par précipitation dans un mélange dichlorométhane-heptane : 70-30. RMN 1H CDCl₃ δppm : 1,67(s, 6H), 2,56(s, 3H), 7,09(t, 1H, J = 8,19Hz), 7,32(m, 3H), 7,43 (m, 2H), 7,73(d, 1H, J = 15,24Hz), 8,73(d, 2H, J = 8,73Hz)
SM(ES-MS) : 375,1 (m+1)
F°C = 142,2-144,6

### Composé 30 selon l'invention :

### 1-(2,3,4,5,6-Pentaméthylphényl)-3-(4-tertiobutyloxycarbonyldiméthylméthyloxy-3,5-diméthylphényl)prop-2-èn-1-one

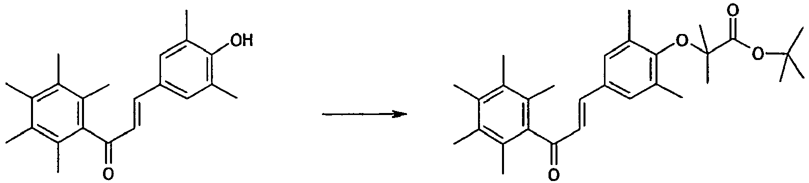

Ce composé est synthétisé à partir du composé intermédiaire 12 et de bromoisobutyrate de tertiobutyle selon la méthode générale 4 précédemment décrite.

Purification par chromatographie sur gel de silice (élution : cyclohexane-acétate d'éthyle : 95-5).
RMN 1H CDCl₃ δppm : 1,44(s, 6H), 1,53(s, 9H), 2,11 (s, 6H), 2,22(s, 6H), 2,23(s, 6H), 2,28(s, 3H), 6,84(d, 1H, J = 16,26Hz), 7,06(d, 1H, J = 16,26Hz), 7,16(s, 2H)

### Composé 31 selon l'invention :

### 1-(2,3,4,5,6-Pentaméthylphényl)-3-(4-carboxydiméthylméthyloxy-3,5-diméthylphényl)prop-2-èn-1-one

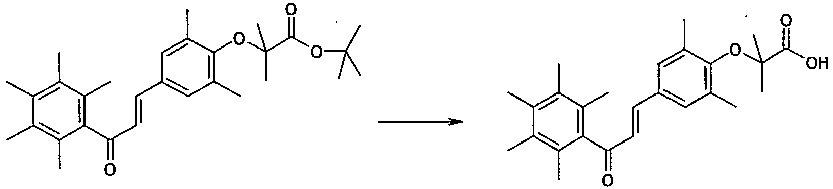

Ce composé est synthétisé à partir du composé 30 selon la méthode générale 6 précédemment décrite.

Purification par chromatographie sur gel de silice (élution : dichlorométhane-méthanol : 98-2).
RMN 1H CDCl₃ δppm : 1,53(s, 6H), 2,11 (s, 6H), 2,22(s, 6H), 2,24(s, 6H), 2,28(s, 3H), 6,87(d, 1H, J = 16,20Hz), 7,08(d, 1H, J = 16,20Hz), 7,19(s, 2H)
SM(ES-MS) : 409,1(m+1)
F°C = 192,8-194,2

### Composé 32 selon l'invention:

### 1-(4-Phényloxyphényl)-3-(4-tertiobutyloxycarbonyldiméthylméthyloxy-3,5-diméthylphényl)prop-2-èn-1-one

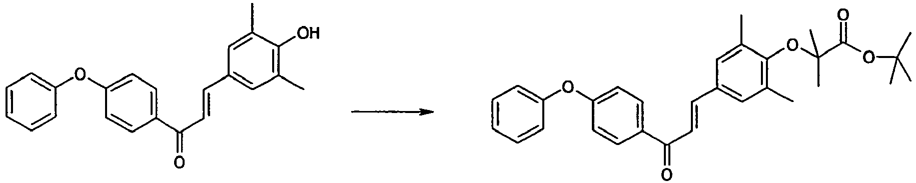

Ce composé est synthétisé à partir du composé intermédiaire 13 et de bromoisobutyrate de tertiobutyle selon la méthode générale 4 précédemment décrite.

Purification par chromatographie sur gel de silice (élution : cyclohexane-acétate d'éthyle : 7-3).
RMN 1H CDCl₃ δppm : 1,47(s, 6H), 1,53(s, 9H), 2,28(s, 6H), 7,02(d, 2H, J = 8,70Hz), 7,1(d, 2H, J = 7,92Hz), 7,21(t, 1H, J = 7,35Hz), 7,29(s, 2H), 7,39-7,46(m, 3H), 7,73(d, 1H, J = 16,20Hz), 8,04(d, 2H, J = 8,70Hz)

### Composé 33 selon l'invention :

### 1-(4-Phényloxyphényl)-3-(4-carboxydiméthylméthyloxy-3,5-diméthylphényl)prop-2-èn-1-one

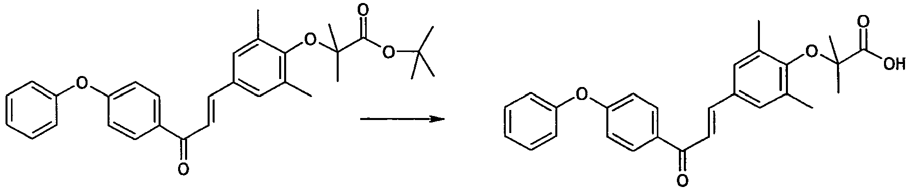

Ce composé est synthétisé à partir du composé 32 selon la méthode générale 6 précédemment décrite.

Purification par chromatographie sur gel de silice (élution : dichlorométhane-méthanol : 98-2).
RMN 1H DMSOd₆ δppm : 1,39(s, 6H), 2,22(s, 6H), 7,08(d, 2H, J = 8,55Hz), 7,15(d, 2H, J = 8,01Hz), 7,25(t, 1H, J = 7,41Hz), 7,47(t, 2H, J = 7,44Hz), 7,55(s, 2H), 7,62 (d, 1H, J = 15,70Hz), 7,82(d, 1H, J = 15,70Hz), 8,19(d, 2H, J = 8,55Hz)
SM(ES-MS): 430,9(m+1)
F°C = 154,0-156,0

### Composé 34 selon l'invention :

### 1-(4-Méthoxy-3-fluorophényl)-3-(4-tertiobutyloxycarbonyldiméthylméthyloxy-3,5-diméthylphényl)prop-2-èn-1-one

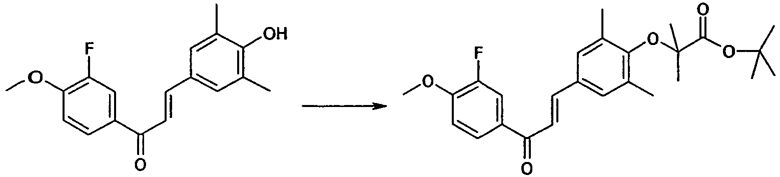

Ce composé est synthétisé à partir du composé intermédiaire 14 et de bromoisobutyrate de tertiobutyle selon la méthode générale 4 précédemment décrite.

Purification par chromatographie sur gel de silice (élution : cyclohexane-acétate d'éthyle : 8-2).
RMN 1H CDCl₃ δppm : 1,50(s, 6H), 1,53(s, 9H), 2,28(s, 6H), 3,98(s, 3H), 7,04(t, 1H, J = 8,07Hz), 7,29(s, 2H), 7,39(d, 1H, J = 15,70Hz), 7,73(d, 1H, J = 15,70Hz), 7,78-7,86 (m, 2H)

### Composé 35 selon l'invention :

### 1-(4-Méthoxy-3-fluorophényl)-3-(4-carboxydiméthylméthyloxy-3,5-diméthylphényl) prop-2-èn-1-one

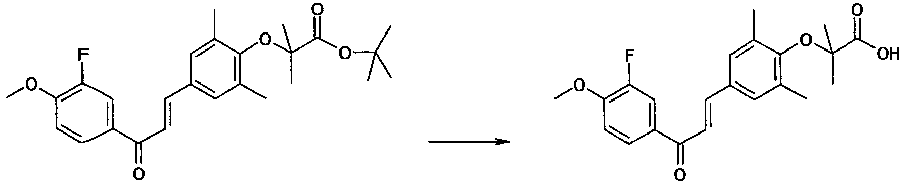

Ce composé est synthétisé à partir du composé 34 selon la méthode générale 6 précédemment décrite.

Purification par chromatographie sur gel de silice (élution : dichlorométhane-méthanol : 98-2).
RMN 1H DMSOd₆ δppm : 1,39(s, 6H), 2,22(s, 6H), 3,95(s, 3H), 7,31 (t, 1H, J = 7,35Hz), 7,57(s, 2H), 7,60(d, 1H, J = 15,78Hz), 7,83 (d, 1H, J = 15,78Hz), 7,99-8,06 (m, 2H)
SM(ES-MS): 387,1(m+1)
F°C = 167,0-169,0

### Composé 36 selon l'invention :

### 1-(4-Méthoxy-3-méthylphényl)-3-(4-tertiobutyloxycarbonyldiméthylméthyloxy-3,5-diméthylphényl)prop-2-èn-1-one

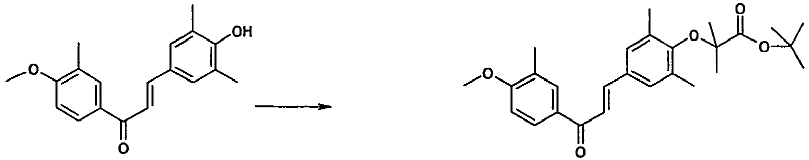

Ce composé est synthétisé à partir du composé intermédiaire 15 et de bromoisobutyrate de tertiobutyle selon la méthode générale 4 précédemment décrite.
RMN 1H CDCl₃ δppm : 1,46(s, 6H), 1,52(s, 9H), 2,27(s, 9H), 3,90(s, 3H), 6,88 (d, 1H, J=8,73Hz), 7,28(s, 2H), 7,45(d, 1H, J=16,11 Hz), 7,70(d, 1H, J=16,11Hz), 7,87(s, 1H), 7,92(dd, 1H, J=8,73Hz, J = 1,65Hz)

### Composé 37 selon l'invention :

### 1-(4-Méthoxy-3-méthylphényl)-3-(4-carboxydiméthylméthyloxy-3,5-diméthylphényl) prop-2-èn-1-one

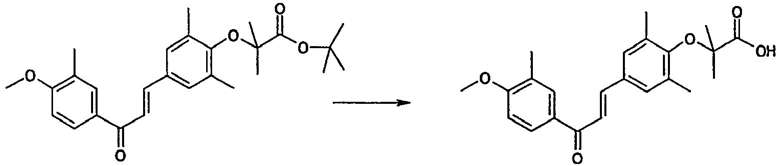

Ce composé est synthétisé à partir du composé 36 selon la méthode générale 6 précédemment décrite.

Purification par chromatographie sur gel de silice (élution : dichlorométhane-méthanol: 98-2) puis par recristallisation dans l'acétonitrile.
RMN 1H CDCl₃ δppm : 1,39(s, 6H), 2,22(s, 6H), 2,24(s, 3H), 3,90(s, 3H), 7,08 (d, 1H, J = 8,55Hz), 7,56 (s, 2H), 7,58 (d, 1H, J = 16,71 Hz), 7,82(d, 1H, J = 15,51 Hz), 7,99 (s, 1H), 8,06 (d, 1H, 8,55), 12,95(s, 1H)
SM(ES-MS): 383,2 (m+1)
F°C = 157,0-159,0

### Composé 38 selon l'invention :

### 1-(4-Hexylthio-3,5-diméthylphényl)-3-(4-tertiobutyloxycarbonyldiméthylméthyloxy-3,5-diméthylphényl) prop-2-èn-1-one

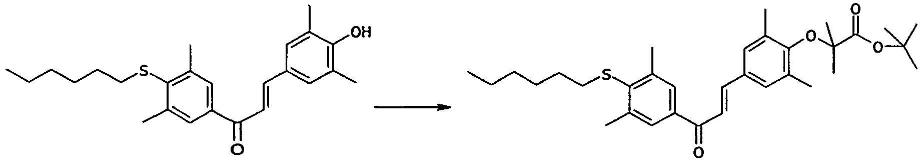

Ce composé est synthétisé à partir du composé intermédiaire 16 et de bromoisobutyrate de tertiobutyle selon la méthode générale 4 précédemment décrite.

Purification par chromatographie sur gel de silice (élution : cyclohexane-acétate d'éthyle : 9-1).
RMN 1H CDCl₃ δppm : 0,88(t, 3H, J = 6,84Hz), 1,25-1,62(m, 8H), 1,47(s, 6H), 1,53(s, 9H), 2,29(s, 6H), 2,62(s, 6H), 2,70(t, 2H, J = 6,96Hz), 7,30(s, 2H), 7,39(d, 1H, J = 15,90Hz), 7,70(d, 1H, J = 15,51 Hz), 7,71 (s, 2H)

### Composé 39 selon l'invention :

### 1-(4-Hexylthio-3,5-diméthylphényl)-3-(4-carboxydiméthylméthyloxy-3,5-diméthylphényl) prop-2-èn-1-one

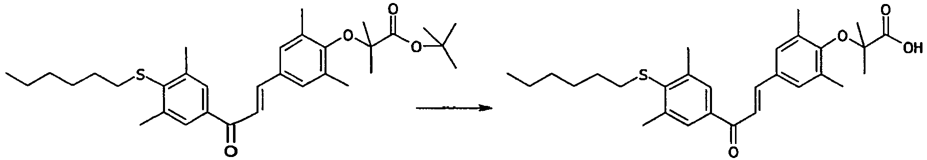

Ce composé est synthétisé à partir du composé 38 selon la méthode générale 6 précédemment décrite.

Purification par chromatographie sur gel de silice (élution : dichlorométhane-méthanol : 98-2).
RMN 1H DMSOd₆ δppm : 0,84(m, 3H), 1,22-1,40(m, 8H), 2,08(s, 6H), 2,22(s, 6H), 2,58(s, 6H), 2,73(t, 2H, J = 6,90Hz), 7,57(s, 2H), 7,63(d, 1H, J = 15,35Hz), 7,8(d, 1H, J = 15,35Hz), 7,89(s, 2H)
SM(ES-MS): 483,2(m+1)
F°C = 130,0-132,0

### Composé 40 selon l'invention :

### 1-(2,5-Diméthoxyphényl)-3-(4-tertiobutyloxycarbonyldiméthylméthyloxy-3,5-diméthylphényl)prop-2-èn-1-one

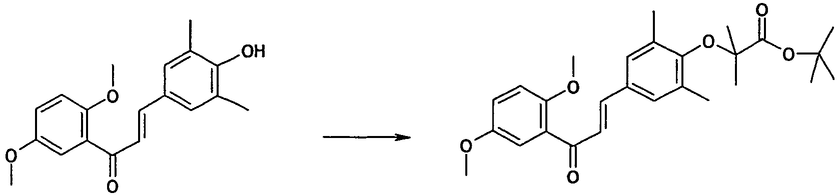

Ce composé est synthétisé à partir du composé intermédiaire 17 et de bromoisobutyrate de tertiobutyle selon la méthode générale 4 précédemment décrite.

Purification par chromatographie sur gel de silice (élution : cyclohexane-acétate d'éthyle : 7-3).
RMN 1H CDCl₃ δppm : 1,45(s, 6H), 1,52(s, 9H), 2,25(s, 6H), 3,81 (s, 3H), 3,86(s, 3H), 6,93(d, 1H, J = 9,24Hz), 7,01(dd, 1H, J = 8,82Hz, J = 2,7Hz), 7,14(d, 1H, J = 2,8Hz), 7,22 (s, 2H), 7,26 (d, 1H, 15,60Hz), 7,52 (d, 1H, J = 15,60Hz)

### Composé 41 selon l'invention :

### 1-(2,5-Diméthoxyphényl)-3-(4-carboxydiméthylméthyloxy-3,5-diméthylphényl)prop-2-èn-1-one

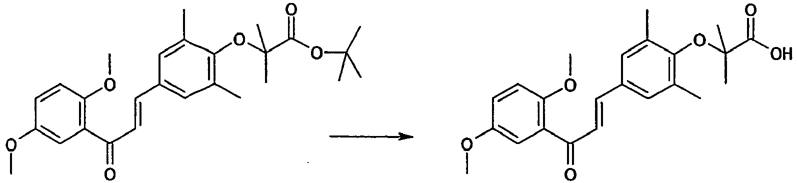

Ce composé est synthétisé à partir du composé 40 selon la méthode générale 6 précédemment décrite.

Purification par chromatographie sur gel de silice (élution : dichlorométhane-méthanol : 98-2).
RMN 1H DMSOd₆ δppm : 1,38 (s, 6H), 2,19 (s, 6H), 3,75 (s, 3H), 3,8 (s, 3H), 7,00 (d, 1H, J = 2,16 Hz), 7,12 (m, 2H), 7,26 (d, 1H, J = 16,2 Hz), 7,37 (d, 1H, J = 13,5Hz), 7,4 (s, 2H)
SM(ES-MS): 398,3(m-1)
F°C = produit huileux

### Composé 42 selon l'invention :

### Chlorhydrate de 1-(3,5-Diméthyl-4.-(morpholin-4-yléthyloxy)phényl)-3-(4-éthyloxycarbonyl diméthylméthyloxy-3,5-diméthylphényl)-prop-2-èn-1-one

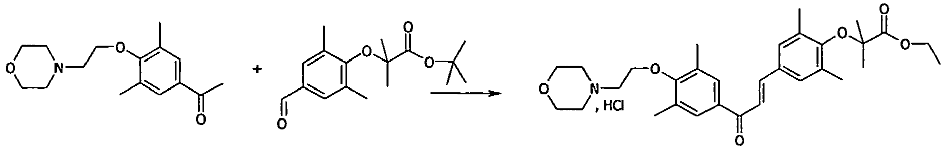

Ce composé est synthétisé à partir des matières premières 18 et 3 selon la méthode générale 1 précédemment décrite. Après élimination de l'éthanol par évaporation sous pression réduite. Le composé 42 est obtenu après trituration de l'huile résiduelle dans l'éther diéthylique.
RMN 1H CDCl₃ δppm : 1,36(t, 3H, J = 6,84Hz), 1,49(s, 6H), 2,24(s, 6H), 2,38(s, 6H), 3,20(s, 2H), 3,50(s, 2H), 3,73(d, 2H, J = 11,04Hz), 4,03(d, 2H, J = 11,04Hz), 4,30-4,45(m, 6H), 7,36(d, 1H, J = 15,75Hz), 7,28(s, 2H), 7,66(m, 3H), 13,39(s, 1H, N,HCl, éch/D2O)

### Composé 43 selon l'invention :

### 1-(3,5-Diméthyl-4-(morpholin-4-yléthyloxy)phényl)-3-(4-carboxydiméthylméthyloxy-3,5-diméthylphényl)-prop-2-èn-1-one

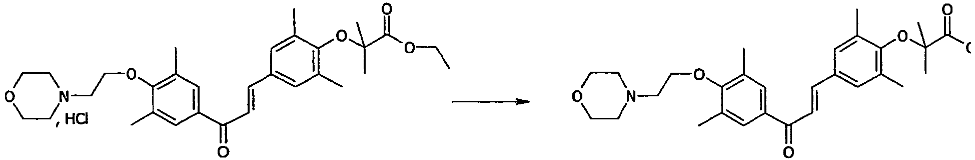

Le composé 42 est solubilisé dans de l'éthanol puis une solution de soude 2M est ajoutée. L'ensemble est maintenu 16h00 sous agitation à température ambiante . Le milieu réactionnel est versé dans de l'eau, la phase aqueuse est lavée par de l'acétate d'éthyle, neutralisée par addition d'acide acétique. La phase aqueuse est extraite par de l'éther diéthylique, Le précipité qui se forme dans la phase éthérée est essoré et recristallisé dans l'éthanol absolu.
RMN 1H CDCl₃ δppm : 1,50(s, 6H), 2,28(s, 6H), 2,36(s, 6H), 2,89(m, 4H), 3,06(t, 2H, J=5,46Hz), 3,87(m, 4H), 4,06(t, 2H, J=5,46Hz), 6,50(s, 1H), 7,40(d, 1H, J=15,78Hz), 7,27(s, 2H), 7,68 (m, 3H).
SM(MALDI-TOF): 496(m+1)
F°C = 167-169

### Composé 44 selon l'invention :

### 1-(4-Bromophényl)-3-(4-tertiobutyloxycarbonyldiméthylméthyloxy-3,5-difluorophényl)-prop-2-èn-1-one

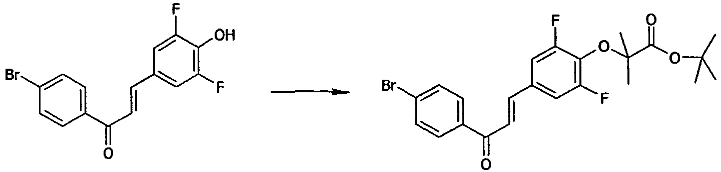

Ce composé est synthétisé à partir du composé intermédiaire 18 et de bromoisobutyrate de tertiobutyle selon la méthode générale 4 précédemment décrite.

Purification par chromatographie sur gel de silice (élution : cyclohexane-acétate d'éthyle : 95-5).
RMN 1H CDCl₃ δppm : 1,50(s, 9H), 1,57(s, 6H), 7,38(d, 2H, J=8,50Hz), 7,65(d, 1H, J=15,78Hz), 7,66(m, 3H), 7,89(d, 2H, J=8,50Hz)

### Composé 45 selon l'invention :

### 1-(4-Bromophényl)-3-(4-carboxydiméthylméthyloxy-3,5-difluorophényl)-prop-2-èn-1-one

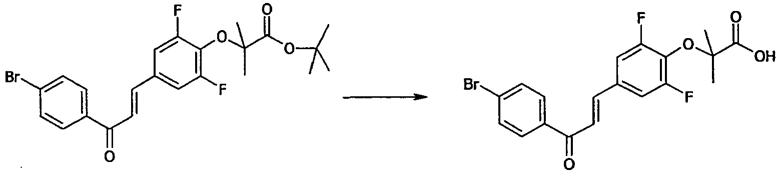

Ce composé est synthétisé à partir du composé 44 selon la méthode générale 6 précédemment décrite.

Purification par recristallisation dans l'éther diisopropylique.
RMN 1H CDCl₃ δppm : 1,65(s, 6H), 7,24(d, 2H, J= 8,50Hz), 7,41(d, 1H, J=15,84 Hz), 7,66(m, 3H), 7,89(d, 2H, J=8,50Hz)
SM(ES-MS): 425(m+1)
F°C = 142

### Composé 46 selon l'invention :

### 1-(4-Méthoxy-3-trifluorométhylphényl)-3-(4-tertiobutyloxycarbonyldiméthylméthyloxy-3,5-diméthylphényl)-prop-2-èn-1-one

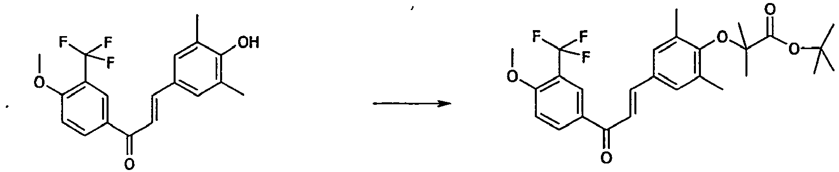

Ce composé est synthétisé à partir du composé intermédiaire 19 et de bromoisobutyrate de tertiobutyle selon la méthode générale 4 précédemment décrite.

Purification par chromatographie sur gel de silice (élution : cyclohexane-acétate d'éthyle : 9-1).
RMN 1H CDCl₃ δppm : 1,47(s, 6H), 1,53(s, 9H), 2,29(s, 6H), 4,00(s, 3H), 7,10(d, 1H, J = 8,65 Hz), 7,30(s, 2H), 7,40(d, 1H, J = 15,27Hz), 7,75(d, 1H), 8,25(d, 1H, J = 8,65Hz), 8,28(s, 1H)

### Composé 47 selon l'invention :

### 1-(4-Méthoxy-3-trifluorométhylphényl)-3-(4-carboxydiméthylméthyloxy-3,5-diméthylphényl)-prop-2-èn-1-one

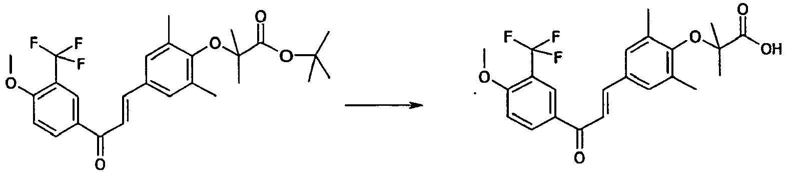

Ce composé est synthétisé à partir du composé 46 selon la méthode générale 6 précédemment décrite. Le composé 47 pur a été obtenu après élimination des solvants par évaporation sous pression réduite.
RMN 1H DMSOd₆ δppm : 1,40(s, 6H), 2,23(s, 6H), 4,03(s, 3H), 7,43(d, 1H, J= 8,7Hz), 7,60(s, 2H), 7,65(d, 1H, J=15,40Hz), 7,88(d, 1H, J=15,40Hz), 8,31 (s, 1H), 8,51 (d, 1H, J= 8,70Hz), 12,80(s, 1H).
SM(ES-MS): 437,3(m+1)
F°C = 182,5

### Exemple 2 : Evaluation des propriétés antioxydantes des composés selon l'invention

### Protection de l'oxydation des LDL induite par le cuivre:

Les composés testés sont les composés selon l'invention dont la préparation est décrite dans les exemples décrits ci-dessus.

L'oxydation des LDL est une modification importante et joue un rôle prépondérant dans la mise en place et le développement de l'athérosclérose (Jurgens, Hoff *et al.* 1987). Le protocole suivant permet la mise en évidence des propriétés antioxydantes des composés. Sauf mention différente, les réactifs proviennent de chez Sigma (St Quentin, France).

Les LDL sont préparés suivant la méthode décrite par Lebeau et al. (Lebeau, Furman *et al.* 2000).

Les solutions de composés à tester sont préparées à 10⁻² M dans un tampon bicarbonate (pH = 9) et diluées dans du PBS pour avoir des concentrations finales allant de 0,1 à 100 µM.

Avant l'oxydation, l'EDTA est retiré de la préparation de LDL par dialyse. L'oxydation a ensuite lieu à 30°C en ajoutant 100 µl d'une solution à 16,6 µM de CuSO₄ à 160 µL de LDL (125 µg de protéines/ml) et 20 µl d'une solution du composé à tester. La formation de diènes, l'espèce à observer, se mesure par densité optique à 232 nm dans les échantillons traités avec les composés avec ou sans cuivre. La mesure de la densité optique à 232 nm est réalisée toutes les 10 minutes pendant 8 heures à l'aide d'un spectrophotomètre thermostaté (tecan Ultra 380). Les analyses sont réalisées en triplicata. Nous considérons que les composés ont une activité antioxydante lorsqu'ils induisent un retardement de la *lag phase* et diminuent la vitesse d'oxydation et la quantité de diènes formés par rapport à l'échantillon témoin. Les inventeurs mettent en évidence que les composés selon l'invention présentent au moins une des propriétés antioxydantes citées ci dessus, ceci indiquant que les composés selon l'invention possèdent un caractère antioxydant intrinsèque.

Des exemples de résultats sont donnés sur les figures 1 a, 1b, 1 c, 2a, 2b, 2c, 3a, 4a, 4b, 4c, 5a, 5b, 5c, 6a, 6b, 6c, 7a, 7b, 7c, 8a, 8b, 8c où les propriétés antioxydantes des composés selon l'invention sont illustrées.

### Exemple 3: mesure des propriétés antioxydantes des composés selon l'invention sur des cultures de cellules

### Protocole de culture :

Les lignées cellulaires utilisées pour ce type d'expériences sont de type neuronales, neuroblastomes (humains) et cellules PC12 (rat). Les cellules PC12 ont été préparées à partir d'un phéochromocytome de rat et sont caractérisées par la méthode de Greene et Tischler (Greene and Tischler, 1976). Ces cellules sont couramment utilisées pour des études de différenciation neuronale, transduction du signal et mort neuronale. Les cellules PC12 sont cultivées comme précédemment décrit (Farinelli, Park *et al.* 1996), dans du milieu complet RPMI (Invitrogen) complémenté avec 10% de sérum de cheval et 5% de sérum de veau foetal.

Des cultures (primaires) de cellules endothéliales et de muscles lisses sont également utilisées. Les cellules sont commandées chez Promocell (Promocell GmBH, Heidelberg) et sont cultivées selon les indications du fournisseur.

Les cellules sont traitées avec différentes doses de composés de 5 à 300 µM pendant 24 heures. les cellules sont alors récupérées et l'augmentation de l'expression des gènes cibles est évaluée par PCR quantitative.

### Mesure des ARNm :

Les ARNm sont extraits des cellules en culture traitées ou non avec les composés selon l'invention. L'extraction est réalisée à l'aide des réactifs du kit Absolutely RNA RT-PCR miniprep Kit (Stratagene, France) selon les indications du fournisseur. Les ARNm sont ensuite dosés par spectrométrie et quantifiés par RT-PCR quantitative à l'aide du kit Light Cycler Fast start DNA Master Sybr Green I kit (Roche) sur un appareil Light Cycler System (Roche, France). Des paires d'amorces spécifiques des gènes de la Super Oxyde Dismutase (SOD), de la Catalase et de la Glutathion Peroxydase (GPx), enzymes anti-oxydantes, sont utilisées comme sondes. Des paires d'amorces spécifiques des gènes β-actine et cyclophiline sont utilisées comme sondes témoin.

L'augmentation de l'expression des ARNm, mesurée par RT-PCR quantitative, des gènes des enzymes antioxydantes est mise en évidence dans les différents types cellulaires utilisés, lorsque les cellules sont traitées avec les composés selon l'invention.

### Contrôle du stress Oxydatif :

### Mesure des espèces oxydantes dans les cellules en culture :

Les propriétés antioxydantes des composés sont également évaluées à l'aide d'un indicateur fluorescent dont l'oxydation est suivie par l'apparition d'un signal fluorescent. La diminution d'intensité du signal fluorescent émis est mesurée dans les cellules traitées avec les composés de la manière suivante : les cellules PC12 cultivées comme précédemment décrit (plaques noires 96 puits fonds transparents, Falcon) sont incubées avec des doses croissantes de H₂O₂ (0,25 mM - 1 mM) dans du milieu sans sérum pendant 2 à 24 heures. Après l'incubation, le milieu est enlevé et les cellules sont incubées avec une solution de dichlorodihydrofluorescéine diacetate (DCFDA, Molecular Probes, Eugene, USA) 10 µM dans du PBS pendant 30 min à 37°C et dans une atmosphère contenant 5% de CO₂. Les cellules sont ensuite rincées avec du PBS. La détection de la fluorescence émise par l'indicateur de l'oxydation est mesurée à l'aide d'un fluorimètre (Tecan Ultra 384) à une longueur d'onde d'excitation de 495 nm et une longueur d'onde d'émission de 535 nm. Les résultats sont exprimés en pourcentage de protection par rapport au témoin oxydé.

L'intensité de fluorescence est plus faible dans les cellules incubées avec les composés selon l'invention que dans les cellules non traitées. Ces résultats indiquent que les composés selon l'invention favorisent l'inhibition de la production d'espèces oxydantes dans des cellules soumises à un stress oxydatif. Les propriétés antioxydantes décrites précédemment sont également efficaces pour induire une protection antiradicalaire dans des cellules en culture.

### Mesure de la peroxydation lipidique :

L'effet protecteur des composés sur la peroxydation lipidique sur des cultures de cellules (modèles cellulaires cités précédemment) est déterminé de la façon suivante : les différentes lignées cellulaires ainsi que les cellules en culture primaire sont traitées comme précédemment, le surnageant des cellules est récupéré après le traitement et les cellules sont lysées et récupérées pour la détermination de la concentration protéique. La détection de la peroxydation lipidique est déterminée de la manière suivante :

La peroxydation lipidique est mesurée à l'aide d'acide thiobarbiturique (TBA) qui réagit avec la lipoperoxydation des aldéhydes tel que le malondialdéhyde (MDA). Après les traitements, le surnageant des cellules est collecté (900 NI) et 90 NI d'hydroxytoluène butylé y sont ajoutés (Morliere, Moysan et al. 1991). 1 ml d'une solution de TBA à 0,375% dans 0,25M HCL contenant 15% d'acide trichloroacétique est également ajouté aux milieux réactionnels. Le mélange est chauffé à 80°C pendant 15 min, refroidi sur glace et la phase organique est extraite avec du butanol. L'analyse de la phase organique se fait par spectrofluorométrie (λ_{exc}=515 nm et λₑₘ=550 nm) à l'aide du spectrofluorimètre Shimazu 1501 (Shimadzu Corporation, Kyoto, Japon). Les TBARS sont exprimés en équivalents MDA en utilisant un standard le tetra-ethoxypropane. Les résultats sont normalisés par rapport au contenu en protéines.

La diminution de la peroxydation lipidique observée dans les cellules traitées avec les composés selon l'invention confirme les résultats obtenus précédemment.

Les composés selon l'invention présentent avantageusement des propriétés antioxydantes intrinsèques qui permettent de ralentir et/ou d'inhiber les effets d'un stress oxydatif. Les inventeurs montrent également que les composés selon l'invention sont capables d'induire l'expression des gènes d'enzymes antioxydants. Ces caractéristiques particulières des composés selon l'invention permettent aux cellules de lutter plus efficacement contre le stress oxydatif et donc d'être protégées vis à vis des dommages induits par les radicaux libres.

### Exemple 4 : Evaluation de l'activation des PPARs in vitro par les composés selon l'invention

Les composés selon l'invention testés sont les composés possédant une fonction acide carboxylique et dont la préparation est décrite dans les exemples décrits ci-dessus.

Les récepteurs nucléaires membres de la sous-famille des PPARs qui sont activés par deux classes majeures de composés pharmaceutiques, les fibrates et les glitazones, abondamment utilisées en clinique humaine pour le traitement des dyslipidémies et du diabète, jouent un rôle important dans l'homéostasie lipidique et glucidique. Les données expérimentales suivantes montrent que les composés selon l'invention activent PPARα,PPARγ et PPAR□ *in vitro.*

L'activation des PPARs est évaluée *in vitro* dans des lignées cellulaires de type epithéloïde RK13 ou COS-7 par la mesure de l'activité transcriptionnelle de chimères constituées du domaine de liaison à l'ADN du facteur de transcription Gal4 de levure et du domaine de liaison du ligand des différents PPARs. Ces derniers résultats sont ensuite confirmés dans des lignées cellulaires selon les protocoles suivants :
L'exemple est donné pour les cellules RK13 et les cellules COS-7.

### Protocoles de culture

Les cellules RK13 proviennent de l' ECACC (Porton Down, UK), les cellules COS-7 proviennent de l'ATCC (American Type Culture Collection) et sont cultivées dans du milieu DMEM supplémenté de 10% vol/vol de sérum de veau foetal, 100 U/ml pénicilline (Gibco, Paisley, UK) et de 2 mM L-Glutamine (Gibco, Paisley, UK). Le milieu de culture est changé tous les deux jours. Les cellules sont conservées à 37°C dans une atmosphère humide contenant 5% de CO₂ et 95% d'air.

### Description des plasmides utilisés en transfection

Les plasmides pG5TkpGL3, pRL-CMV, pGal4-hPPARa, pGal4-hPPARγ, pGal4-hPPARδ et pGal4-φ ont été décrits par Raspe, Madsen et al. (1999). Les constructions pGal4-mPPARα, pGal4-hPPARγ et pGal4-hPPARδ ont été obtenues par clonage dans le vecteur pGal4-φ de fragments d'ADN amplifiés par PCR correspondants aux domaines DEF des récepteurs nucléaires PPARα, PPARγ et PPARδ humains.

### Transfection

Les cellules RK13 sont ensemencées dans des boîtes de culture de 24 puits à raison de 5x10⁴ cellules/puits, les cellules COS-7 dans des plaques de 96 puits à raison de 5x10⁴ cellules/puits et sont transfectées pendant 2 heures avec le plasmide rapporteur pG5TkpGL3 (50 ng/puits), les vecteurs d'expression pGal4-φ, pGal4-mPPARα, pGal4-hPPARα, pGal4-hPPARγ, pGal4-hPPARδ (100 ng/puits) et le vecteur de contrôle de l'efficacité de transfection pRL-CMV (1 ng/puits) suivant le protocole décrit précédemment (Raspe, Madsen et al. 1999) puis incubées pendant 36 heures avec les composés testés. A l'issue de l'expérience, les cellules sont lysées (Gibco, Paisley, UK) et les activités luciférase sont déterminées à l'aide du kit de dosage Dual-Luciferase^{™} Reporter Assay System (Promega, Madison, WI, USA) pour les cellules RK13 et Steady Glow Luciferase (Promega) pour les cellules COS-7 selon la notice du fournisseur comme décrit précédemment. Le contenu en protéines des extraits cellulaires est ensuite évalué à l'aide du kit de dosage Bio-Rad Protein Assay (Bio-Rad, München, Allemagne) selon la notice du fournisseur.

Les inventeurs mettent en évidence une augmentation de l'activité luciférase dans les cellules traitées avec les composés selon l'invention et transfectées avec le plasmide pGal4-hPPARα. Cette induction de l'activité luciférase indique que les composés selon l'invention, sont des activateurs de PPARα. Des exemples de résultats sont donnés sur les figures 9a, 10a, 11a, 12a, 13a, 14a, 15a, 16a, 17a et 18a où les propriétés activatrices PPARα des composés selon l'invention sont illustrées.

Les inventeurs mettent en évidence une augmentation de l'activité luciférase dans les cellules traitées avec les composés selon l'invention et transfectées avec le plasmide pGal4-hPPARγ. Cette induction de l'activité luciférase indique que les composés selon l'invention sont des activateurs de PPARγ.

Des exemples de résultats sont représentés par la figure 9b, 10b, 11b, 12b, 14b, 15b et 18b où les propriétés activatrices PPARγ des composés sont illustrées. Les inventeurs mettent en évidence une augmentation de l'activité luciférase dans les cellules traitées avec les composés selon l'invention et transfectées avec le plasmide pGal4-hPPARδ. Cette induction de l'activité luciférase indique que les composés selon l'invention sont des activateurs de PPARδ.

Des exemples de résultats sont représentés par la figure 11 c, 13b, 14c et 15c où les propriétés activatrices PPARδ des composés sont illustrées.

### Exemple 5: évaluation des propriétés anti-inflammatoires des composés selon l'invention

La réponse inflammatoire apparaît dans de nombreux désordres neurologiques, comme les scléroses multiples, les maladie d'Alzheimer et de Parkinson, les ischémies cérébrales et les accidents traumatiques du cerveau. De plus, l'inflammation est l'un des facteurs importants de la neurodégénérescence. Lors d'accidents cérébraux, une des premières réactions des cellules de la glie est de libérer des cytokines et des radicaux libres. La conséquence de cette libération de cytokines et de radicaux libres est une réponse inflammatoire au niveau cérébral qui peut mener à la mort des neurones (Rothwell, 1997).

Les lignées cellulaires et les cellules primaires sont cultivées comme décrit précédemment.

Le LPS (LipoPolySaccharide), endotoxine bactérienne (*Escherichia coli* 011 1 :B4) (Sigma, France), est reconstitué dans de l'eau distillée et conservée à 4°C. les cellules sont traitées avec une concentration de LPS de 1 µg/ml pendant 24 heures. Pour éviter toutes interférences avec d'autres facteurs le milieu de culture des cellules est totalement changé.

Le TNF-α est un facteur important de la réponse inflammatoire à un stress (oxydant par exemple). Pour évaluer la sécrétion de TNF-α en réponse à une stimulation par des doses croissantes de LPS, le milieu de culture des cellules stimulées est prélevé et la quantité de TNF-α est évaluée avec un kit ELISA-TNF-α (Immunotech, France). Les échantillons sont dilués 50 fois afin d'être en adéquation avec la gamme étalon (Chang, Hudson *et al.* 2000).

La propriété anti-inflammatoire des composés est caractérisée de la manière suivante : le milieu de culture des cellules est totalement changé et les cellules sont incubées avec les composés à tester pendant 2 heures. Après cette incubation, du LPS est rajouté au milieu de culture à une concentration finale de 1 µg/ml. Après 24 heures d'incubation, le surnageant de cellules est récupéré et stocké à -80°C lorsqu'il n'est pas traité directement. Les cellules sont lysées et la quantité de protéines est quantifiée, à l'aide du kit de dosage Bio-Rad Protein Assay (Bio-Rad, München, Allemagne) selon la notice du fournisseur.

La mesure de la diminution de sécrétion de TNF-α favorisée par le traitement avec les composés testés est exprimée en pg/ml/µg de protéine et rapportée en pourcentage par rapport au témoin. Ces résultats montrent que les composés selon l'invention possèdent des propriétés anti-inflammatoires.

### Exemple 6 : Evaluation des effets sur le métabolisme lipidique in vivo

Les composés selon l'invention testés sont les composés dont la préparation est décrite dans les exemples décrits ci-dessus.

Les fibrates, abondamment utilisés en clinique humaine pour le traitement des dyslipidémies impliquées dans le développement de l'athérosclérose, une des principales causes de mortalité et de morbidité dans les sociétés occidentales, sont de puissants activateurs du récepteur nucléaire PPARα. Celui-ci régule l'expression de gènes impliqués dans le transport (apolipoprotéines telles que Apo Al, Apo AII et Apo CIII, transporteurs membranaires tel que FAT) ou le catabolisme des lipides (ACO, CPT-I ou CPT-II). Un traitement par les activateurs de PPARα se traduit donc, chez l'homme et le rongeur, par une diminution des taux circulants de cholestérol et de triglycérides.

Les protocoles suivants permettent de mettre en évidence une baisse du taux de triglycérides et du taux de cholestérol circulant, ainsi que l'intérêt des composés selon l'invention dans le cadre de la prévention et/ou du traitement des maladies cardio-vasculaires.

### Traitement des animaux

Des souris transgéniques Apo E2/E2 sont maintenues sous un cycle lumière/obscurité de 12/12 heures à une température constante de 20 ± 3°C. Après une acclimatation d'une semaine, les souris sont pesées et rassemblées par groupes de 6 animaux sélectionnés de telle sorte que la distribution de leur poids corporel soit uniforme. Les composés testés sont suspendus dans la carboxyméthylcellulose et administrés par gavage intra-gastrique, à raison d'une fois par jour pendant 7 jours, aux doses indiquées. Les animaux ont un accès libre à l'eau et à la nourriture. A l'issue de l'expérience les animaux sont pesés et sacrifiés sous anesthésie. Le sang est collecté sur EDTA. Le plasma est préparé par centrifugation à 3000 tours/minutes pendant 20 minutes. Des échantillons de foie sont prélevés et conservés congelés dans de l'azote liquide pour analyse ultérieure.

### Mesure des lipides et apolipoprotéines sériques

Les concentrations sériques des lipides (cholestérol total et cholestérol libre, triglycérides et phospholipides) sont mesurées par dosage colorimétrique (Boehringer, Mannheim, Allemagne) selon les indications du fournisseur. Les concentrations sériques des apolipoprotéines AI, AII et CIII sont mesurées selon les méthodes décrites précédemment (Raspé et al. 1999, Asset G *et al.,* Lipids, 1999).

Un exemple de résultats est donné sur les figures 19a, 19b, 19c et 19d où l'activité du composé 2 sur le métabolisme des triglycérides et du cholestérol est illustrée. Un exemple de résultats est donné sur les figures 20a, 20b, 20c et 20d où l'activité des composés 13, 33 et 39 sur le métabolisme des triglycérides et du cholestérol est illustrée.

### Analyse des ARNs

L'ARN total a été isolé des fragments de foie par extraction à l'aide du mélange thiocyanate de guanidine/phénol acide/chloroforme suivant le protocole décrit précédemment (Raspé *et al.* 1999). Les ARN messagers ont été quantifiés par RT-PCR quantitative à l'aide du kit Light Cycler Fast Start DNA Master Sybr Green I kit (Hoffman-La Roche, Basel, Suisse) sur un appareil Light Cycler System (Hoffman-La Roche, Basel, Suisse). Des paires d'amorces spécifiques des gènes ACO, Apo CIII et Apo AII ont été utilisées comme sondes. Des paires d'amorces spécifiques des gènes 36B4, β-actine et cyclophiline ont été utilisées comme sondes témoin. Alternativement, l'ARN total a été analysé par Northern Blot ou Dot Blot suivant le protocole décrit précédemment (Raspé et al., 1999).

### Exemple 7 : Evaluation des effets neuro-protecteurs des composés selon l'invention dans un modèle d'ischémie-reperfusion cérébral

### Modèle Prophylactique

### 1/ Traitements des animaux

### 1.1 Animaux et administration des composés

Des souris C57 black/6 (sauvages) sont utilisées pour cette expérience.

Les animaux sont maintenus sous un cycle lumière/obscurité de 12/12 heures à une température de 20 +/- 3°C. Les animaux ont un accès libre à l'eau et à la nourriture. La prise de nourriture et la prise de poids sont enregistrées.

Les animaux sont traités par gavage avec les composés selon l'invention ou le véhicule (carboxycellulose 0,5%), pendant 14 jours avant l'induction de l'ischémie de l'artère moyenne cérébrale.

### 1.2 induction d'une ischémie-reperfusion par occlusion intraluminale de l'artère moyenne cérébrale :

Les animaux sont anesthésiés à l'aide d'une injection intra-péritonéale de 300 mg/kg d'hydrate de chloral. Une sonde rectale est mise en place et la température du corps est maintenue à 37 +/- 0,5°C. La pression artérielle est mesurée au cours de toute l'expérience.

Sous un microscope chirurgical, la carotide droite est mise à jour à l'aide d'une incision cervicale médiale. L'artère ptérygopalatine a été ligaturée à son origine et une artèriotomie est réalisée dans l'artère carotide externe afin d'y glisser un mono-filament de nylon. Ce filament est alors doucement avancé dans l'artère carotide commune puis dans l'artère carotide interne afin d'obturer l'origine de l'artère cérébrale moyenne. Après 1 heure, le filament est retiré pour permettre la reperfusion.

### 2/ Mesure du volume de l'infarctus cérébral :

24 heures après la reperfusion, les animaux préalablement traités ou non traités avec les composés sont tués par une overdose de pentobarbital.

Les cerveaux sont rapidement congelés et sectionnés. Les sections sont colorées au violet Cresyl. Les zones non colorées des sections cérébrales ont été considérées comme lésées par l'infarctus. Les aires ont été mesurées et les volumes de l'infarctus et des deux hémisphères ont été calculés par la formule suivante (Volume de l'infarctus corrigé = Volume de l'infarctus - (volume de l'hémisphère droit - volume de l'hémisphère gauche)) pour compenser l'oedème cérébral.

L'analyse des coupes de cerveaux d'animaux traités révèle une nette diminution du volume de l'infarctus par rapport aux animaux non traités. Lorsque les composés selon l'invention sont administrés aux animaux avant l'ischémie (effet prophylactique), ils sont capables d'induire une neuroprotection.

### 3/ Mesure de l'activité des enzymes anti-oxydantes :

Les cerveaux des souris sont congelés, écrasés et réduits en poudres puis re-suspendus dans une solution saline. Les différentes activités enzymatiques sont ensuite mesurées comme décrits par les auteurs suivants : superoxide dismutase (Flohe and Otting 1984); glutathion peroxidase (Paglia and Valentine 1967); glutathion reductase (Spooner, Delides et al. 1981); glutathion-S-transferase (Habig and Jakoby 1981); catalase (Aebi 1984).

Les différentes activités enzymatiques mentionnées ci-dessus sont augmentées dans les préparations de cerveaux des animaux traités avec les composés selon l'invention.

### Modèle curatif ou traitement de la phase aiguë

### 1/ Induction d'une ischémie-reperfusion par occlusion intraluminale de l'artère moyenne cérébrale.

Des animaux tels que décrits précédemment sont utilisés pour cette expérience. Les animaux sont anesthésiés à l'aide d'une injection intra-péritonéale de 300 mg/kg d'hydrate de chloral. Une sonde rectale est mise en place et la température du corps est maintenue à 37 +/- 0,5°C. La pression artérielle est mesurée au cours de toute l'expérience.

Sous un microscope chirurgical, la carotide droite est mise à jour à l'aide d'une incision cervicale médiale. L'artère ptérygopalatine a été ligaturée à son origine et une artériotomie est réalisée dans l'artère carotide externe afin d'y glisser un mono-filament de nylon. Ce filament est ensuite doucement avancé dans l'artère carotide commune puis dans l'artère carotide interne afin d'obturer l'origine de l'artère cérébrale moyenne. Après 1 heure, le filament est retiré pour permettre la reperfusion.

### 2/ Traitement des animaux :

Les animaux ayant subi une ischémie-reperfusion préalable sont traités par les composés selon l'invention pendant 24 ou 72 heures par voie orale (gavage), 2 fois par jour.

### 3/ Mesure du volume de l'infarctus cérébral :

24 ou 72 heures après la reperfusion, les animaux préalablement traités ou non traités avec les composés sont tués par une overdose de pentobarbital.

Les cerveaux sont rapidement congelés et sectionnés. Les sections sont colorées au violet Cresyl. Les zones non colorées des sections cérébrales ont été considérées comme lésées par l'infarctus. Les aires ont été mesurées et le volume de l'infarctus et des deux hémisphères ont été calculés par la formule suivante (Volume de l'infarctus corrigé = Volume de l'infarctus - (volume de l'hémisphère droit - volume de l'hémisphère gauche)) pour compenser l'oedème cérébral.

Dans les cas d'un traitement curatif (traitement de la phase aiguë), les animaux traités avec les composés selon l'invention ont des dommages au niveau cérébral réduit par rapport aux animaux non traités. En effet, le volume de l'infarctus est diminué lorsque les composés selon l'invention sont administrés une ou plusieurs fois après l'ischémie-reperfusion.

### Exemple 8: Evaluation des effets protecteurs des composés selon l'invention dans un modèle animal d'athérosclérose

Les composés selon l'invention, de part leurs propriétés PPAR activatrices et antioxydantes, ont une action bénéfique sur la progression de la plaque athéromateuse.

### 1/Traitement des animaux

Les souris transgéniques Apo E2/E2 femelles âgées d'environ 2 mois sont maintenues sous un cycle lumière/obscurité de 12/12 heures à une température constante de 20 ± 3°C pendant la période d'acclimatation et toute la durée de l'expérimentation.

Après la période d'acclimatation d'une semaine, les souris sont pesées et rassemblées par groupes de 8 animaux sélectionnés de sorte que la distribution de leur poids corporel soit uniforme.

Les animaux ont un accès libre à l'eau et à la nourriture, ils sont soumis à un régime alimentaire de type western contenant 21 % de graisse et 0,15% de cholestérol pendant une période de deux semaines avant traitement.

A l'issue de cette période, les composés à tester sont introduits dans la nourriture aux doses indiquées. Le traitement dure 6 semaines.

Les animaux sont pesés et sacrifiés sous anesthésie, par dislocation cervicale.
■ Le coeur est perfusé *in situ* puis préparé en vue de l'analyse histologique, une aiguille est introduite dans le ventricule droit et l'aorte est sectionnée dans sa partie abdominale
■ Des échantillons de sang sont prélevés avant le début de l'expérience, chaque semaine puis à l'issue de l'expérience. Le sang est collecté sur EDTA. Le plasma est préparé par centrifugation à 3000 tours/minutes pendant 20 minutes (mesures des taux plasmatiques de cholestérol et de triglycérides).

### 2/Préparation des coupes en vue de l'analyse histologique

Une solution Krebs Ringer est introduite pendant 10 minutes. Les tissus sont fixés avec du PAF 4% dans une solution 10mM de PBS à -4°C pendant une nuit. Les échantillons sont ensuite lavés avec une solution 100mM de PBS. Les coeurs sont placés dans une solution 30% de sucrose-Tris pendant une journée puis immergés dans de l'OCT (tissue Teck) sous vide pendant 30 minutes puis dans un moule contenant de l'OCT, immergés dans de l'isopentane et refroidi avec de l'azote liquide. Les échantillons sont conservés à -80°C.

Des cryosections de 10µm d'épaisseur sont réalisées à partir de l'arc aortique jusqu'à disparition des valves. Elles sont collectées sur des lames recouvertes de gélatine.

### 3/Analyse histologique

Les lames sont colorées avec une solution d'huile rouge et de l'hématoxyline de façon à différencier la partie médiane de l'intima. Les différents paramètres morphogéniques sont déterminés à l'aide d'un microscope Olympus et d'une caméra couleur couplée au système d'analyse d'images Analysis. La quantification des surfaces lésées est réalisée de façon manuelle à l'aide d'une tablette graphique couplée au même système informatique.

La surface globale des lésions athéromateuses est exprimée en µM², elle est comparée au groupe témoin. Les composés selon l'invention testés permettent une diminution significative de la surface des lésions traduisant une réduction de la progression des lésions.

### Exemple 9: effets in vitro des composés selon l'invention sur la différenciation et la maturation des cellules dendritiques

Le composé 39 est testé sur la différenciation des cellules dendritiques issues de monocytes (par suivi de l'acquisition du phénotype des cellules dendritiques). Pour ces expériences, les échantillons de sang proviennent de donneurs volontaires (Etablissement Français du Sang) et les monocytes sont isolés à l'aide d'un protocole standard utilisant des billes magnétiques conjuguées aux anti-CD14 (Miltenyi Biotec). On induit ensuite la différenciation des monocytes ainsi isolés en les incubant 6 jours dans un milieu de culture contenant un mélange de cytokines GM-CSF et IL-4 (20 ng/ml pour chacune des cytokines).

Le composé 39 est ajouté à t=0. L'acquisition du phénotype des cellules dendritiques est ensuite étudiée au travers de l'expression du marqueur CD1a à la surface cellulaire. Les inventeurs ont ainsi montré que le composé 39 interfère de manière importante avec la différenciation des cellules dendritiques en supprimant presque totalement l'expression de CD1a à la surface des cellules (Figure 21). L'expression de la molécule de costimulation CD80 est aussi réduite, à un moindre niveau, tandis que l'expression de CD86 est légèrement augmentée (données non présentées). Des résultats similaires sont obtenus en testant les composés 13 et 31 de l'invention. Ces résultats suggèrent que les composés selon l'invention interfèrent avec la différenciation des cellules dendritiques et stimulent les cellules dendritiques vers l'acquisition d'un phénotype atypique.

Les effets de ces composés sont ensuite étudiés sur la maturation des cellules dendritiques induites par le LPS (LipoPolySaccharide). Pour ces expériences, les cellules dendritiques dérivées des monocytes, obtenues à j=6 de la différentiation, sont pré-traitées avec les composés selon l'invention pendant 4 heures puis stimulées avec le LPS pendant 16 heures. Il a été ainsi montré que les composés interfèrent de manière importante avec la transcription induite par le LPS du récepteur CCR7 et de son ligand ELC (Figure 22). La figure 22 montre, de plus, que la sécrétion induite par le LPS de la cytokine de l'inflammation TNFalpha est aussi réduite de manière importante.

La réduction de l'expression de ELC et de CCR7, gènes clés de la motilité des cellules dendritiques, suggèrent que les composés selon l'invention inhibent la migration des cellules dendritiques jusqu'aux organes lymphoïdes secondaires et ainsi interfèrent avec l'initiation de la réponse immunitaire déclenchée par ces cellules.

Les inventeurs ont ainsi montré que les cellules dendritiques dérivées des monocytes traitées avec le composé 31 présentent une capacité réduite à induire la prolifération des cellules T CD4+ naïves, par une Réaction Mixte Lymphocytaire (MLR) (Figure 23). Pour cette expérience, des quantités croissantes de cellules dendritiques matures (traitées ou non avec le composé) sont incubées avec une quantité fixe de cellules T CD4+ naïves dérivées d'un autre donneur. Après une incubation de 5 jours, la BrdU (BromodeoxyUridine) est ajoutée pour 24 heures et son incorporation dans les cellules T est mesurée par ELISA avec des anticorps anti-BrdU couplés à des molécules chemiluminescentes.

### Exemple 10 : effets in vivo des composés selon l'invention dans un modèle d'asthme allergique induite par l'ovalbumine (OVA) chez la souris

Les effets des composés selon l'invention sont ensuite étudiés dans le cadre d'analyses *in vivo* utilisant un modèle d'asthme allergique induite par l'ovalbumine (OVA) chez la souris.

Pour ces expériences, les souris sont sensibilisées par des injections intra-péritonéales d'ovalbumine en présence d'hydroxydes d'aluminium, à j = 0 et j = 10 de l'expérience. De J = 18 à j = 22, les souris reçoivent quotidiennement 50 mg/kg à 200 mg/kg des composés selon l'invention par gavage. 3 administrations consécutives d'ovalbumine en aérosols sont effectuées à j = 20, 21 et 22. Le composé est administré environ 1 heure avant chaque pulvérisation. Les souris sont ensuite sacrifiées à j - 24 et le liquide de lavage broncho-alvéolaire (BAL) est collecté pour la détermination de la cellularité (macrophages, éosinophiles, lymphocytes, neutrophiles) et pour la mesure des cytokines IL-5, IL-13, IL-4.

Les résultats obtenus montrent que les composés de la présente invention interfèrent avec la différenciation et la maturation des cellules dendritiques et inhibent la migration de ces cellules vers les organes lymphoïdes secondaires. De plus, les composés présentent une capacité réduite à induire la prolifération des cellules T CD4+ naïves. Les composés selon l'invention interfèrent donc avec l'initiation de la réponse immunitaire et représentent donc un outil thérapeutique avantageux pour le traitement de l'asthme.

### BIBLIOGRAPHIE

Aebi, H. (1984). "Catalase in vitro." Methods Enzymol 105: 121-6.
Angeli V, Hammad H, Staels B, Capron M, Lambrecht BN, Trottein F (2003) "Peroxisome proliferator-activated receptor gamma inhibits the migration of dendritic cells: consequences for the immune response" J Immunol. 170(10):5295-301.
Asset G, Staels B, Wolff RL, Bauge E, Madj Z, Fruchart JC, Dallongeville J. (1999). "Effects of Pinus pinaster and Pinus koraiensis seed oil supplementation on lipoprotein metabolism in the rat." Lipids 34(1): 39-44
Chang, R. C., P. Hudson, et al. (2000). "Influence of neurons on lipopolysaccharide-stimulated production of nitric oxide and tumor necrosis factor-alpha by cultured glia." Brain Res 853(2): 236-44.
Desvergne, B. and W. Wahli (1999). "Peroxisome proliferator-activated receptors: nuclear control of metabolism." Endocr Rev 20(5): 649-88.
Dirnagl, U., C. ladecola, et al. (1999). "Pathobiology of ischaemic stroke: an integrated view." Trends Neurosci 22(9): 391-7.
Farinelli, S. E., D. S. Park, et al. (1996). "Nitric oxide delays the death of trophic factor-deprived PC12 cells and sympathetic neurons by a cGMP-mediated mechanism." J Neurosci 16(7): 2325-34.
Flohe, L. and F. Otting (1984). "Superoxide dismutase assays." Methods Enzymol 105: 93-104.
Gilgun-Sherki, Y., E. Melamed, et al. (2001). "Oxidative stress induced-neurodegenerative diseases: the need for antioxidants that penetrate the blood brain barrier." Neuropharmacology 40(8): 959-75.
Gosset, P., Charbonnier A.S., Delerive P., Fontaine J., Staels B., Pestel J., Tonnel A.B., Trottein F. (2001). "Peroxisome proliferator-activated receptor gamma activators affect the maturation of human monocyte-derived dendritic cells" Eur J Immunol. 10: 2857-65.
Greene, L. A. and A. S. Tischler (1976). "Establishment of a noradrenergic clonal line of rat adrenal pheochromocytoma cells which respond to nerve growth factor." Proc Natl Acad Sci USA 73(7): 2424-8.
Guerre-Millo, M., P. Gervois, et al. (2000). "Peroxisome proliferator-activated receptor alpha activators improve insulin sensitivity and reduce adiposity." J Biol Chem 275(22): 16638-42.
Habig, W. H. and W. B. Jakoby (1981). "Assays for differentiation of glutathione S-transferases." Methods Enzymol 77: 398-405.
Hourton, D., P. Delerive, et al. (2001). "Oxidized low-density lipoprotein and peroxisome-proliferator-activated receptor alpha down-regulate platelet-activating-factor receptor expression in human macrophages." Biochem J 354(Pt 1): 225-32.
International Atherosclerosis Society "Harmonized Clinical Guidelines on Prevention of Atherosclerotic Vascular Disease" 2003.
Jurgens, G., H. F. Hoff, et al. (1987). "Modification of human serum low density lipoprotein by oxidation-- characterization and pathophysiological implications." Chem Phys Lipids 45(2-4): 315-36.
Komuves, L. G., K. Hanley, et al. (2000). "Stimulation of PPARalpha promotes epidermal keratinocyte differentiation in vivo." J Invest Dermatol 115(3): 353-60.
Lebeau, J., C. Furman, et al. (2000). "Antioxidant properties of di-tert-butylhydroxylated flavonoids." Free Radic Biol Med 29(9): 900-12.
Mates, J. M., C. Perez-Gomez, et al. (1999). "Antioxidant enzymes and human diseases." Clin Biochem 32(8): 595-603.
Morliere, P., A. Moysan, et al. (1991). "UVA-induced lipid peroxidation in cultured human fibroblasts." Biochim Biophys Acta 1084(3): 261-8.
Nencioni A, Grunebach F, Zobywlaski A, Denzlinger C, Brugger W, Brossart P., (2002) "Dendritic cell immunogenicity is regulated by peroxisome proliferator-activated receptor gamma" J Immunol. 169(3):1228-35.
Paglia, D. E. and W. N. Valentine (1967). "Studies on the quantitative and qualitative characterization of erythrocyte glutathione peroxidase." J Lab Clin Med 70(1): 158-69.
Ram VJ (2003). "Therapeutic role of peroxisome proliferator-activated receptors in obesity, diabetes and inflammation. Prog Drug Res. 60: 93-132.
Raspe, E., L. Madsen, et al. (1999). "Modulation of rat liver apolipoprotein gene expression and serum lipid levels by tetradecylthioacetic acid (TTA) via PPARalpha activation." J Lipid Res 40(11): 2099-110.
Rothwell, N. J. (1997). "Cytokines and acute neurodegeneration." Mol Psychiatry 2(2): 120-1.
Spiegelman B. M. (1998)"PPARgamma in monocytes: less pain, any gain?" Cell., 93(2):153-5
Spiegelmam B. M. (1998) "PPAR-gamma: adipogenic regulator and thiazolidinedione receptor. Diabetes. 47(4):507-14. Review.
Spooner, R. J., A. Delides, et al. (1981). "Heat stability and kinetic properties of human serum glutathione reductase activity in various disease states." Biochem Med 26(2): 239-48.
Staels, B. and J. Auwerx (1998). "Regulation of apo A-I gene expression by fibrates." Atherosclerosis 137 Suppl: S19-23.

## Revendications

1. Composés dérivés de 1,3-diphénylprop-2-èn-1-one substitués de formule générale (I) suivante : 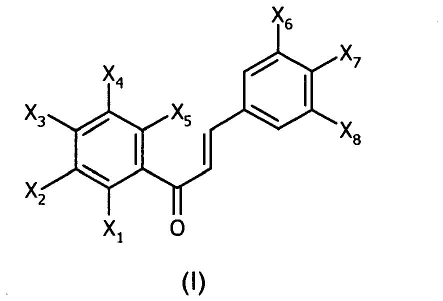 dans laquelle :
X₇ représente un groupement répondant à la formule suivante : G₇-R₇ dans laquelle G₇ est un atome d'oxygène et R₇ est une chaîne alkyle de 1 à 7 atomes de carbone substituée par un groupement carboxy de formule : -COORₐ, avec Rₐ représentant un atome d'hydrogène ou un radical alkyle de 1 à 7 atomes de carbone,
les groupements Xᵢ avec i = 1, 2, 3, 4 ou 5, identiques ou différents, représentent un atome d'halogène ou un groupement thionitroso ou répondent respectivement à la formule (Gᵢ-Rᵢ)ₙ-G'ᵢ-R'ᵢ dans laquelle :
■ n peut prendre les valeurs 0 ou 1
■ Gᵢ et G'ᵢ, identiques ou différents, représentent une simple liaison, un atome d'oxygène ou un atome de soufre,
■ Rᵢ et R'ᵢ, identiques ou différents, représentent un radical alkyle de 1 à 7 atomes de carbone, phényle, dithiolanes, pyridine, furanne, thiophène ou morpholine,
■ R'ᵢ peut également représenter un atome d'hydrogène,
les groupements Xᵢ avec i = 6 ou i = 8, identiques ou différents, représentent un atome d'halogène ou répondent à la formule G'ᵢ-R'ᵢ , G'ᵢ et R'ᵢ étant tels que définis précédemment, X₆ et X₈ ne représentant pas simultanément un atome d'hydrogène, Xi avec i = 1, 2, 3, 4, 5, 6 ou 8 ne pouvant représenter un radical dithiolane, pyridine, furanne, thiophène ou morpholine directement lié au cycle aromatique de la 1,3-diphényl prop-2-én-1-one,
à l'exclusion des composés de formule (I) pour lesquels simultanément :
■ un des groupements X₁, X₂, X₃, X₄ ou X₅ est un groupement hydroxyle,
■ G₇ est un atome d'oxygène,
■ et un des groupements X₆ ou X₈ est un atome d'hydrogène ou d'halogène ou un hydroxyle ou un groupement alkyloxy,
à l'exclusion des composés de formule (I) pour lesquels simultanément :
■ les groupements X₁, X₂ et X₄ représentent simultanément un atome d'hydrogène,
■ les groupements X₆ et X₈ représentent G'iR'i,
■ le groupement X₅ représente un groupement thionitroso ou un groupement G'ᵢR'ᵢ,
■ le groupement X₃ représente un halogène ou un groupement G'ᵢR'ᵢ,
dans lesquels G'ᵢ représente un atome d'oxygène, un atome de soufre ou une simple liaison et R'ᵢ représente un groupement alkyle saturé, linéaire, ramifié ou cyclique, halogéné ou non, ou un atome d'hydrogène.

2. Composés selon la revendication 1, **caractérisés en ce que** X₁ et X₅ sont des atomes d'hydrogène.

3. Composés selon la revendication 1 ou 2, **caractérisés en ce que** X₂ et X₄ sont des radicaux alkyle de 1 à 7 atomes de carbone.

4. Composés selon la revendication 1, **caractérisés en ce que** X₁, X₃ et X₄ sont des radicaux alkyle de 1 à 7 atomes de carbone.

5. Composés selon la revendication 1, **caractérisés en ce que** X₁, X₂, X₄ et X₅ sont des atomes d'hydrogène.

6. Composés selon l'une quelconque des revendications précédentes, **caractérisés en ce que** X₆ et X₈ sont des radicaux alkyle de 1 à 7 atomes de carbone.

7. Composés selon la revendication précédente, **caractérisés en ce que** X₁ et X₅ sont des atomes d'hydrogène.

8. Composés selon la revendication précédente, **caractérisés en ce que** X₂ et X₄ sont des radicaux alkyle de 1 à 7 atomes de carbone.

9. Composés selon la revendication 1, **caractérisés en ce que** X₁, X₃, X₄, X₆ et X₈ sont des radicaux alkyle de 1 à 7 atomes de carbone.

10. Composés selon la revendication 1, **caractérisés en ce que** X₆ et X₈ sont des radicaux alkyles de 1 à 7 atomes de carbone et X₁, X₂, X₄ et X₅ sont des atomes d'hydrogène.

11. Composés selon l'une quelconque des revendications précédentes, **caractérisés en ce que** X₃ représente un atome d'halogène ou un groupement thionitroso ou répond à la formule (Gᵢ-Rᵢ)ₙ-G'ᵢ-R'ᵢ telle que définie dans la revendication 1, dans laquelle G'ᵢ représente un atome d'oxygène ou un atome de soufre.

12. Composés selon l'une quelconque des revendications précédentes, **caractérisés en ce qu'**au moins un des groupements Gi ou G'i représente un atome de soufre avec i pouvant prendre une des valeurs 1, 2, 3, 4, 5, 6 ou 8.

13. Composés selon l'une quelconque des revendications précédentes, **caractérisés en ce qu'**ils sont choisis parmi :
La 1-(4-((R,S)-5-[1,2]dithiolan-3-ylpentyloxy)-3,5-diméthylphényl)-3-(4-carboxydiméthylméthyloxy-3,5-diméthylphényl)-prop-2-èn-1-one ;
La 1-(4-Mercapto-3,5-diméthylphényl)-3-(4-carboxydiméthylméthyloxy-3,5-diméthyl phényl)-prop-2-èn-1-one ;
La 1-(4-Cyclohexyléthylthio-3,5-diméthylphényl)-3-(4-carboxydiméthylméthyloxy-3,5-diméthylphényl)-prop-2-èn-1-one ;
La 1-(2,5-Dihydroxy-3,4,6-triméthylphényl)-3-(4-carboxydiméthylméthyloxy-3,5-diméthylphényl)-prop-2-èn-1-one ;
La 1-(2,5-Diméthoxy-3,4,6-triméthylphényl)-3-(4-carboxydiméthylméthoxy-3,5-diméthylphényl)-prop-2-èn-1-one ;
La 1-(2,5-Dihydroxyphényl)-3-(4-carboxydiméthylméthyloxy-3,5-diméthylphényl)-prop-2-èn-1-one ;
La 1-(2,5-Diméthoxyphényl)-3-(4-carboxydiméthylméthyloxy-3,5-diméthylphényl)-prop-2-èn-1-one ;
La 1-(4-Phényléthyloxyphényl)-3-(4-carboxydiméthylméthyloxy-3,5-diméthylphényl)-prop-2-èn-1-one ;
La 1-(4-(Morpholin-4-yléthyloxy)phényl)-3-(4-carboxydiméthylméthyloxy-3,5-diméthylphényl)-prop-2-èn-1-one ;
La 1-(4-(Pentylthioéthyloxy)phényl)-3-(4-tertiobutyloxycarbonyldiméthylméthyloxy-3,5-diméthylphényl)prop-2-èn-1-one ;
La 1-(4-(Pentylthioéthyloxy)phényl)-3-(4-carboxydiméthylméthyloxy-3,5-diméthylphényl)prop-2-èn-1-one ;
La 1-(4-Hydroxy-3,5-diméthylphényl)-3-(4-tertiobutyloxycarbonyldiméthylméthyloxy-3,5-diméthylphényl)-prop-2-ène-1-one ;
La 1-(4-Hydroxy-3,5-diméthylphényl)-3-(4-carboxydiméthylméthyloxy-3,5-diméthylphényl)-prop-2-ène-1-one ;
La 1-(4-((R,S)-5-[1,2]dithiolan-3-ylpentyloxy)phényl)-3-(4-tertiobutyloxycarbonyldiméthylméthyloxy-3,5-diméthylphényl)-prop-2-èn-1-one ;
La 1-(4-((R,S)-5-[1,2]dithiolan-3-ylpentyloxy)phényl)-3-(4-carboxydiméthylméthyloxy-3,5-diméthylphényl)-prop-2-èn-1-one ;
La 1-(4-Méthylthiophényl)-3-(4-tertiobutyloxycarbonyldiméthylméthyloxy-3,5-dibromophényl)-prop-2-ène-1-one ;
La 1-(4-Méthylthiophényl)-3-(4-carboxydiméthylméthyloxy-3,5-dibromophényl)-prop-2-ène-1-one ;
La 1-(4-Cyclohexyléthyloxyphényl)-3-(4-tertiobutyloxycarbonyldiméthylméthyloxy-3,5-diméthylphényl)prop-2-èn-1-one ;
La 1-(4-Cyclohexyléthyloxyphényl)-3-(4-carboxydiméthylméthyloxy-3,5-diméthylphényl) prop-2-èn-1-one ;
La 1-(4-Méthylthio-3,5-diméthylphényl)-3-(4-tertiobutyloxycarbonyldiméthylméthyloxy-3,5-diméthylphényl) prop-2-èn-1-one ;
La 1-(4-Méthylthio-3,5-diméthylphényl)-3-(4-carboxydiméthylméthyloxy-3,5-diméthylphényl) prop-2-èn-1-one ;
La 1-(4-Propyloxy-3,5-diméthylphényl)-3-(4-tertiobutyloxycarbonyldiméthylméthyloxy-3,5-diméthylphényl)prop-2-èn-1-one ;
La 1-(4-Propyloxy-3,5-diméthylphényl)-3-(4-carboxydiméthylméthyloxy-3,5-diméthylphényl)prop-2-èn-1-one ;
La 1-(4-Méthoxy-3,5-diméthylphényl)-3-(4-tertiobutyloxycarbonyldiméthylméthyloxy-3,5-diméthylphényl)prop-2-èn-1-one ;
La 1-(4-Méthoxy-3,5-diméthylphényl)-3-(4-carboxydiméthylméthyloxy-3,5-diméthylphényl)prop-2-èn-1-one ;
La 1-(4-Hexyloxy-3,5-diméthylphényl)-3-(4-tertiobutyloxycarbonyldiméthylméthyloxy-3,5-diméthylphényl)prop-2-èn-1-one ;
La 1-(4-Hexyloxy-3,5-diméthylphényl)-3-(4-carboxydiméthylméthyloxy-3,5-diméthyl phényl)prop-2-èn-1-one ;
La 1-(4-Cyclohexyléthyloxy-3, 5-diméthylphényl)-3-(4-tertiobutyloxycarbonyldiméthylméthyloxy-3,5-diméthylphényl) prop-2-èn-1-one ;
La 1-(4-Cyclohexyléthyloxy-3, 5-diméthylphényl)-3-(4-carboxydiméthylméthyloxy-3,5-diméthylphényl) prop-2-èn-1-one ;
La 1-(4-Cyclohexylthioéthyloxyphényl)-3-(4-tertiobutyloxycarbonyldiméthylméthyloxy-3,5-diméthylphényl) prop-2-èn-1-one ;
La 1-(4-Cyclohexylthioéthyloxyphényl)-3-(4-carboxydiméthylméthyloxy-3,5-diméthylphényl) prop-2-èn-1-one ;
La 1-(2,4,5-Triméthylphényl)-3-(4-tertiobutyloxycarbonyldiméthylméthyloxy-3,5-diméthylphényl) prop-2-èn-1-one ;
La 1-(2,4,5-Triméthylphényl)-3-(4-carboxydiméthylméthyloxy-3,5-diméthylphényl) prop-2-èn-1-one ;
La 1-(4-Cyclohexylthioéthyloxy-3,5-diméthylphényl)-3-(4-tertiobutyloxycarbonyldiméthylméthyloxy-3,5-diméthylphényl) prop-2-èn-1-one ;
La 1-(4-Cyclohexylthioéthyloxy-3,5-diméthylphényl)-3-(4-carboxydiméthylméthyloxy-3,5-diméthylphényl)prop-2-èn-1-one ;
La 1-(4-Méthylthiophényl)-3-(4-tertiobutyloxycarbonyldiméthylméthyloxy-3-tluorophényl)prop-2-èn-1-one ;
La 1-(4-Méthylthiophényl)-3-(4-carboxydiméthylméthyloxy-3-fluorophényl)prop-2-èn-1-one ;
La 1-(2,3,4,5,6-Pentaméthylphényl)-3-(4-tertiobutyloxycarbonyldiméthylméthyloxy-3,5-diméthylphényl)prop-2-èn-1-one ;
La 1-(2,3,4,5,6-Pentaméthylphényl)-3-(4-carboxydiméthylméthyloxy-3,5-diméthylphényl)prop-2-èn-1-one ;
La 1-(4-Phényloxyphényl)-3-(4-tertiobutyloxycarbonyldiméthylméthyloxy-3,5-diméthylphényl)prop-2-èn-1-one ;
La 1-(4-Phényloxyphényl)-3-(4-carboxydiméthylméthyloxy-3,5-diméthylphényl)prop-2-èn-1-one ;
La 1-(4-Méthoxy-3-fluorophényl)-3-(4-tertiobutyloxycarbonyldiméthylméthyloxy-3,5-diméthylphényl)prop-2-èn-1-one ;
La 1-(4-Méthoxy-3-fluorophényl)-3-(4-carboxydiméthylméthyloxy-3,5-diméthylphényl) prop-2-èn-1-one ;
La 1-(4-Méthoxy-3-méthylphényl)-3-(4-tertiobutyloxycarbonyldiméthylméthyloxy-3,5-diméthylphényl)prop-2-èn-1-one ;
La 1-(4-Méthoxy-3-méthylphényl)-3-(4-carboxydiméthylméthyloxy-3,5-diméthylphényl) prop-2-èn-1-one ;
La 1-(4-Hexylthio-3,5-diméthylphényl)-3-(4-tertiobutyloxycarbonyldiméthylméthyloxy-3,5-diméthylphényl) prop-2-èn-1-one ;
La 1-(4-Hexylthio-3,5-diméthylphényl)-3-(4-carboxydiméthylméthyloxy-3,5-diméthylphényl) prop-2-èn-1-one ;
La 1-(2,5-Diméthoxyphényl)-3-(4-tertiobutyloxycarbonyldiméthylméthytoxy-3,5-diméthylphényl)prop-2-èn-1-one ;
La 1-(2,5-Diméthoxyphényl)-3-(4-carboxydiméthylméthyloxy-3,5-diméthylphényl)prop-2-èn-1-one ;
Le chlorhydrate de 1-(3,5-Diméthyl-4-(morpholin-4-yléthyloxy)phényl)-3-(4-ethyloxycarbonyl diméthylméthyloxy-3,5-diméthylphényl)-prop-2-èn-1-one ;
La 1-(3,5-Diméthyl-4-(morpholin-4-yléthyloxy)phényl)-3-(4-carboxydiméthylméthyloxy-3,5-diméthylphényl)-prop-2-èn-1-one ;
La 1-(4-Bromophényl)-3-(4-tertiobutyloxycarbonyldiméthylméthyloxy-3,5-difluorophényl)-prop-2-èn-1-one ;
La 1-(4-Bromophényl)-3-(4-carboxydiméthylméthyloxy-3,5-difluorophényl)-prop-2-èn-1-one ;
La 1-(4-Méthoxy-3-trifluorométhylphényl)-3-(4-tertiobutyloxycarbonyldiméthylméthyloxy-3,5-diméthylphényl)-prop-2-èn-1-one ;
La 1-(4-Méthoxy-3-trifluorométhylphényl)-3-(4-carboxydiméthylméthyloxy-3,5-diméthylphényl)-prop-2-èn-1-one.

14. Procédé de préparation de composés de formule (I) telle que définie dans l'une des revendications précédentes, **caractérisé en ce qu'**il comprend une mise en contact en milieu basique ou en milieu acide d'au moins un composé de formule (A) avec au moins un composé de formule (B), les formules (A) et (B) étant : 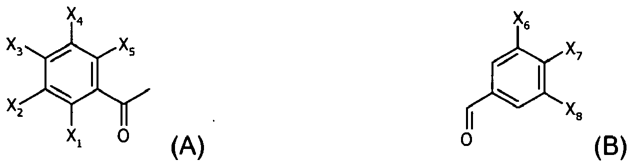 formules dans lesquelles X₁, X₂, X₃, X₄, X₅, X₆, X₇ et X₈ ont les définitions données dans l'une des revendications précédentes, X₇ pouvant également représenter un groupement hydroxyle ou thiol.

15. Composés intermédiaires, **caractérisés en ce qu'**ils sont choisis parmi :
La 1-(4-(Pentylthioéthyloxy)phényl)-3-(4-hydroxy-3,5-diméthylphényl)prop-2-èn-1-one ;
La 1-(4-((R,S)-5-[1,2]dithiolan-3-ylpentyloxy)phényl)-3-(4-hydroxy-3,5-diméthylphényl) -prop-2-èn-1-one ;
La 1-(4-Méthylthiophényl)-3-(4-hydroxy-3,5-dibromophényl)prop-2-èn-1-one ;
La 1-(4-(Cydohexyléthyloxy)phényl)-3-(4-hydroxy-3,5-diméthylphényl)prop-2-èn-1-one ;
La 1-(4-Méthylthio-3,5-diméthylphénylphényl)-3-(4-hydroxy-3,5-diméthylphényl)prop-2-èn-1-one ;
La 1-(4-Méthoxy-3,5-diméthylphényl)-3-(4-hydroxy-3,5-diméthylphényl)prop-2-èn-1-one ;
La 1-(4-(Cyclohexyléthyloxy)-3,5-diméthylphényl)-3-(4-hydroxy-3,5-diméthylphényl)prop-2-èn-1-one ;
La 1-(4-(Cyclohexylthioéthyloxy)phényl)-3-(4-hydroxy-3,5-diméthylphényl)prop-2-èn-1-one ;
La 1-(2,4,5-Triméthylphényl)-3-(4-hydroxy-3,5-diméthylphényl)prop-2-èn-1-one ;
La 1-(4-(Cyclohexylthioéthyloxy)-3,5-diméthylphényl)-3-(4-hydroxy-3,5-diméthylphényl)prop-2-èn-1-one ;
La 1-(4-Méthylthiophényl)-3-(4-hydroxy-3-fluorophényl)prop-2-èn-1-one ;
La 1-(2,3,4,5,6-Pentaméthylphényl)-3-(4-hydroxy-3,5-diméthylphényl)prop-2-èn-1-one ;
La 1-(4-Phénoxyphényl)-3-(4-hydroxy-3,5-diméthylphényl)prop-2-èn-1-one ;
La 1-(4-Méthoxy-3-fluorophényl)-3-(4-hydroxy-3,5-diméthylphényl)prop-2-èn-1-one ;
La 1-(4-Méthoxy-3-méthylphényl)-3-(4-hydroxy-3,5-diméthylphényl)prop-2-èn-1-one ;
La 1-(4-Hexylthio-3,5-diméthylphényl)-3-(4-hydroxy-3,5-diméthylphényl)prop-2-èn-1-one ;
La 1-(2,5-Diméthoxyphényl)-3-(4-hydroxy-3,5-diméthylphényl)prop-2-èn-1-one ;
La 1-(4-Bromophényl)-3-(4-hydroxy-3,5-difluorophényl)-prop-2-èn-1-one ;
La 1-(4-Méthoxy-3-trifluorométhylphényl)-3-(4-hydroxy-3,5-diméthylphényl)-prop-2-èn-1-one.

16. Composés selon l'une quelconque des revendications précédentes 1 à 13, à titre de médicaments.

17. Composition pharmaceutique ou cosmétique comprenant, dans un support acceptable sur le plan pharmaceutique ou cosmétique, au moins un composé de formule générale (I) tel que défini dans l'une des revendications 1 à 13, éventuellement en association avec un autre actif thérapeutique et/ou cosmétique.

18. Composition pharmaceutique ou cosmétique, selon la revendication 17, destinée au traitement des maladies cardiovasculaires, des dyslipidémies, des pathologies associées au syndrome X, du diabète, de l'obésité, de l'hypertension, des maladies inflammatoires, des maladies dermatologiques (psoriasis, dermatites atopiques, acné), de l'asthme, des désordres liés au stress oxydatif ou au traitement du vieillissement en général, en particulier du vieillissement cutané.

## Claims

1. Compounds derived from substituted 1,3-diphenylprop-2-en-1-one represented by general formula (I) below : 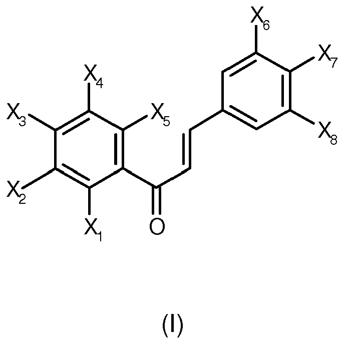 in which :
X₇ represents a group corresponding to the following formula: G₇-R₇ in which G₇ is an oxygen and R₇ is an alkyl chain from 1 to 7 carbon atoms substituted by a carboxy group of formula: -COORₐ, with Rₐ representing a hydrogen atom or an alkyl group from 1 to 7 carbon atoms,
the Xᵢ groups with i = 1, 2, 3, 4 or 5, which are the same or different, represent a halogen atom or a thionitroso group or respectively correspond to the formula (Gᵢ-Rᵢ)ₙ-G'ᵢ;-R'ᵢ in which:
■ n can have the values 0 or 1,
■ Gᵢ and G'ᵢ, which are the same or different, represent a single bond, an oxygen atom or a sulfur atom,
■ Rᵢ and R'ᵢ, which are the same or different, represent an alkyl group from 1 to 7 carbon atoms, phenyl, dithiolane, pyridine, furan, thiophene or morpholine group,
■ R'ᵢ can also represent a hydrogen atom,
the Xᵢ groups with i = 6 or i = 8, which are the same or different, represent a halogen atom or correspond to the formula G'ᵢ-R'ᵢ , G'ᵢ and R'ᵢ being such as defined hereinabove, X₆ and X₈ not simultaneously representing a hydrogen atom,
Xi with i = 1, 2, 3, 4, 5, 6 or 8 not representing a dithiolane, pyridine, furan, thiophene or morpholine group bound directly to the aromatic ring of the 1,3-diphenyl prop-2-en-1-one,
with the exception of compounds represented by formula (I) in which simultaneously:
■ one of the groups X₁, X₂, X₃, X₄ or X₅ is a hydroxyl group,
■ G₇ is an oxygen atom,
■ and one of the groups X₆ or X₈ is a hydrogen atom or a halogen or a hydroxyl or alkyloxy group,
with the exception of compounds represented by formula (I) in which simultaneously :
■ the X₁, X₂ and X₄ groups simultaneously represent a hydrogen atom,
■ the X₆ and X₈ groups represent G'ᵢR'ᵢ,
■ the X₅ group represents a thionitroso group or a G'ᵢR'ₗ group,
■ the X₃ group represents a halogen or a G'ᵢR'ᵢ group,
in which G'ᵢ represents an oxygen atom, a sulfur atom or a single bond and R'ᵢ represents a saturated, linear, branched or cyclic alkyl group, halogenated or not, or a hydrogen atom.

2. Compounds according to claim 1, **characterized in that** X₁ and X₅ are hydrogen atoms.

3. Compounds according to either one of claims 1 or 2, **characterized in that** X₂ and X₄ are alkyl groups from 1 to 7 carbon atoms.

4. Compounds according to claim 1, **characterized in that** X₁, X₃ and X₄ are alkyl groups from 1 to 7 carbon atoms.

5. Compounds according to claim 1, **characterized in that** X₁, X₂, X₄ and X₅ are hydrogen atoms.

6. Compounds according to any one of the previous claims, **characterized in that** X₆ and X₈ are alkyl groups from 1 to 7 carbon atoms.

7. Compounds according to the previous claim, **characterized in that** X₁ and X₅ are hydrogen atoms.

8. Compounds according to the previous claim, **characterized in that** X₂ and X₄ are alkyl groups from 1 to 7 carbon atoms.

9. Compounds according to claim 1, **characterized in that** X₁, X₃, X₄, X₆ and X₈ are alkyl groups from 1 to 7 carbon atoms.

10. Compounds according to claim 1, **characterized in that** X₆ and X₈ are alkyl groups from 1 to 7 carbon atoms and X₁, X₂, X₄ and X₅ are hydrogen atoms.

11. Compounds according to any one of the previous claims, **characterized in that** X₃ represents a halogen atom or a thionitroso group or corresponds to the formula (Gᵢ-Rᵢ)ₙ-G'ᵢ-R'ᵢ such as defined in claim 1, in which G'ᵢ represents an oxygen atom or a sulfur atom.

12. Compounds according to any one of the previous claims, **characterized in that** at least one of the groups Gi or G'i represents a sulfur atom with i taking the values 1, 2, 3,4,5,6or8.

13. Compounds according to any one of the previous claims, **characterized in that** they are selected in the group consisting of :
1-(4-((R,S)-5-[1,2]dithiolan-3-ylpentyloxy)-3,5-dimethylphenyl)-3-(4-carboxydimethylmethyloxy-3,5-dimethylphenyl)prop-2-en-1-one;
1-(4-Mercapto-3,5-dimethylphenyl)-3-(4-carboxydimethylmethyloxy-3,5-dimethyl phenyl)prop-2-en-1-one;
1-(4-Cyclohexylethylthio-3,5-dimethylphenyl)-3-(4-carboxydimethylmethyloxy-3,5-dimethylphenyl)prop-2-en-1-one;
1-(2,5-Dihydroxy-3,4,6-trimethylphenyl)-3-(4-carboxydimethylmethyloxy-3,5-dimethylphenyl)prop-2-en-1-one;
1-(2,5-Dimethoxy-3,4,6-trimethylphenyl)-3-(4-carboxydimethylmethoxy-3,5-dimethylphenyl)prop-2-en-1-one;
1-(2,5-Dihydroxyphenyl)-3-(4-carboxydimethylmethyloxy-3,5-dimethylphenyl)prop-2-en-1-one;
1-(2,5-Dimethoxyphenyl)-3-(4-carboxydimethylmethyloxy-3,5-dimethylphenyl)prop-2-en-1-one;
1-(4-Phenylethyloxyphenyl)-3-(4-carboxydimethylmethyloxy-3,5-dimethylphenyl)prop-2-en-1-one;
1-(4-(Morpholin-4-ylethyloxy)phenyl)-3-(4-carboxydimethylmethyloxy-3,5-dimethylphenyl)prop-2-en-1-one;
1-(4-(Pentylthioethyloxy)phenyl)-3-(4-tert-butyloxycarbonyldimethylmethyloxy-3,5-dimethylphenyl)prop-2-en-1-one;
1-(4-(Pentylthioethyloxy)phenyl)-3-(4-carboxydimethylmethyloxy-3,5-dimethylphenyl)prop-2-en-1-one;
1-(4-Hydroxy-3,5-dimethylphenyl)-3-(4-tert-butyloxycarbonyldimethylmethyloxy-3,5-dimethylphenyl)prop-2-ene-1-one;
1-(4-Hydroxy-3,5-dimethylphenyl)-3-(4-carboxydimethylmethyloxy-3,5-dimethylphenyl)prop-2-ene-1-one;
1-(4-((R,S)-5-[1,2]dithiolan-3-ylpentyloxy)phenyl)-3-(4-tert-butyloxycarbonyl dimethyl methyloxy-3,5-dimethylphenyl)prop-2-en-1-one;
1-(4-((R,S)-5-[1,2]dithiolan-3-ylpentyloxy)phenyl)-3-(4-carboxydimethylmethyloxy-3,5-dimethylphenyl)prop-2-en-1-one;
1-(4-Methylthiophenyl)-3-(4-tert-butyloxycarbonyldimethylmethyloxy-3,5-dibromophenyl)prop-2-ene-1-one;
1-(4-Methylthiophenyl)-3-(4-carboxydimethylmethyloxy-3,5-dibromophenyl)prop-2-ene-1-one;
1-(4-Cyclohexylethyloxyphenyl)-3-(4-tert-butyloxycarbonyldimethylmethyloxy-3,5-dimethylphenyl)prop-2-en-1-one;
1-(4-Cyclohexylethyloxyphenyl)-3-(4-carboxydimethylmethyloxy-3,5-dimethylphenyl)prop-2-en-1-one;
1-(4-Methylthio-3,5-dimethylphenyl)-3-(4-tert-butyloxycarbonyldimethylmethyloxy-3,5-dimethylphenyl)prop-2-en-1-one;
1-(4-Methylthio-3,5-dimethylphenyl)-3-(4-carboxydimethylmethyloxy-3,5-dimethylphenyl)prop-2-en-1-one;
1-(4-Propyloxy-3,5-dimethylphenyl)-3-(4-tert-butyloxycarbonyldimethylmethyloxy-3,5-dimethylphenyl)prop-2-en-1-one;
1-(4-Propyloxy-3,5-dimethylphenyl)-3-(4-carboxydimethylmethyloxy-3,5-dimethylphenyl)prop-2-en-1-one;
1-(4-Methoxy-3,5-dimethylphenyl)-3-(4-tert-butyloxycarbonyldimethylmethyloxy-3,5-dimethylphenyl)prop-2-en-1-one;
1-(4-Methoxy-3,5-dimethylphenyl)-3-(4-carboxydimethylmethyloxy-3,5-dimethylphenyl)prop-2-en-1-one;
1-(4-Hexyloxy-3,5-dimethylphenyl)-3-(4-tert-butyloxycarbonyldimethyl methyloxy -3,5-dimethylphenyl)prop-2-en-1-one;
1-(4-Hexyloxy-3,5-dimethyl phenyl)-3-(4-carboxydimethyl methyloxy-3, 5-di methyl phenyl)prop-2-en-1-one;
1-(4-Cyclohexylethyloxy-3,5-dimethylphenyl)-3-(4-tert-butyloxycarbonyldimethyl methyloxy-3,5-dimethylphenyl)prop-2-en-1-one;
1-(4-Cyclohexylethyloxy-3,5-dimethylphenyl)-3-(4-carboxydimethylmethyloxy-3,5-dimethylphenyl)prop-2-en-1-one;
1-(4-Cyclohexylthioethyloxyphenyl)-3-(4-tert-butyloxycarbonyldimethylmethyloxy-3,5-dimethylphenyl)prop-2-en-1-one;
1-(4-Cyclohexylthioethyloxyphenyl)-3-(4-carboxydimethylmethyloxy-3,5-dimethylphenyl)prop-2-en-1-one;
1-(2,4,5-Trimethylphenyl)-3-(4-tert-butyloxycarbonyldimethylmethyloxy-3,5-dimethylphenyl)prop-2-en-1-one;
1-(2,4,5-Trimethylphenyl)-3-(4-carboxydimethylmethyloxy-3,5-dimethylphenyl)prop-2-en-1-one;
1-(4-Cyclohexylthioethyloxy-3,5-dimethylphenyl)-3-(4-tert-butyloxycarbonyldimethylmethyloxy-3,5-dimethylphenyl)prop-2-en-1-one;
1-(4-Cyclohexylthioethyloxy-3,5-dimethylphenyl)-3-(4-carboxydimethylmethyloxy-3,5-dimethylphenyl)prop-2-en-1-one;
1-(4-Methylthiophenyl)-3-(4-tert-butyloxycarbonyldimethylmethyloxy-3-fluorophenyl)prop-2-en-1-one;
1-(4-Methylthiophenyl)-3-(4-carboxydimethylmethyloxy-3-fluorophenyl)prop-2-en-1-one;
1-(2,3,4,5,6-Pentamethylphenyl)-3-(4-tert-butyloxycarbonyldimethylmethyloxy-3,5-dimethylphenyl)prop-2-en-1-one;
1-(2,3,4,5,6-Pentamethylphenyl)-3-(4-carboxydimethylmethyloxy-3,5-dimethylphenyl)prop-2-en-1-one;
1-(4-Phenyloxyphenyl)-3-(4-tert-butyloxycarbonyldimethylmethyloxy-3,5-dimethylphenyl)prop-2-en-1-one;
1-(4-Phenyloxyphenyl)-3-(4-carboxydimethylmethyloxy-3,5-dimethylphenyl)prop-2-en-1-one;
1-(4-Methoxy-3-fluorophenyl)-3-(4-tert-butyloxycarbonyldimethylmethyloxy-3,5-dimethylphenyl)prop-2-en-1-one;
1-(4-Methoxy-3-fluorophenyl)-3-(4-carboxydimethylmethyloxy-3,5-dimethylphenyl)prop-2-en-1-one;
1-(4-Methoxy-3-methylphenyl)-3-(4-tert-butyloxycarbonyldimethylmethyloxy-3,5-dimethylphenyl)prop-2-en-1-one;
1-(4-Methoxy-3-methylphenyl)-3-(4-carboxydimethylmethyloxy-3,5-dimethylphenyl)prop-2-en-1-one;
1-(4-Hexylthio-3,5-dimethylphenyl)-3-(4-tert-butyloxycarbonyldimethylmethyloxy-3,5-dimethylphenyl)prop-2-en-1-one;
1-(4-Hexylthio-3,5-dimethylphenyl)-3-(4-carboxydimethylmethyloxy-3,5-dimethylphenyl)prop-2-en-1-one;
1-(2,5-Dimethoxyphenyl)-3-(4-tert-butyloxycarbonyldimethylmethyloxy-3,5-dimethylphenyl)prop-2-en-1-one;
1-(2,5-Dimethoxyphenyl)-3-(4-carboxydimethylmethyloxy-3,5-dimethylphenyl)prop-2-en-1-one;
1-(3,5-Dimethyl-4-(morpholin-4-ylethyloxy)phenyl)-3-(4-ethyloxycarbonyl dimethylmethyloxy-3,5-dimethylphenyl)prop-2-en-1-one hydrochloride;
1-(3,5-Dimethyl-4-(morpholin-4-ylethyloxy)phenyl)-3-(4-carboxydimethylmethyloxy-3,5-dimethylphenyl)prop-2-en-1-one;
1-(4-Bromophenyl)-3-(4-tert-butyloxycarbonyldimethylmethyloxy-3,5-difluorophenyl)prop-2-en-1-one;
1-(4-Bromophenyl)-3-(4-carboxydimethylmethyloxy-3,5-difluorophenyl)prop-2-en-1-one;
1-(4-Methoxy-3-trifluoromethylphenyl)-3-(4-tert-butyloxycarbonyldimethylmethyloxy-3,5-dimethylphenyl)prop-2-en-1-one;
1-(4-Methoxy-3-trifluoromethylphenyl)-3-(4-carboxydimethylmethyloxy-3,5-dimethylphenyl)prop-2-en-1-one.

14. Method for preparing compounds represented by formula (I) such as defined in any one of the previous claims, **characterized in that** it comprises contacting in basic medium or in acidic medium at least one compound corresponding to formula (A) with at least one compound corresponding to formula (B), formulas (A) and (B) being 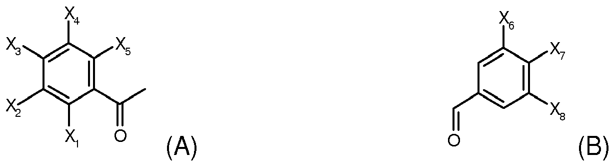 formulas in which X₁, X₂, X₃, X₄, X₅, X₆, X₇ and X₈ are defined as in any one of the previous claims, X₇ can also represent a hydroxyl or thiol group.

15. Intermediate compounds, **characterized in that** they are selected in the group consisting of:
1-(4-(Pentylthioethyloxy)phenyl)-3-(4-hydroxy-3,5-dimethylphenyl)prop-2-en-1-one;
1-(4-((R,S)-5-[1,2]dithiolan-3-ylpentyloxy)phenyl)-3-(4-hydroxy-3,5-dimethylphenyl) prop-2-en-1-one;
1-(4-Methylthiophenyl)-3-(4-hydroxy-3,5-dibromophenyl)prop-2-en-1-one;
1-(4-(Cyclohexylethyloxy)phenyl)-3-(4-hydroxy-3,5-dimethylphenyl)prop-2-en-1-one;
1-(4-Methylthio-3,5-dimethylphenyl)-3-(4-hydroxy-3,5-dimethylphenyl)prop-2-en-1-one;
1-(4-Methoxy-3,5-dimethylphenyl)-3-(4-hydroxy-3,5-dimethylphenyl)prop-2-en-1-one;
1-(4-(Cyclohexylethyloxy)-3,5-dimethylphenyl)-3-(4-hydroxy-3,5-dimethylphenyl)prop-2-en-1-one;
1-(4-(Cyclohexylthioethyloxy)phenyl)-3-(4-hydroxy-3,5-dimethylphenyl)prop-2-en-1-one;
1-(2,4,5-Trimethylphenyl)-3-(4-hydroxy-3,5-dimethylphenyl)prop-2-en-1-one;
1-(4-(Cyclohexylthioethyloxy)-3,5-dimethylphenyl)-3-(4-hydroxy-3,5-dimethylphenyl)prop-2-en-1-one;
1-(4-Methylthiophenyl)-3-(4-hydroxy-3-fluorophenyl)prop-2-en-1-one;
1-(2,3,4,5,6-Pentamethylphenyl)-3-(4-hydroxy-3,5-dimethylphenyl)prop-2-en-1-one;
1-(4-Phenoxyphenyl)-3-(4-hydroxy-3,5-dimethylphenyl)prop-2-en-1-one;
1-(4-Methoxy-3-fluorophenyl)-3-(4-hydroxy-3,5-dimethylphenyl)prop-2-en-1-one;
1-(4-Methoxy-3-methylphenyl)-3-(4-hydroxy-3,5-dimethylphenyl)prop-2-en-1-one;
1-(4-Hexylthio-3,5-dimethylphenyl)-3-(4-hydroxy-3,5-dimethylphenyl)prop-2-en-1-one;
1-(2,5-Dimethoxyphenyl)-3-(4-hydroxy-3,5-dimethylphenyl)prop-2-en-1-one;
1-(4-Bromophenyl)-3-(4-hydroxy-3,5-difluorophenyl)prop-2-en-1-one;
1-(4-Methoxy-3-trifluoromethylphenyl)-3-(4-hydroxy-3,5-dimethylphenyl)prop-2-en-1-one.

16. Compounds according to any one of claims 1 to 13, as medicaments.

17. Pharmaceutical or cosmetic composition comprising, in a pharmaceutically acceptable support, at least one compound represented by general formula (I) such as defined in any one of claims 1 to 13, optionally in association with another therapeutic and/or cosmetic active agent.

18. Pharmaceutical or cosmetic composition according to claim 17, intended for the treatment of cardiovascular diseases, dyslipidemias, pathologies associated with syndrome X, diabetes, obesity, hypertension, inflammatory diseases, dermatological diseases (psoriasis, atopic dermatitis, acne, etc.), asthma, disorders related to oxidative stress or for the treatment of ageing in general, in particular skin ageing.

## Patentansprüche

1. Substituierte 1,3-Diphenylprop-2-en-1-on-Verbindungsderivate der folgenden allgemeinen Formel (I): 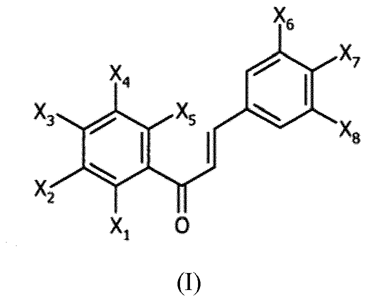 in der:
X₇ für eine Gruppe steht, die der folgenden Formel entspricht: G₇-R₇, in der G₇ ein Sauerstoffatom ist und R₇ eine Alkylkette mit 1 bis 7 Kohlenstoffatomen ist, die mit einer Carboxygruppe der Formel -COORₐ substituiert ist, wobei Rₐ für ein Wasserstoffatom oder einen Alkylrest mit 1 bis 7 Kohlenstoffatomen steht,
die Gruppen Xᵢ mit i = 1, 2, 3, 4 oder 5, die identisch oder verschieden sind, für ein Halogenatom oder eine Thionitrosogruppe stehen oder beziehungsweise der Formel (Gᵢ-Rᵢ)ₙ-G'ᵢ-R'ᵢ entsprechen, in der
■ n die Werte 0 oder 1 einnehmen kann,
■ Gᵢ und G'ᵢ, die identisch oder verschieden sind, für eine Einfachbindung, ein Sauerstoffatom oder ein Schwefelatom stehen,
■ Rᵢ und R'ᵢ, die identisch oder verschieden sind, für einen Alkylrest mit 1 bis 7 Kohlenstoffatomen, einen Phenyl-, Dithiolan-, Pyridin-, Furan-, Thiophen- oder Morpholinrest stehen,
■ R'ᵢ ebenfalls für ein Wasserstoffatom stehen kann,
die Gruppen Xᵢ mit i = 6 oder i = 8, die identisch oder verschieden sind, für ein Halogenatom stehen oder der Formel G'ᵢ-R'ᵢ entsprechen, wobei G'ᵢ und R'ᵢ wie zuvor definiert sind, X₆ und X₈ nicht gleichzeitig für ein Wasserstoffatom stehen, Xi mit i = 1, 2, 3, 4, 5, 6 oder 8 nicht für einen direkt an den aromatischen Ring des 1,3-Diphenylprop-2-en-1-ons gebundenen Dithiolan-, Pyridin-, Furan-, Thiophen- oder Morpholinrest stehen kann,
unter Ausschluss der Verbindungen der Formel (I), bei denen gleichzeitig:
■ eine der Gruppen X₁, X₂, X₃, X₄ oder X₅ eine Hydroxylgruppe ist,
■ G₇ ein Sauerstoffatom ist,
■ und eine der Gruppen X₆ oder X₈ ein Wasserstoff- oder Halogenatom oder eine Hydroxyl- oder Alkyloxygruppe ist,
unter Ausschluss der Verbindungen der Formel (I), bei denen gleichzeitig:
■ die Gruppen X₁, X₂ und X₄ gleichzeitig für ein Wasserstoffatom stehen,
■ die Gruppen X₆ und X₈ für G'ᵢR'ᵢ stehen,
■ die Gruppe X₅ für eine Thionitrosogruppe oder eine G'ᵢR'ᵢ-Gruppe steht,
■ die Gruppe X₃ für ein Halogen oder eine G'ᵢR'ᵢ-Gruppe steht,
in denen G'ᵢ für ein Sauerstoffatom, ein Schwefelatom oder eine Einfachbindung steht und R'ᵢ für eine gesättigte, lineare, verzweigte oder cyclische, eventuell halogenierte, Alkylgruppe oder ein Wasserstoffatom steht.

2. Verbindungen gemäß Anspruch 1, **dadurch gekennzeichnet, dass** X₁ und X₅ Wasserstoffatome sind.

3. Verbindungen gemäß Anspruch 1 oder 2, **dadurch gekennzeichnet, dass** X₂ und X₄ Alkylreste mit 1 bis 7 Kohlenstoffatomen sind.

4. Verbindungen gemäß Anspruch 1, **dadurch gekennzeichnet, dass** X₁, X₃ und X₄ Alkylreste mit 1 bis 7 Kohlenstoffatomen sind.

5. Verbindungen gemäß Anspruch 1, **dadurch gekennzeichnet, dass** X₁, X₂, X₄ und X₅ Wasserstoffatome sind.

6. Verbindungen gemäß einem der vorhergehenden Ansprüche, **dadurch gekennzeichnet, dass** X₆ und X₈ Alkylreste mit 1 bis 7 Kohlenstoffatomen sind.

7. Verbindungen gemäß dem vorhergehenden Anspruch, **dadurch gekennzeichnet, dass** X₁ und X₅ Wasserstoffatome sind.

8. Verbindungen gemäß dem vorhergehenden Anspruch, **dadurch gekennzeichnet, dass** X₂ und X₄ Alkylreste mit 1 bis 7 Kohlenstoffatomen sind.

9. Verbindungen gemäß Anspruch 1, **dadurch gekennzeichnet, dass** X₁, X₃, X₄, X₆ und X₈ Alkylreste mit 1 bis 7 Kohlenstoffatomen sind.

10. Verbindungen gemäß Anspruch 1, **dadurch gekennzeichnet, dass** X₆ und X₈ Alkylreste mit 1 bis 7 Kohlenstoffatomen sind und X₁, X₂, X₄ und X₅ Wasserstoffatome sind.

11. Verbindungen gemäß einem der vorhergehenden Ansprüche, **dadurch gekennzeichnet, dass** X₃ für ein Halogenatom oder eine Thionitrosogruppe steht oder der wie in Anspruch 1 definierten Formel (Gᵢ-Rᵢ)ₙ-G'ᵢ-R'ᵢ entspricht, in der G'ᵢ für ein Sauerstoffatom oder ein Schwefelatom steht.

12. Verbindungen gemäß einem der vorhergehenden Ansprüche, **dadurch gekennzeichnet, dass** wenigstens eine der Gruppen Gᵢ oder G'ᵢ für ein Schwefelatom steht, wobei i einen der Werte 1, 2, 3, 4, 5, 6 oder 8 einnehmen kann.

13. Verbindungen gemäß einem der vorhergehenden Ansprüche, **dadurch gekennzeichnet, dass** sie ausgewählt sind aus:
1-(4-((R,S)-5-[1,2]Dithiolan-3-ylpentyloxy)-3,5-dimethylphenyl)-3-(4-carboxydimethylmethyloxy-3,5-dimethylphenyl)prop-2-en-1-on,
1-(4-Mercapto-3,5-dimethylphenyl)-3-(4-carboxydimethylmethyloxy-3,5-dimethylphenyl)prop-2-en-1-on;
1-(4-Cyclohexylethylthio-3,5-dimethylphenyl)-3-(4-carboxydimethylmethyloxy-3,5-dimethylphenyl)prop-2-en-1-on;
1-(2,5-Dihydroxy-3,4,6-trimethylphenyl)-3-(4-carboxydimethylmethyloxy-3,5-dimethylphenyl)prop-2-en-1-on;
1-(2,5-Dimethoxy-3,4,6-trimethylphenyl)-3-(4-carboxydimethylmethoxy-3,5-dimethylphenyl)prop-2-en-1-on;
1-(2,5-Dihydroxyphenyl)-3-(4-carboxydimethylmethyloxy-3,5-dimethylphenyl)prop-2-en-1-on;
1-(2,5-Dimethoxyphenyl)-3-(4-carboxydimethylmethyloxy-3,5-dimethylphenyl)prop-2-en-1-on;
1-(4-Phenylethyloxyphenyl)-3-(4-carboxydimethylmethyloxy-3,5-dimethylphenyl)prop-2-en-1-on;
1-(4-(Morpholin-4-ylethyloxy)phenyl)-3-(4-carboxydimethylmethyloxy-3,5-dimethylphenyl)prop-2-en-1-on;
1-(4-(Pentylthioethyloxy)phenyl)-3-(4-tertiobutyloxycarbonyldimethylmethyloxy-3,5-dimethylphenyl)prop-2-en-1-on;
1-(4-(Pentylthioethyloxy)phenyl)-3-(4-carboxydimethylmethyloxy-3,5-dimethylphenyl)prop-2-en-1-on;
1-(4-Hydroxy-3,5-dimethylphenyl)-3-(4-tertiobutyloxycarbonyldimethylmethyloxy-3,5-dimethylphenyl)prop-2-en-1-on;
1-(4-Hydroxy-3,5-dimethylphenyl)-3-(4-carboxydimethylmethyloxy-3,5-dimethylphenyl)prop-2-en-1-on;
1-(4-((R,S)-5-[1,2]Dithiolan-3-ylpentyloxy)phenyl)-3-(4-tertiobutyloxycarbonyldimethylmethyloxy-3,5-dimethylphenyl)prop-2-en-1-on;
1-(4-((R,S)-5-[1,2]Dithiolan-3-ylpentyloxy)phenyl)-3-(4-carboxydimethylmethyloxy-3,5-dimethylphenyl)prop-2-en-1-on;
1-(4-Methylthiophenyl)-3-(4-tertiobutyloxycarbonyldimethylmethyloxy-3,5-dibromphenyl)prop-2-en-1-on;
1-(4-Methylthiophenyl)-3-(4-carboxydimethylmethyloxy-3,5-dibromphenyl)prop-2-en-1-on;
1-(4-Cyclohexylethyloxyphenyl)-3-(4-tertiobutyloxycarbonyldimethylmethyloxy-3,5-dimethylphenyl)prop-2-en-1-on;
1-(4-Cyclohexylethyloxyphenyl)-3-(4-carboxydimethylmethyloxy-3,5-dimethylphenyl)prop-2-en-1-on;
1-(4-Methylthio-3,5-dimethylphenyl)-3-(4-tertiobutyloxycarbonyldimethylmethyloxy-3,5-dimethylphenyl)prop-2-en-1-on;
1-(4-Methylthio-3,5-dimethylphenyl)-3-(4-carboxydimethylmethyloxy-3,5-dimethylphenyl)prop-2-en-1-on;
1-(4-Propyloxy-3,5-dimethylphenyl)-3-(4-tertiobutyloxycarbonyldimethylmethyloxy-3,5-dimethylphenyl)prop-2-en-1-on;
1-(4-Propyloxy-3,5-dimethylphenyl)-3-(4-carboxydimethylmethyloxy-3,5-dimethylphenyl)prop-2-en-1-on;
1-(4-Methoxy-3,5-dimethylphenyl)-3-(4-tertiobutyloxycarbonyldimethylmethyloxy-3,5-dimethylphenyl)prop-2-en-1-on;
1-(4-Methoxy-3,5-dimethylphenyl)-3-(4-carboxydimethylmethyloxy-3,5-dimethylphenyl)prop-2-en-1-on;
1-(4-Hexyloxy-3,5-dimethylphenyl)-3-(4-tertiobutyloxycarbonyldimethylmethyloxy-3,5-dimethylphenyl)prop-2-en-1-on;
1-(4-Hexyloxy-3,5-dimethylphenyl)-3-(4-carboxydimethylmethyloxy-3,5-dimethylphenyl)prop-2-en-1-on;
1-(4-Cyclohexylethyloxy-3,5-dimethylphenyl)-3-(4-tertiobutyloxycarbonyldimethylmethyloxy-3,5-dimethylphenyl)prop-2-en-1-on;
1-(4-Cyclohexylethyloxy-3,5-dimethylphenyl)-3-(4-carboxydimethylmethyloxy-3,5-dimethylphenyl)prop-2-en-1-on;
1-(4-Cyclohexylthioethyloxyphenyl)-3-(4-tertiobutyloxycarbonyldimethylmethyloxy-3,5-dimethylphenyl)prop-2-en-1-on;
1-(4-Cyclohexylthioethyloxyphenyl)-3-(4-carboxydimethylmethyloxy-3,5-dimethylphenyl)prop-2-en-1-on;
1-(2,4,5-Trimethylphenyl)-3-(4-tertiobutyloxycarbonyldimethylmethyloxy-3,5-dimethylphenyl)prop-2-en-1-on;
1-(2,4,5-Trimethylphenyl)-3-(4-carboxydimethylmethyloxy-3,5-dimethylphenyl)prop-2-en-1-on;
1-(4-Cyclohexylthioethyloxy-3,5-dimethylphenyl)-3-(4-tertiobutyloxycarbonyldimethylmethyloxy-3,5-dimethylphenyl)prop-2-en-1-on;
1-(4-Cyclohexylthioethyloxy-3,5-dimethylphenyl)-3-(4-carboxydimethylmethyloxy-3,5-dimethylphenyl)prop-2-en-1-on;
1-(4-Methylthiophenyl)-3-(4-tertiobutyloxycarbonyldimethylmethyloxy-3-fluorphenyl)prop-2-en-1-on;
1-(4-Methylthiophenyl)-3-(4-carboxydimethylmethyloxy-3-fluorphenyl)prop-2-en-1-on;
1-(2,3,4,5,6-Pentamethylphenyl)-3-(4-tertiobutyloxycarbonyldimethylmethyloxy-3,5-dimethylphenyl)prop-2-en-1-on;
1-(2,3,4,5,6-Pentamethylphenyl)-3-(4-carboxydimethylmethyloxy-3,5-dimethylphenyl)prop-2-en-1-on;
1-(4-Phenyloxyphenyl)-3-(4-tertiobutyloxycarbonyldimethylmethyloxy-3,5-dimethylphenyl)prop-2-en-1-on;
1-(4-Phenyloxyphenyl)-3-(4-carboxydimethylmethyloxy-3,5-dimethylphenyl)prop-2-en-1-on;
1-(4-Methoxy-3-fluorphenyl)-3-(4-tertiobutyloxycarbonyldimethylmethyloxy-3,5-dimethylphenyl)prop-2-en-1-on;
1-(4-Methoxy-3-fluorphenyl)-3-(4-carboxydimethylmethyloxy-3,5-dimethylphenyl)prop-2-en-1-on;
1-(4-Methoxy-3-methylphenyl)-3-(4-tertiobutyloxycarbonyldimethylmethyloxy-3,5-dimethylphenyl)prop-2-en-1-on;
1-(4-Methoxy-3-methylphenyl)-3-(4-carboxydimethylmethyloxy-3,5-dimethylphenyl)prop-2-en-1-on,
1-(4-Hexylthio-3,5-dimethylphenyl)-3-(4-tertiobutyloxycarbonyldimethylmethyloxy-3,5-dimethylphenyl)prop-2-en-1-on;
1-(4-Hexylthio-3,5-dimethylphenyl)-3-(4-carboxydimethylmethyloxy-3,5-dimethylphenyl)prop-2-en-1-on;
1-(2,5-Dimethoxyphenyl)-3-(4-tertiobutyloxycarbonyldimethylmethyloxy-3,5-dimethylphenyl)prop-2-en-1-on;
1-(2,5-Dimethoxyphenyl)-3-(4-carboxydimethylmethyloxy-3,5-dimethylphenyl)prop-2-en-1-on;
1-(3,5-Dimethyl-4-(morpholin-4-ylethyloxy)phenyl)-3-(4-ethyloxycarbonyldimethylmethyloxy-3,5-dimethylphenyl)prop-2-en-1-on Hydrochlorid;
1-(3,5-Dimethyl-4-(morpholin-4-ylethyloxy)phenyl)-3-(4-carboxydimethylmethyloxy-3,5-dimethylphenyl)prop-2-en-1-on;
1-(4-Bromphenyl)-3-(4-tertiobutyloxycarbonyldimethylmethyloxy-3,5-difluorphenyl)prop-2-en-1-on,
1-(4-Bromphenyl)-3-(4-carboxydimethylmethyloxy-3,5-difluorphenyl)prop-2-en-1-on;
1-(4-Methoxy-3-trifluormethylphenyl)-3-(4-tertiobutyloxycarbonyldimethylmethyloxy-3,5-dimethylphenyl)prop-2-en-1-on;
1-(4-Methoxy-3-trifluormethylphenyl)-3-(4-carboxydimethylmethyloxy-3,5-dimethylphenyl)prop-2-en-1-on.

14. Verfahren zur Herstellung von Verbindungen der wie in einem der vorhergehenden Ansprüche definierten Formel (I), **dadurch gekennzeichnet, dass** das Verfahren ein Inkontaktbringen von wenigstens einer Verbindung der Formel (A) mit wenigstens einer Verbindung der Formel (B) in basischem Milieu oder saurem Milieu umfasst, wobei die Formeln (A) und (B): 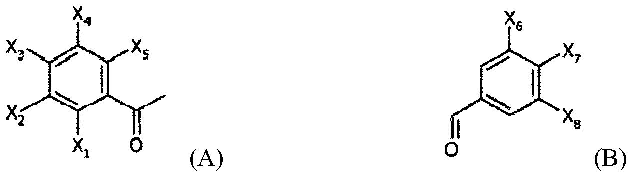 Formeln darstellen, in denen X₁, X₂, X₃, X₄, X₅, X₆, X₇ und X₈ die in einem der vorhergehenden Ansprüche genannten Definitionen besitzen, wobei X₇ gleichermaßen für eine Hydroxyl- oder für eine Thiolgruppe stehen kann.

15. Intermediäre Verbindungen, **dadurch gekennzeichnet, dass** sie ausgewählt sind aus:
1-(4-(Pentylthioethyloxy)phenyl)-3-(4-hydroxy-3,5-dimethylphenyl)prop-2-en-1-on;
1-(4-((R,S)-5-[1,2]Dithiolan-3-ylpentyloxy)phenyl)-3-(4-hydroxy-3,5-dimethylphenyl)prop-2-en-1-on;
1-(4-Methylthiophenyl)-3-(4-hydroxy-3,5-dibromphenyl)prop-2-en-1-on,
1-(4-(Cyclohexylethyloxy)phenyl)-3-(4-hydroxy-3,5-dimethylphenyl)prop-2-en-1-on;
1-(4-Methylthio-3,5-dimethylphenylphenyl)-3-(4-hydroxy-3,5-dimethylphenyl)prop-2-en-1-on;
1-(4-Methoxy-3,5-dimethylphenyl)-3-(4-hydroxy-3,5-dimethylphenyl)prop-2-en-1-on;
1-(4-(Cyclohexylethyloxy)-3,5-dimethylphenyl)-3-(4-hydroxy-3,5-dimethylphenyl)prop-2-en-1-on;
1-(4-(Cyclohexylthioethyloxy)phenyl)-3-(4-hydroxy-3,5-dimethylphenyl)prop-2-en-1-on,
1-(2,4,5-Trimethylphenyl)-3-(4-hydroxy-3,5-dimethylphenyl)prop-2-en-1-on;
1-(4-(Cyclohexylthioethyloxy)-3,5-dimethylphenyl)-3-(4-hydroxy-3,5-dimethylphenyl)prop-2-en-1-on;
1-(4-Methylthiophenyl)-3-(4-hydroxy-3-fluorphenyl)prop-2-en-1-on;
1-(2,3,4,5,6-Pentamethylphenyl)-3-(4-hydroxy-3,5-dimethylphenyl)prop-2-en-1-on;
1-(4-Phenoxyphenyl)-3-(4-hydroxy-3,5-dimethylphenyl)prop-2-en-1-on,
1-(4-Methoxy-3-fluorphenyl)-3-(4-hydroxy-3,5-dimethylphenyl)prop-2-en-1-on;
1-(4-Methoxy-3-methylphenyl)-3-(4-hydroxy-3,5-dimethylphenyl)prop-2-en-1-on;
1-(4-Hexylthio-3,5-dimethylphenyl)-3-(4-hydroxy-3,5-dimethylphenyl)prop-2-en-1-on;
1-(2,5-Dimethoxyphenyl)-3-(4-hydroxy-3,5-dimethylphenyl)prop-2-en-1-on;
1-(4-Bromphenyl)-3-(4-hydroxy-3,5-difluorphenyl)prop-2-en-1-on;
1-(4-Methoxy-3-trifluormethylphenyl)-3-(4-hydroxy-3,5-dimethylphenyl)prop-2-en-1-on;

16. Verbindungen gemäß einem der vorhergehenden Ansprüche 1 bis 13 als Medikamente.

17. Kosmetische oder pharmazeutische Zusammensetzung, umfassend in einem pharmazeutisch oder kosmetisch verträglichen Träger wenigstens eine Verbindung der allgemeinen Formel (I), wie in einem der Ansprüche 1 bis 13 definiert, gegebenenfalls zusammen mit einer weiteren therapeutisch und/oder kosmetisch aktiven Verbindung.

18. Kosmetische oder pharmazeutische Zusammensetzung gemäß Anspruch 17 für die Behandlung von kardiovaskulären Erkrankungen, Dyslipidämien, mit Syndrom X assoziierten Krankheiten, Diabetes, Obesität, Hypertonie, Entzündungskrankheiten, Hautkrankheiten (Psorias, atopischen Dermatiden, Akne), Asthma, mit oxidativem Stress verbundenen Störungen oder für die Behandlung allgemeiner Alterserscheinungen, insbesondere der Hautalterung.
